# EUROPEAN PATENT SPECIFICATION

(11) **EP 1 622 572 B1**
(45) Date of publication and mention of the grant of the patent: **20.12.2017**
(21) Application number: 04775924.6
(22) Date of filing: 30.04.2004
(51) Int. Cl.: A61K 47/54, C12N 15/87

(54) **CONJUGATES AND COMPOSITIONS FOR CELLULAR DELIVERY**
KONJUGATE UND ZUSAMMENSETZUNGEN FÜR DIE ZELLULÄRE ABGABE
CONJUGUES ET COMPOSITIONS POUR LA LIVRAISON CELLULAIRE

(30) Priority: 30.04.2003 US 427160; 13.02.2004 US 780447
(43) Date of publication of application: 08.02.2006
(60) Divisional application: 17000190.3
(73) Proprietor: Sirna Therapeutics, Inc., Cambridge, MA 02142 (US)
(72) Inventor: VARGEESE, Chandra, Broomfield, CO 80020 (US); HAEBERLI, Peter, Berthoud, CO 80513 (US); WANG, Weimin, Superior, CO 80027 (US); CHEN, Tongqian, Longmont, CO 80503 (US)
(74) Representative: Müller-Boré & Partner Patentanwälte PartG mbB
(86) International application number: PCT/US2004/013456
(87) International publication number: WO 2005/041859

(56) References cited:
- WO-A1-00/44914
- WO-A1-90/12096
- WO-A1-91/14696
- WO-A1-96/34876
- WO-A2-02/094185
- US-A- 5 416 203
- US-A- 5 643 889
- US-B1- 6 168 778
- CONNOLLY D.T. ET AL.: 'Binding and Endocytosis of Cluster Glycosides by Rabbit Hepatocytes' vol. 257, no. 2, January 1982, pages 939 - 945, XP003010765
- RENSEN P.C.N. ET AL.: 'Determination of the Upper Size Limit for Uptake and Processing of Ligands by the Asialoglycoprotein Receptor on Hepatocytes in Vitro ad in Vivo' JOURNAL OF BIOLOGICAL CHEMISTRY vol. 276, no. 40, October 2001, pages 37577 - 37584, XP003010766

### SEQUENCE LISTING

<110> Sirna Therapeutics, Inc.
   Vargeese, Chandra
   Haeberli, Peter
   Wang, Weimin
   Chen, Tongqian
<120> Conjugates and Compositions for Cellular Delivery
<130> 600/032PCT (MBHB02-312-H)
<150> > US 10/780447
   <151> 2004-02-13
<150> US 10/427,160
   <151> 2003-04-30
<150> PCT/US 02/15876
   <151> 2002-05-17
<150> US 60/292,217
   <151> 2001-05-18
<150> US 60/362,016
   <151> 2002-03-06
<150> US 60/306,883
   <151> 2001-07-20
<150> US 60/311,865
   <151> 2001-08-13
<150> PCT/US 03/05346
   <151> 2003-02-20
<150> PCT/US 03/05028
   <151> 2003-02-20
<150> US 60/358,580
   <151> 2002-02-20
<150> US 60/363,124
   <151> 2002-03-11
<150> US 60/386,782
   <151> 2002-06-06
<150> US 60/406,784
   <151> 2002-08-29
<150> US 60/408,378
   <151> 2002-09-05
<150> US 60/409,293
   <151> 2002-09-09
<150> US 60/440,129
   <151> 2003-01-15
<160> 24
<170> PatentIn version 3.2
<210> 1
   <211> 10
   <212> RNA
   <213> Artificial Sequence
<220>
   <223> Description of Artificial Sequence: Example of a Stem II region
<400> 1
   gccguuaggc 10
<210> 2
   <211> 15
   <212> RNA
   <213> Artificial Sequence
<220>
   <223> Description of Artificial Sequence: Generic Target Nucleic Acid
<220>
   <221> misc_feature
   <222> (1)..(6)
   <223> n stands for a, c, g, or u
<220>
   <221> misc_feature
   <222> (9)..(15)
   <223> n stands for a, c, g, or u
<220>
   <221> misc_feature
   <222> (8)..(8)
   <223> n stands for a, u, or c
<400> 2
   nnnnnnunnn nnnnn 15
<210> 3
   <211> 36
   <212> RNA
   <213> Artificial Sequence
<220>
   <223> Description of Artificial Sequence: Enzymatic Nucleic Acid
<220>
   <221> misc_feature
   <222> (1)..(7)
   <223> n stands for a, c, g, or u
<220>
   <221> misc_feature
   <222> (16)..(18)
   <223> n stands for a, c, g, or u
<220>
   <221> misc_feature
   <222> (23)..(25)
   <223> n stands for a, c, g, or u
<220>
   <221> misc_feature
   <222> (31)..(36)
   <223> n stands for a, c, g, or u
<220>
   <221> misc_feature
   <222> (1)..(8)
   <223> 2'-O-Methyl
<220>
   <221> misc_feature
   <222> (12)..(12)
   <223> 2'-O-Methyl
<220>
   <221> misc_feature
   <222> (14)..(26)
   <223> 2'-O-Methyl
<220>
   <221> misc_feature
   <222> (28)..(29)
   <223> 2'-O-Methyl
<220>
   <221> misc_feature
   <222> (31)..(36)
   <223> 2'-O-Methyl
<220>
   <221> misc_feature
   <222> (9)..(9)
   <223> 2'-deoxy-2'-C-Allyl
<400> 3
   nnnnnnncug augagnnnga aannncgaaa nnnnnn 36
<210> 4
   <211> 14
   <212> RNA
   <213> Artificial Sequence
<220>
   <223> Description of Artificial Sequence: Generic Target Nucleic Acid
<220>
   <221> misc_feature
   <222> (1)..(5)
   <223> n stands for a, c, g, or u
<220>
   <221> misc_feature
   <222> (8)..(14)
   <223> n stands for a, c, g, or u
<220>
   <221> misc_feature
   <222> (7)..(7)
   <223> n stands for a, u, or c
<400> 4
   nnnnncnnnn nnnn 14
<210> 5
   <211> 35
   <212> RNA
   <213> Artificial Sequence
<220>
   <223> Description of Artificial Sequence: Enzymatic Nucleic Acid
<220>
   <221> misc_feature
   <222> (1)..(7)
   <223> n stands for a, c, g, or u
<220>
   <221> misc_feature
   <222> (16)..(18)
   <223> n stands for a, c, g, or u
<220>
   <221> misc_feature
   <222> (23)..(25)
   <223> n stands for a, c, g, or u
<220>
   <221> misc_feature
   <222> (31)..(35)
   <223> n stands for a, c, g, or u
<220>
   <221> misc_feature
   <222> (1)..(8)
   <223> 2'-O-Methyl
<220>
   <221> misc_feature
   <222> (12)..(12)
   <223> 2'-O-Methyl
<220>
   <221> misc_feature
   <222> (14)..(26)
   <223> 2'-O-Methyl
<220>
   <221> misc_feature
   <222> (28)..(29)
   <223> 2'-O-Methyl
<220>
   <221> misc_feature
   <222> (31)..(35)
   <223> 2'-O-Methyl
<220>
   <221> misc_feature
   <222> (30)..(30)
   <223> n stands for Inosine
<220>
   <221> misc_feature
   <222> (9)..(9)
   <223> 2'-deoxy-2'-C-Allyl
<400> 5
   nnnnnnncug augagnnnga aannncgaan nnnnn 35
<210> 6
   <211> 15
   <212> RNA
   <213> Artificial Sequence
<220>
   <223> Description of Artificial Sequence: Generic Target Nucleic Acid
<220>
   <221> misc_feature
   <222> (1)..(6)
   <223> n stands for a, c, g, or u
<220>
   <221> misc_feature
   <222> (9)..(15)
   <223> n stands for a, c, g, or u
<220>
   <221> misc_feature
   <222> (7)..(7)
   <223> n stands for u, or c
<400> 6
   nnnnnnngnn nnnnn 15
<210> 7
   <211> 35
   <212> RNA
   <213> Artificial Sequence
<220>
   <223> Description of Artificial Sequence: Enzymatic Nucleic Acid
<220>
   <221> misc_feature
   <222> (1)..(7)
   <223> n stands for a, c, g, or u
<220>
   <221> misc_feature
   <222> (29)..(35)
   <223> n stands for a, c, g, or u
<220>
   <221> misc_feature
   <222> (1)..(8)
   <223> 2'-O-Methyl
<220>
   <221> misc_feature
   <222> (13)..(16)
   <223> 2'-O-Methyl
<220>
   <221> misc_feature
   <222> (18)..(35)
   <223> 2'-O-Methyl
<220>
   <221> misc_feature
   <222> (11)..(11)
   <223> Phosphorothioate 3'-Internucleotide Linkage
<400> 7
   nnnnnnnuga uggcaugcac uaugcgcgnn nnnnn 35
<210> 8
   <211> 48
   <212> RNA
   <213> Artificial Sequence
<220>
   <223> Description of Artificial Sequence: Enzymatic Nucleic Acid
<220>
   <221> misc_feature
   <222> (1)..(8)
   <223> 2'-O-Methyl
<220>
   <221> misc_feature
   <222> (12)..(21)
   <223> 2'-O-Methyl
<220>
   <221> misc_feature
   <222> (32)..(37)
   <223> 2'-O-Methyl
<220>
   <221> misc_feature
   <222> (44)..(48)
   <223> 2'-O-Methyl
<220>
   <221> misc_feature
   <222> (9)..(10)
   <223> 2'-deoxy-2'-amino
<220>
   <221> misc_feature
   <222> (22)..(26)
   <223> 2'-deoxy-2'-amino
<220>
   <221> misc_feature
   <222> (38)..(40)
   <223> 2'-deoxy-2'-amino
<400> 8
   gugugcaacc ggaggaaacu cccuucaagg acgaaagucc gggacggg 48
<210> 9
   <211> 16
   <212> RNA
   <213> Artificial Sequence
<220>
   <223> Description of Artificial Sequence: Target Nucleic Acid
<400> 9
   gccguggguu gcacac 16
<210> 10
   <211> 36
   <212> RNA
   <213> Artificial Sequence
<220>
   <223> Description of Artificial Sequence: Enzymatic Nucleic Acid
<220>
   <221> misc_feature
   <222> (1)..(7)
   <223> 2'-O-Methyl
<220>
   <221> misc_feature
   <222> (36)..(36)
   <223> 3'-3' inverted abasic moiety
<220>
   <221> misc_feature
   <222> (9)..(15)
   <223> 2'-O-Methyl
<220>
   <221> misc_feature
   <222> (19)..(19)
   <223> 2'-O-Methyl
<220>
   <221> misc_feature
   <222> (21)..(21)
   <223> 2'-O-Methyl
<220>
   <221> misc_feature
   <222> (23)..(23)
   <223> 2'-O-Methyl
<220>
   <221> misc_feature
   <222> (26)..(26)
   <223> 2'-O-Methyl
<220>
   <221> misc_feature
   <222> (28)..(35)
   <223> 2'-O-Methyl
<220>
   <221> misc_feature
   <222> (1)..(4)
   <223> Phosphorothioate 3'-Internucleotide Linkage
<220>
   <221> misc_feature
   <222> (17)..(17)
   <223> 2'-Deoxy-2'-Amino
<220>
   <221> misc_feature
   <222> (27)..(27)
   <223> 2'-Deoxy-2'-Amino
<400> 10
   gugccuggcc gaaaggcgag ugaggucugc cgcgcn 36
<210> 11
   <211> 15
   <212> RNA
   <213> Artificial Sequence
<220>
   <223> Description of Artificial Sequence: Target Nucleic Acid
<400> 11
   gcgcggcgca ggcac 15
<210> 12
   <211> 16
   <212> DNA
   <213> Artificial Sequence
<220>
   <223> Description of Artificial Sequence: Enzymatic Nucleic Acid Motif
<400> 12
   rggctagcta caacga 16
<210> 13
   <211> 33
   <212> RNA
   <213> Artificial Sequence
<220>
   <223> Description of Artificial Sequence: Enzymatic Nucleic Acid
<220>
   <221> misc_feature
   <222> (1)..(4)
   <223> Phosphorothioate 3'-Internucleotide Linkage
<220>
   <221> misc_feature
   <222> (1)..(15)
   <223> 2'-O-Methyl
<220>
   <221> misc_feature
   <222> (17)..(22)
   <223> 2'-O-Methyl
<220>
   <221> misc_feature
   <222> (25)..(25)
   <223> 2'-O-Methyl
<220>
   <221> misc_feature
   <222> (27)..(33)
   <223> 2'-O-Methyl
<220>
   <221> misc_feature
   <222> (16)..(16)
   <223> 2'-deoxy-2'-amino
<220>
   <221> misc_feature
   <222> (26)..(26)
   <223> 2'-deoxy-2'-amino
<220>
   <221> misc_feature
   <222> (33)..(33)
   <223> 3'-3 attached terminal deoxyabasic moeity
<400> 13
   gcaguggccg aaaggcgagu gaggucuagc uca 33
<210> 14
   <211> 16
   <212> PRT
   <213> Artificial Sequence
<220>
   <221> misc_feature
   <223> Synthetic peptide
<220>
   <221> misc_feature
   <222> (16)..(16)
   <223> Amide-substituted carboxy terminus on the lysine residue.
<400> 14
   Arg Gln Ile Lys Ile Trp Phe Gln Asn Arg Arg Met Lys Trp Lys Lys
   1               5                   10                  15
<210> 15
   <211> 26
   <212> PRT
   <213> Artificial Sequence
<220>
   <221> misc_feature
   <223> Synthetic peptide
<400> 15
   Ala Ala Val Ala Leu Leu Pro Ala Val Leu Leu Ala Leu Leu Ala Pro
   1               5                   10                  15
   Val Gln Arg Lys Arg Gln Lys Leu Met Pro
               20                  25
<210> 16
   <211> 17
   <212> PRT
   <213> Artificial Sequence
<220>
   <221> misc_feature
   <223> Synthetic peptide
<400> 16
   Met Gly Leu Gly Leu His Leu Leu Val Leu Ala Ala Ala Leu Gln Gly
   1               5                   10                  15
   Ala
<210> 17
   <211> 24
   <212> PRT
   <213> Artificial Sequence
<220>
   <221> misc_feature
   <223> Synthetic peptide
<400> 17
   Gly Ala Leu Phe Leu Gly Phe Leu Gly Ala Ala Gly Ser Thr Met Gly
   1 5 10 15
   Ala Pro Lys Ser Lys Arg Lys Val
   20
<210> 18
   <211> 9
   <212> PRT
   <213> Artificial Sequence
<220>
   <221> misc_feature
   <223> Synthetic peptide
<400> 18
   Arg Lys Lys Arg Arg Gln Arg Arg Arg
   1 5
<210> 19
   <211> 24
   <212> PRT
   <213> Artificial Sequence
<220>
   <221> misc_feature
   <223> Synthetic peptide
<400> 19
   Gly Leu Phe Glu Ala Ile Ala Gly Phe Ile Glu Asn Gly Trp Glu Gly
   1 5 10 15
   Met Ile Asp Gly Gly Gly Tyr Cys
   20
<210> 20
   <211> 5
   <212> PRT
   <213> Artificial Sequence
<220>
   <221> misc_feature
   <222> (1)..(1)
   <223> Xaa stands for any amino acid
<220>
   <221> misc_feature
   <222> (5)..(5)
   <223> Xaa stands for any amino acid
<220>
   <221> misc_feature
   <223> Synthetic peptide
<400> 20
   Xaa Arg Gly Asp Xaa
   1 5
<210> 21
   <211> 27
   <212> PRT
   <213> Artificial Sequence
<220>
   <221> misc_feature
   <223> Synthetic peptide
<400> 21
   Gly Trp Thr Leu Asn Ser Ala Gly Tyr Leu Leu Gly Lys Ile Asn Leu
   1 5 10 15
   Lys Ala Leu Ala Ala Leu Ala Lys Lys Ile Leu
   20 25
<210> 22
   <211> 9
   <212> PRT
   <213> Artificial Sequence
<220>
   <221> misc_feature
   <222> (1)..(1)
   <223> Ser stands for optional Serine for coupling
<220>
   <221> misc_feature
   <222> (2)..(2)
   <223> Phe stands for optional D isomer for stability
<220>
   <221> misc_feature
   <222> (5)..(5)
   <223> Trp stands for optional D isomer for stability
<220>
   <221> misc_feature
   <223> Synthetic peptide
<400> 22
   Ser Phe Cys Tyr Trp Lys Thr Cys Thr
   1 5
<210> 23
   <211> 9
   <212> PRT
   <213> Artificial Sequence
<220>
   <221> misc_feature
   <222> (1)..(1)
   <223> Ser stands for optional Serine for coupling
<220>
   <221> misc_feature
   <223> Synthetic peptide
<400> 23
   Ser Asp His Gln Leu Asn Pro Ala Phe
   1               5
<210> 24
   <211> 34
   <212> RNA
   <213> Artificial Sequence
<220>
   <223> Description of Artificial Sequence: Nucleic Acid
<220>
   <221> misc_feature
   <222> (1)..(4)
   <223> Phosphorothioate 3'-Internucleotide Linkage
<220>
   <221> misc_feature
   <222> (1)..(8)
   <223> 2'-O-Methyl
<220>
   <221> misc_feature
   <222> (13)..(25)
   <223> 2'-O-Methyl
<220>
   <221> misc_feature
   <222> (27)..(28)
   <223> 2'-O-Methyl
<220>
   <221> misc_feature
   <222> (30)..(34)
   <223> 2'-O-Methyl
<220>
   <221> misc_feature
   <222> (11)..(11)
   <223> 2'-deoxy-2'-C-Allyl
<220>
   <221> misc_feature
   <222> (34)..(34)
   <223> 3'-3 attached terminal deoxyabasic moeity
<400> 24
   gaguugcuga ugaggccgaa aggccgaaag ucug 34

## Description

The present invention relates to siNA conjugates. The discussion is provided only for understanding of the invention that follows. This summary is not an admission that any of the work described below is prior art to the claimed invention.

The cellular delivery of various therapeutic compounds, such as antiviral and chemotherapeutic agents, is usually compromised by two limitations. First the selectivity of therapeutic agents is often low, resulting in high toxicity to normal tissues. Secondly, the trafficking of many compounds into living cells is highly restricted by the complex membrane systems of the cell. Specific transporters allow the selective entry of nutrients or regulatory molecules, while excluding most exogenous molecules such as nucleic
acids and proteins. Various strategies can be used to improve transport of compounds into cells, including the use of lipid carriers and various conjugate systems. Conjugates are often selected based on the ability of certain molecules to be selectively transported into specific cells, for example via receptor mediated endocytosis. By attaching a compound of interest to molecules that are actively transported across the cellular membranes, the effective transfer of that compound into cells or specific cellular organelles can be realized. Alternately, molecules that are able to penetrate cellular membranes without active transport mechanisms, for example, various lipophilic molecules, can be used to deliver compounds of interest. Examples of molecules that can be utilized as conjugates include but are not limited to peptides, hormones, fatty acids, vitamins, flavonoids, sugars, reporter molecules, reporter enzymes, chelators, porphyrins, intercalcators, and other molecules that are capable of penetrating cellular membranes, either by active transport or passive transport.

The delivery of compounds to specific cell types, for example, cancer cells or cells specific to particular tissues and organs, can be accomplished by utilizing receptors associated with specific cell types. Particular receptors are overexpressed in certain cancerous cells, including the high affinity folic acid receptor. For example, the high affinity folate receptor is a turnor marker that is overexpressed in a variety of neoplastic tissues, including breast, ovarian, cervical, colorectal, renal, and nasoparyngeal tumors, but is expressed to a very limited extent in normal tissues. The use of folic acid based conjugates to transport exogenous compounds across cell membranes can provide a targeted delivery approach to the treatment and diagnosis of disease and can provide a reduction in the required dose of therapeutic compounds. Furthermore, therapeutic bioavialability, pharmacodynamics, and pharmacokinetic parameters can be modulated through the use of bioconjugates, including folate bioconjugates. Godwin et al., 1972, J. Biol. Chem., 247, 2266-2271, report the synthesis of biologically active pteroyloligo-L-glutamates. Habus et al., 1998, Bioconjugate Chem., 9, 283-291, describe a method for the solid phase synthesis of certain oligonucleotide-folate conjugates. Cook, US Patent No. 6,721,208, describes certain oligonucleotides modified with specific conjugate groups. The use of biotin and folate conjugates to enhance transmembrane transport of exogenous molecules, including specific oligonucleotides has been reported by Low et al., US Patent Nos. 5,416,016, 5,108,921, and International PCT publication No. WO 90/12096. Manoharan et al., International PCT publication No. WO 99/66063 describe certain folate conjugates, including specific nucleic acid folate conjugates with a phosphoramidite moiety attached to the nucleic acid component of the conjugate, and methods for the synthesis of these folate conjugates. Nomura et al., 2000, J. Org. Chem., 65, 5016-5021, describe the synthesis of an intermediate, alpha-[2-(trimethylsilyl)ethoxycarbonl]folic acid, useful in the synthesis of ceratin types of folate-nucleoside conjugates. Guzaev et al., US 6,335,434, describes the synthesis of certain folate oligonucleotide conjugates.

The delivery of compounds to other cell types can be accomplished by utilizing receptors associated with a certain type of cell, such as hepatocytes. For example, drug delivery systems utilizing receptor-mediated endocytosis have been employed to achieve drug targeting as well as drug-uptake enhancement. The asialoglycoprotein receptor (ASGPr) (see for example Wu and Wu, 1987, J. Biol. Chem. 262, 4429-4432) is unique to hepatocytes and binds branched galactose-terminal glycoproteins, such as asialoorosomucoid (ASOR). Binding of such glycoproteins or synthetic glycoconjugates to the receptor takes place with an affinity that strongly depends on the degree of branching of the oligosaccharide chain, for example, triatennary structures are bound with greater affinity than biatenarry or monoatennary chains (Baenziger and Fiete, 1980, Cell, 22, 611-620; Connolly et al., 1982, J. Biol. Chem., 257, 939-945). Lee and Lee, 1987, Glycoconjugate J., 4, 317-328, obtained this high specificity through the use of N-acetyl-D-galactosamine as the carbohydrate moiety, which has higher affinity for the receptor, compared to galactose. This "clustering effect" has also been described for the binding and uptake of mannosyl-terminating glycoproteins or glycoconjugates (Ponpipom et al., 1981, J. Med. Chem., 24, 1388-1395). The use of galactose and galactosamine based conjugates to transport exogenous compounds across cell membranes can provide a targeted delivery approach to the treatment of liver disease such as HBV and HCV infection or hepatocellular carcinoma. The use of bioconjugates can also provide a reduction in the required dose of therapeutic compounds required for treatment. Furthermore, therapeutic bioavialability, pharmacodynamics, and pharmacokinetic parameters can be modulated through the use of bioconjugates.

A number of peptide based cellular transporters have been developed by several research groups. These peptides are capable of crossing cellular membranes *in vitro* and *in vivo* with high efficiency. Examples of such fusogenic peptides include a 16-amino acid fragment of the homeodomain of ANTENNAPEDIA, a Drosophila transcription factor (Wang et al., 1995, PNAS USA., 92, 3318-3322); a 17-mer fragment representing the hydrophobic region of the signal sequence of Kaposi fibroblast growth factor with or without NLS domain (Antopolsky et al., 1999, Bioconj. Chem., 10, 598-606); a 17-mer signal peptide sequence of caiman crocodylus Ig(5) light chain (Chaloin et al., 1997, Biochem. Biophys.. Res. Comm., 243, 601-608); a 17-amino acid fusion sequence of HIV envelope glycoprotein gp4114, (Morris et al., 1997, Nucleic Acids Res., 25, 2730-2736); the HIV-1 Tat49-57 fragment (Schwarze et al., 1999, Science, 285, 1569-1572); a transportan A - achimeric 27-mer consisting of N-terminal fragment of neuropeptide galanine and membrane interacting wasp venom peptide mastoporan (Lindgren et al., 2000, Bioconjugate Chem., 11, 619-626); and a 24-mer derived from influenza virus hemagglutinin envelop glycoprotein (Bongartz et al., 1994, Nucleic Acids Res., 22, 4681-4688).

These peptides were successfully used as part of an antisense oligonucleotide-peptide conjugate for cell culture transfection without lipids. In a number of cases, such conjugates demonstrated better cell culture efficacy then parent oligonucleotides transfected using lipid delivery. In addition, use of phage display techniques has identified several organ targeting and tumor targeting peptides *in vivo* (Ruoslahti, 1996, Ann. Rev. Cell Dev. Biol., 12, 697-715). Conjugation of tumor targeting peptides to doxorubicin has been shown to significantly improve the toxicity profile and has demonstrated enhanced efficacy of doxorubicin in the *in vivo* murine cancer model MDA-MB-435 breast carcinoma (Arap et al., 1998, Science, 279, 377-380).

Hudson et al., 1999, Int. J. Pharm., 182, 49-58, describes the cellular delivery of specific hammerhead ribozymes conjugated to a transferrin receptor antibody. Janjic et al., US 6,168,778, describes specific VEGF nucleic acid ligand complexes for targeted drug delivery. Bonora et al., 1999, Nucleosides Nucleotides, 18, 1723-1725, describes the biological properties of specific antisense oligonucleotides conjugated to certain polyethylene glycols. Davis and Bishop, International PCT publication No. WO 99/17120 and Jaeschke et al., 1993, Tetrahedron Lett., 34, 301-4 describe specific methods of preparing polyethylene glycol conjugates. Tullis, International PCT Publication No. WO 88/09810; Jaschke, 1997, ACS Sympl Ser., 680, 265-283; Jaschke et al., 1994, Nucleic Acids Res., 22, 4810-17; Efimov et al., 1993, Bioorg. Khim., 19, 800-4; and Bonora et al., 1997, Bioconjugate Chem., 8, 793-797, describe specific oligonucleotide polyethylene glycol conjugates. Manoharan, International PCT Publication No. WO 00/76554, describes the preparation of specific ligand-conjugated oligodeoxyribonucleotides with certain cellular, serum, or vascular proteins. Defrancq and Lhomme, 2001, Bioorg Med Chem Lett., 11, 931-933; Cebon et al., 2000, Aust. J. Chem., 53, 333-339; and Salo et al., 1999, Bioconjugate Chem., 10, 815-823 describe specific aminooxy peptide oligonucleotide conjugates.

In this context, WO 96/34876 A1 describes *inter alia* complexes comprising nucleic acid ligands in association with lipophilic compounds. Further US-Patent 5,643,889 describes cholesterol-cordycepin conjugates having increased antiviral activity and/or metabolic stability. Furthermore, US-Patent 5,416,203 describes oligonucleotide-steroid conjugates. Moreover, WO 91/14696 A1 describes *inter alia* composition for the delivery of oligonucleotides into a cell, comprising oligonucleotides that are conjugated to a transport agent via a molecular linker containing at least one disulfide bond. Finally, WO 02/094185 A2 describes conjugates of biologically active compounds.

### Summary of the Invention

The present invention relates to a compound having Formula 107: 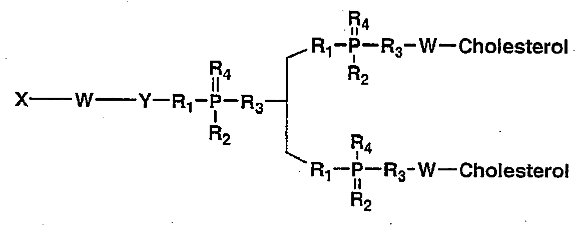 wherein X comprises a siNA molecule or a portion thereof; each W independently comprises a linker molecule or chemical linkage selected from the group consisting of amide, phosphate, phosphate ester, phosphoramidate, or thiophosphate ester linkage; Y comprises a linker molecules that can be present or absent; and each R₁, R₂, R₃, and R₄ independently comprises O, OH, H, alkyl, alkylhalo, O-alkyl, O-alkylcyano, S, S-alkyl, S-alkylcyano, N or substituted N; wherein said W-cholesterol comprises a compound having Formula 109: 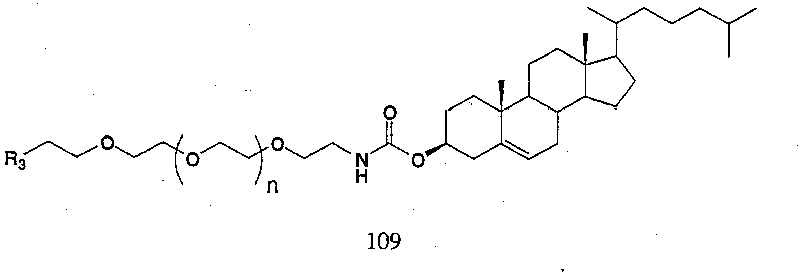 wherein n is independently an integer from about 1 to about 20.

In preferred embodiments, said siNA molecule comprises a sense strand and an antisense strand, and wherein said sense strand is conjugated with a compound comprising Formula 107.

The present invention features compositions and conjugates to facilitate delivery of molecules into a biological system, such as cells. The conjugates provided by the instant invention can impart therapeutic activity by transferring therapeutic compounds across cellular membranes. Described herein is the design and synthesis of novel agents for the delivery of molecules, including but not limited to small molecules, lipids, nucleosides, nucleotides, nucleic acids, polynucleotides, oligonucleotides, antibodies, toxins, negatively charged polymers and other polymers, for example proteins, peptides, hormones, carbohydrates, or polyamines, across cellular membranes. In general, the transporters described are designed to be used either individually or as part of a multi-component system, with or without degradable linkers. The compounds of the invention generally shown in the Formula above are expected to improve delivery of molecules into a number of cell types originating from different tissues, in the presence or absence of serum.

Described herein is a pharmaceutical composition comprising a compound of the invention and a pharmaceutically acceptable carrier.

Described herein is a method of treating a cancer patient, comprising contacting cells of the patient with a pharmaceutical composition described herein under conditions suitable for the treatment. This treatment can comprise the use of one or more other drug therapies under conditions suitable for the treatment. The cancers contemplated include but are not limited to breast cancer, lung cancer, colorectal cancer, brain cancer, esophageal cancer, stomach cancer, bladder cancer, pancreatic cancer, cervical cancer, head and neck cancer, ovarian cancer, melanoma, lymphoma, glioma, or multidrug resistant cancers.

Described herein is a method of treating a patient infected with a virus, comprising contacting cells of the patient with a pharmaceutical composition described herein, under conditions suitable for the treatment. This treatment can comprise the use of one or more other drug therapies under conditions suitable for the treatment. The viruses contemplated include but are not limited to HIV, HBV, HCV, CMV, RSV, HSV, poliovirus, influenza, rhinovirus, west nile virus, Ebola virus, foot and mouth virus, and papilloma virus.

Described herein is a kit for detecting the presence of a nucleic acid molecule or other target molecule in a sample, for example, a gene in a cancer cell, comprising a compound of the instant invention.

Described herein is a kit for detecting the presence of a nucleic acid molecule, or other target molecule in a sample, for example, a gene in a virus-infected cell, comprising a compound of the instant invention.

Described herein is a compound of the instant invention comprising a modified phosphate group, for example, a phosphoramidite, phosphodiester, phosphoramidate, phosphorothioate, phosphorodithioate, alkylphosphonate, arylphosphonate, monophosphate, diphosphate, triphosphate, or pyrophosphate.

According to the present invention, X of compounds having Formula 107 of the invention comprises a siNA molecule or a portion thereof. In one embodiment, the siNA molecule can be conjugated at the 5' end, 3'-end, or both 5' and 3' ends of the sense strand or region of the siNA. In one embodiment, the siNA molecule can be conjugated at the 3'-end of the antisense strand or region of the siNA with a compound of the invention. In one embodiment, both the sense strand and antisense strands or regions of the siNA molecule are conjugated with a compound of the invention. In one embodiment, only the sense strand or region of the siNA is conjugated with a compound of the invention. In one embodiment, only the antisense strand or region of the siNA is conjugated with a compound of the invention.

In one embodiment, W and/or Y of compounds having Formula 107 of the invention comprises a degradable or cleavable linker, for example a nucleic acid sequence comprising ribonucleotides and/or deoxynucleotides, such as a dimer, trimer, or tetramer. A non limiting example of a nucleic acid cleavable linker is an adenosine-deoxythymidine (A-dT) dimer or a cytidine-deoxythymidine (C-dT) dimer. In yet another embodiment, W of compounds having Formula 107 of the invention comprises a N-hydroxy succinimide (NHS) ester linkage, oxime linkage, disulfide linkage, phosphoramidate, phosphorothioate, phosphorodithioate, phosphodiester linkage, or NHC(O), CH₃NC(O), CONH, C(O)NCH₃, S, SO, SO₂, O, NH, NCH₃ group. In another embodiment, the degradable linker, W and/or Y, of compounds having Formula 107 of the invention comprises a linker that is susceptible to cleavage by carboxypeptidase activity.

In another embodiment, W and/or Y of Formula 107 comprises a polyethylene glycol linker having Formula 45: 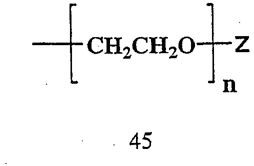 wherein Z comprises H, OH, O-alkyl, SH, S-alkyl, alkyl, substituted alkyl, aryl, substituted aryl, amino, substituted amino, nucleotide, nucleoside, nucleic acid, oligonucleotide, amino acid, peptide, protein, lipid, phospholipid, or label; and n is an integer from about 1 to about 100.

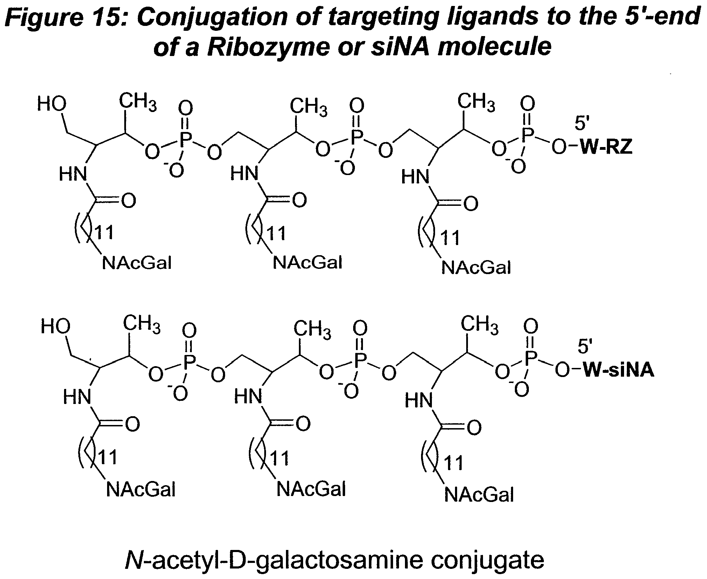

In one embodiment, the nucleic acid conjugates of the instant invention are assembled by solid phase synthesis, for example on an automated peptide synthesizer, for example a Miligen 9050 synthesizer and/or an automated oligonucleotide synthesizer such as an ABI 394, 390Z, or Pharmacia OligoProcess, OligoPilot, OligoMax, or AKTA synthesizer. In another embodiment, the nucleic acid conjugates of the invention are assembled post synthetically, for example, following solid phase oligonucleotide synthesis (see for example **Figure 15**).

In one embodiment, the nucleic acid conjugates of the instant invention are assembled post synthetically, for example, following solid phase oligonucleotide synthesis.

Described herein are compositions and conjugates comprising nucleosidic and non-nucleosidic derivatives. Described herein are also nucleic acid, polynucleotide and oligonucleotide derivatives including RNA, DNA, and PNA based conjugates. The attachment of compounds described herein to nucleosides, nucleotides, non-nucleosides, and nucleic acid molecules is provided at any position within the molecule, for example, at internucleotide linkages, nucleosidic sugar hydroxyl groups such as 5', 3', and 2'-hydroxyls, and/or at nucleobase positions such as amino and carbonyl groups.

The exemplary conjugates of the invention are described as compounds of the formulae herein, however, other peptide, protein, phospholipid, and poly-alkyl glycol derivatives are described herein, including various analogs of the compounds of formulae 1-122, including but not limited to different isomers of the compounds described herein.

Described herein are molecules, compositions and conjugates of molecules, for example, non-nucleosidic small molecules, nucleosides, nucleotides, and nucleic acids, such as enzymatic nucleic acid molecules, antisense nucleic acids, 2-5A antisense chimeras, triplex oligonucleotides, decoys, siNA, allozymes, aptamers, and antisense nucleic acids containing RNA cleaving chemical groups.

The present invention features compositions and conjugates to facilitate delivery of molecules into a biological system such as cells. The conjugates provided by the instant invention can impart therapeutic activity by transferring therapeutic compounds across cellular membranes. Described herein is the design and synthesis of novel agents for the delivery of molecules, including but not limited to small molecules, lipids, nucleosides, nucleotides, nucleic acids, negatively charged polymers and other polymers, for example proteins, peptides, carbohydrates, or polyamines. In general, the transporters described are designed to be used either individually or as part of a multi-component system. The compounds of the invention generally shown in Formulae herein are expected to improve delivery of molecules into a number of cell types originating from different tissues, in the presence or absence of serum.

Described herein are methods to modulate gene expression, for example, genes involved in the progression and/or maintenance of cancer or in a viral infection. For example, described herein is the use of one or more of the nucleic acid-based molecules and methods independently or in combination to inhibit the expression of the gene(s) encoding proteins associated with cancerous conditions, for example breast cancer, lung cancer, colorectal cancer, brain cancer, esophageal cancer, stomach cancer, bladder cancer, pancreatic cancer, cervical cancer, head and neck cancer, ovarian cancer, melanoma, lymphoma, glioma, or multidrug resistant cancer associated genes.

Described herein is the use of one or more of the nucleic acid-based molecules and methods independently or in combination to inhibit the expression of the gene(s) encoding viral proteins, for example HIV, HBV, HCV, CMV, RSV, HSV, poliovirus, influenza, rhinovirus, west nile virus, Ebola virus, foot and mouth virus, and papilloma virus associated genes.

Described herein is the use of an enzymatic nucleic acid molecule conjugate comprising compounds of formulae 1-122, preferably in the hammerhead, NCH, G-cleaver, amberzyme, zinzyme and/or DNAzyme motif, to inhibit the expression of cancer and virus associated genes.

Described herein is the use of an enzymatic nucleic acid molecule as a conjugate. These enzymatic nucleic acids can catalyze the hydrolysis of RNA phosphodiester bonds in trans (and thus can cleave other RNA molecules) under physiological conditions. **Table I** summarizes some of the characteristics of these enzymatic nucleic acids. Without being bound by any particular theory, in general, enzymatic nucleic acids act by first binding to a target RNA. Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA destroys its ability to direct synthesis of an encoded protein. After an enzymatic nucleic acid has bound and cleaved its RNA target, it is released from that RNA to search for another target and can repeatedly bind and cleave new targets. Thus, a single enzymatic nucleic acid molecule is able to cleave many molecules of target RNA. In addition, the enzymatic nucleic acid is a highly specific inhibitor of gene expression, with the specificity of inhibition depending not only on the base-pairing mechanism of binding to the target RNA, but also on the mechanism of target RNA cleavage. Single mismatches, or base-substitutions, near the site of cleavage can completely eliminate catalytic activity of an enzymatic nucleic acid.

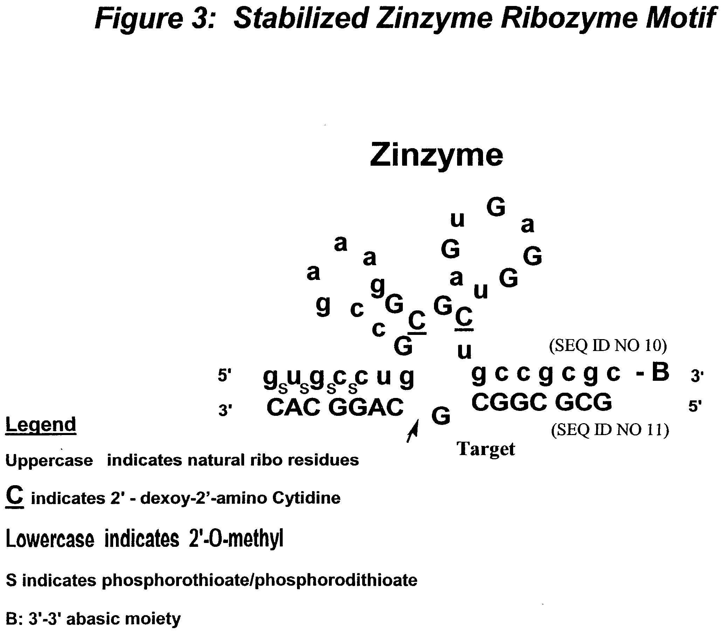

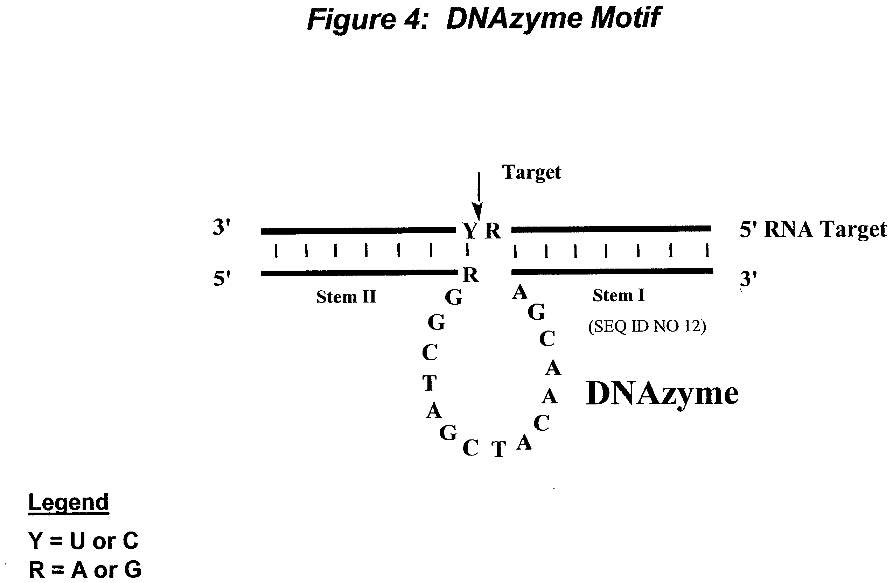

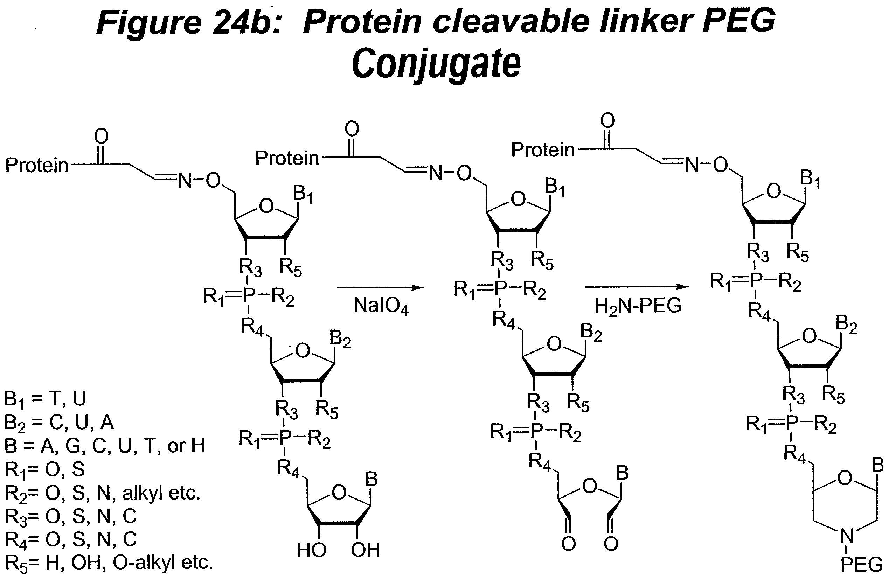

In one embodiment described herein, the enzymatic nucleic acid molecule component of the conjugate is formed in a hammerhead or hairpin motif, but can also be formed in the motif of a hepatitis delta virus, group I intron, group II intron or RNase P RNA (in association with an RNA guide sequence), Neurospora VS RNA, DNAzymes, NCH cleaving motifs, or G-cleavers. Examples of such hammerhead motifs are described by Dreyfus, supra, Rossi et al., 1992, AIDS Research and Human Retroviruses 8, 183; of hairpin motifs by Hampel et al., EP0360257, Hampel and Tritz, 1989 Biochemistry 28, 4929, Feldstein et al., 1989, Gene 82, 53, Haseloff and Gerlach, 1989, Gene, 82, 43, and Hampel et al., 1990 Nucleic Acids Res. 18, 299; Chowrira & McSwiggen, US. Patent No. 5,631,359; of the hepatitis delta virus motif is described by Perrotta and Been, 1992 Biochemistry 31, 16; of the RNase P motif by Guerrier-Takada et al., 1983 Cell 35, 849; Forster and Altman, 1990, Science 249; 783; Li and Altman, 1996, Nucleic Acids Res. 24, 835; Neurospora VS RNA ribozyme motif is described by Collins (Saville and Collins, 1990 Cell 61, 685-696; Saville and Collins, 1991 Proc. Natl. Acad. Sci. USA 88, 8826-8830; Collins and Olive, 1993 Biochemistry 32, 2795-2799; Guo and Collins, 1995, EMBO. J. 14, 363); Group II introns are described by Griffin et al., 1995, Chem. Biol. 2, 761; Michels and Pyle, 1995, Biochemistry 34, 2965; Pyle et al., International PCT Publication No. WO 96/22689; of the Group I intron by Cech et al., U.S. Patent 4,987,071 and of DNAzymes by Usman et al., International PCT Publication No. WO 95/11304; Chartrand et al., 1995, NAR 23, 4092; Breaker et al., 1995, Chem. Bio. 2, 655; Santoro et al., 1997, PNAS 94, 4262, and Beigelman et al., International PCT publication No. WO 99/55857. NCH cleaving motifs are described in Ludwig & Sproat, International PCT Publication No. WO 98/58058; and G-cleavers are described in Kore et al., 1998, Nucleic Acids Research 26, 4116-4120 and Eckstein et al., International PCT Publication No. WO 99/16871. Additional motifs such as the Aptazyme (Breaker et al., WO 98/43993), Amberzyme (Class I motif; Figure 3; Beigelman et al., U.S. Serial No. 09/301,511) and Zinzyme (Figure 4) (Beigelman et al., U.S. Serial No. 09/301,511), can also be used. These specific motifs are not limiting and those skilled in the art will recognize that all that is important in an enzymatic nucleic acid molecule is that it has a specific substrate binding site which is complementary to one or more of the target gene RNA regions, and that it have nucleotide sequences within or surrounding that substrate binding site which impart an RNA cleaving activity to the molecule (Cech et al., U.S. Patent No. 4,987,071).

In one embodiment of the present invention, a nucleic acid molecule component of a conjugate of the instant invention can be about 12 to about 100 nucleotides in length. For example, enzymatic nucleic acid molecules are preferably about 15 to about 50 nucleotides in length, more preferably about 25 to about 40 nucleotides in length, e.g., 34, 36, or 38 nucleotides in length (for example see Jarvis et al., 1996, J. Biol. Chem., 271, 29107-29112). Exemplary DNAzymes are preferably about 15 to about 40 nucleotides in length, more preferably about 25 to about 35 nucleotide in length, e.g., 29, 30, 31, or 32 nucleotides in length (see for example Santoro et al., 1998, Biochemistry, 37, 13330-13342; Chartrand et al., 1995, Nucleic Acids Research, 23, 4092-4096). Exemplary antisense molecules are preferably about 15 to about 75 nucleotides in length, more preferably about 20 to about 35 nucleotides in length, e.g., 25, 26, 27, or 28 nucleotide in length (see, for example, Woolf et al., 1992, PNAS., 89, 7305-7309; Milner et al., 1997, Nature Biotechnology, 15, 537-541). Exemplary triplex forming oligonucleotide molecules are preferably about 10 to about 40 nucleotides in length, more preferably about 12 to about 25 nucleotides in length, e.g., 18, 19, 20, or 21 nucleotides in length (see for example Maher et al., 1990, Biochemistry, 29, 8820-8826; Strobel and Dervan, 1990, Science, 249, 73-75). Exemplary double stranded siNA molecules of the invention comprise about 19 to about 25 nucleotides in length, e.g., about 19, 20, 21, 22, 23, 24, or 25 nucleotide in length, for each strand of the siNA molecule. Exemplary single stranded siNA molecules of the invention are about 38 to about 50 nucleotides in length, e.g., about 38, 39, 40, 41, 42, 43, 44, 45, 46, 47, 48, 49, or 50 nucleotides in length. The length of the nucleic acid molecules described and exemplified herein are not limiting within the general size ranges stated.

The conjugates of the invention are added directly, or can be complexed with cationic lipids, packaged within liposomes, or otherwise delivered to target cells or tissues. The conjugates and/or conjugate complexes can be locally administered to relevant tissues ex vivo, or in vivo through injection or infusion pump, with or without their incorporation in biopolymers. The compositions and conjugates of the instant invention, individually, or in combination or in conjunction with other drugs, can be used to treat diseases or conditions discussed above. For example, to treat a disease or condition associated with the levels of a pathogenic protein, the patient can be treated, or other appropriate cells can be treated, as is evident to those skilled in the art, individually or in combination with one or more drugs under conditions suitable for the treatment.

In a further embodiment, the described molecules can be used in combination with other known treatments to treat conditions or diseases discussed above. For example, the described molecules can be used in combination with one or more known therapeutic agents to treat breast, lung, prostate, colorectal, brain, esophageal, bladder, pancreatic, cervical, head and neck, and ovarian cancer, melanoma, lymphoma, glioma, multidrug resistant cancers, and/or HIV, HBV, HCV, CMV, RSV, HSV, poliovirus, influenza, rhinovirus, west nile virus, Ebola virus, foot and mouth virus, and papilloma virus infection.

Included in another embodiment are a series of multi-domain cellular transport vehicles (MCTV) including compounds of Formula 107 herein that enhance the cellular uptake and transmembrane permeability of negatively charged molecules in a variety of cell types. The compounds of the invention are used either alone or in combination with other compounds with a neutral or a negative charge including but not limited to neutral lipid and/or targeting components, to improve the effectiveness of the formulation or conjugate in delivering and targeting the predetermined compound or molecule to cells. Described herein is the utility of these compounds for increasing the transport of other impermeable and/or lipophilic compounds into cells. Targeting components include ligands for cell surface receptors including, peptides and proteins, glycolipids, lipids, carbohydrates, and their synthetic variants, for example folate receptors.

In another embodiment, the compounds of the invention are provided as a surface component of a lipid aggregate, such as a liposome encapsulated with the predetermined molecule to be delivered. Liposomes, which can be unilamellar or multilamellar, can introduce encapsulated material into a cell by different mechanisms. For example, the liposome can directly introduce its encapsulated material into the cell cytoplasm by
fusing with the cell membrane. Alternatively, the liposome can be compartmentalized into an acidic vacuole (i.e., an endosome) and its contents released from the liposome and out of the acidic vacuole into the cellular cytoplasm.

Described herein is a lipid aggregate formulation of the compounds described herein, including phosphatidylcholine (of varying chain length; e.g., egg yolk phosphatidylcholine), cholesterol, a cationic lipid, and 1,2-distearoyl-sn-glycero-3-phosphoethanolamine-polythyleneglycol-2000 (DSPE-PEG2000). The cationic lipid component of this lipid aggregate can be any cationic lipid known in the art such as dioleoyl 1,2,-diacyl-3-trimethylammonium-propane (DOTAP). In another embodiment this cationic lipid aggregate comprises a covalently bound compound described in any of the Formulae herein.

In another embodiment, polyethylene glycol (PEG) is covalently attached to the compounds of the present invention. The attached PEG can be any molecular weight but is preferably between 2000-50,000 daltons.

The compounds and methods described herein are useful for introducing nucleotides, nucleosides, nucleic acid molecules, lipids, peptides, proteins, and/or non-nucleosidic small molecules into a cell. For example, the embodiments described herein can be used for nucleotide, nucleoside, nucleic acid, lipids, peptides, proteins, and/or non-nucleosidic small molecule delivery where the corresponding target site of action exists intracellularly.

Described herein are compounds that provide conjugates of molecules that can interact with cellular receptors, such as high affinity folate receptors and ASGPr receptors, and provide a number of features that allow the efficient delivery and subsequent release of conjugated compounds across biological membranes. The compounds utilize chemical linkages between the receptor ligand and the compound to be delivered of length that can interact preferentially with cellular receptors. Furthermore, the chemical linkages between the ligand and the compound to be delivered can be designed as degradable linkages, for example by utilizing a phosphate linkage that is proximal to a nucleophile, such as a hydroxyl group. Deprotonation of the hydroxyl group or an equivalent group, as a result of pH or interaction with a nuclease, can result in nucleophilic attack of the phosphate resulting in a cyclic phosphate intermediate that can be hydrolyzed. This cleavage mechanism is analogous RNA cleavage in the presence of a base or RNA nuclease. Alternately, other degradable linkages can be selected that respond to various factors such as UV irradiation, cellular nucleases, pH, temperature etc. The use of degradable linkages allows the delivered compound to be
released in a predetermined system, for example in the cytoplasm of a cell, or in a particular cellular organelle.

Described herein are ligand derived phosphoramidites that are readily conjugated to compounds and molecules of interest. Phosphoramidite compounds permit the direct attachment of conjugates to molecules of interest without the need for using nucleic acid phosphoramidite species as scaffolds. As such, the use of phosphoramidite chemistry can be used directly in coupling the compounds to a compound of interest, without the need for other condensation reactions, such as condensation of the ligand to an amino group on the nucleic acid, for example at the N6 position of adenosine or a 2'-deoxy-2'-amino function. Additionally, compounds described herein can be used to introduce non-nucleic acid based conjugated linkages into oligonucleotides that can provide more efficient coupling during oligonucleotide synthesis than the use of nucleic acid-based phosphoramidites. This improved coupling can take into account improved steric considerations of abasic or non-nucleosidic scaffolds bearing pendant alkyl linkages.

Compounds described herein utilizing triphosphate groups can be utilized in the enzymatic incorporation of conjugate molecules into oligonucleotides. Such enzymatic incorporation is useful when conjugates are used in post-synthetic enzymatic conjugation or selection reactions, (see for example Matulic-Adamic et al., 2000, Bioorg. Med. Chem. Lett., 10, 1299-1302; Lee et al., 2001, NAR., 29, 1565-1573; Joyce, 1989, Gene, 82, 83-87; Beaudry et a/., 1992, Science 257, 635-641; Joyce, 1992, Scientific American 267, 90-97; Breaker et al., 1994, TIBTECH 12, 268; Bartel et al., 1993, Science 261: 1411-1418; Szostak, 1993, TIBS 17, 89-93; Kumar et al., 1995, FASEB J., 9, 1183; Breaker, 1996, Curr. Op. Biotech., 7, 442; Santoro et al., 1997, Proc. Natl. Acad. Sci., 94, 4262; Tang et al., 1997, RNA 3, 914; Nakamaye & Eckstein, 1994, *supra*; Long & Uhlenbeck, 1994, *supra*; Ishizaka *et al.,* 1995, *supra*; Vaish et al., 1997, Biochemistry 36, 6495; Kuwabara et al., 2000, Curr. Opin. Chem. Biol., 4, 669).

Compounds described herein can be used to detect the presence of a target molecule in a biological system, such as tissue, cell or cell lysate. Examples of target molecules include nucleic acids, proteins, peptides, antibodies, polysaccharides, lipids, hormones, sugars, metals, microbial or cellular metabolites, analytes, pharmaceuticals, and other organic and inorganic molecules or other biomolecules in a sample. The compounds can be conjugated to a predetermined compound or molecule that is capable of interacting with the target molecule in the system and providing a detectable signal or response. Various compounds and molecules known in
the art that can be used in these applications include but are not limited to antibodies, labeled antibodies, allozymes, aptamers, labeled nucleic acid probes, molecular beacons, fluorescent molecules, radioisotopes, polysaccharides, and any other compound capable of interacting with the target molecule and generating a detectable signal upon target interaction. For example, such compounds are described in Application entitled "NUCLEIC ACID SENSOR MOLECULES", USSN 09/800,594 filed on March 6, 2001 (Not yet assigned; Attorney Docket No. MBHBOO-816-A 700.001) with inventors Nassim Usman and James A. McSwiggen.

The term "biodegradable nucleic acid linker molecule" as used herein, refers to a nucleic acid molecule that is designed as a biodegradable linker to connect one molecule to another molecule, for example, a biologically active molecule. The stability of the biodegradable nucleic acid linker molecule can be modulated by using various combinations of ribonucleotides, deoxyribonucleotides, and chemically modified nucleotides, for example 2'-O-methyl, 2'-fluoro, 2'-amino, 2'-O-amino, 2'-C-allyl, 2'-O-allyl, and other 2'-modified or base modified nucleotides. The biodegradable nucleic acid linker molecule can be a dimer, trimer, tetramer or longer nucleic acid molecule, for example an oligonucleotide of about 2, 3, 4, 5, 6, 7, 8, 9, 10, 11, 12, 13, 14, 15, 16, 17, 18, 19, or 20 nucleotides in length, or can comprise a single nucleotide with a phosphorus based linkage, for example a phosphoramidate or phosphodiester linkage. The biodegradable nucleic acid linker molecule can also comprise nucleic acid backbone, nucleic acid sugar, or nucleic acid base modifications.

The term "biodegradable" as used herein, refers to degradation in a biological system, for example enzymatic degradation or chemical degradation.

The term "biologically active molecule" as used herein, refers to compounds or molecules that are capable of eliciting or modifying a biological response in a system. Non-limiting examples of biologically active molecules contemplated by the instant invention include therapeutically active molecules such as antibodies, hormones, antivirals, peptides, proteins, chemotherapeutics, small molecules, vitamins, co-factors, nucleosides, nucleotides, oligonucleotides, enzymatic nucleic acids, antisense nucleic acids, triplex forming oligonucleotides, 2,5-A chimeras, siNA, dsRNA, allozymes, aptamers, decoys and analogs thereof. Biologically active molecules of the invention also include molecules capable of modulating the pharmacokinetics and/or pharmacodynamics of other biologically active molecules, for example lipids and polymers such as polyamines, polyamides, polyethylene glycol and other polyethers.

The term "phospholipid" as used herein, refers to a hydrophobic molecule comprising at least one phosphorus group. For example, a phospholipid can comprise a phosphorus containing group and saturated or unsaturated alkyl group, optionally substituted with OH, COOH, oxo, amine, or substituted or unsubstituted aryl groups.

The term "nitrogen containing group" as used herein refers to any chemical group or moiety comprising a nitrogen or substituted nitrogen. Non-limiting examples of nitrogen containing groups include amines, substituted amines, amides, alkylamines, amino acids such as arginine or lysine, polyamines such as spermine or spermidine, cyclic amines such as pyridines, pyrimidines including uracil, thymine, and cytosine, morpholines, phthalimides, and heterocyclic amines such as purines, including guanine and adenine.

The term "target molecule" as used herein, refers to nucleic acid molecules, proteins, peptides, antibodies, polysaccharides, lipids, sugars, metals, microbial or cellular metabolites, analytes, pharmaceuticals, and other organic and inorganic molecules that are present in a system.

By "inhibit" or "down-regulate" it is meant that the expression of the gene, or level of RNAs or equivalent RNAs encoding one or more protein subunits, or activity of one or more protein subunits, such as pathogenic protein, viral protein or cancer related protein subunit(s), is reduced below that observed in the absence of the compounds or combination of compounds of the invention. In one embodiment, inhibition or down-regulation with an enzymatic nucleic acid molecule preferably is below that level observed in the presence of an enzymatically inactive or attenuated molecule that is able to bind to the same site on the target RNA, but is unable to cleave that RNA. In another embodiment, inhibition or down-regulation with antisense oligonucleotides is preferably below that level observed in the presence of, for example, an oligonucleotide with scrambled sequence or with mismatches. In another embodiment, inhibition or down-regulation of viral or oncogenic RNA, protein, or protein subunits with a compound of the instant invention is greater in the presence of the compound than in its absence.

By "up-regulate" is meant that the expression of the gene, or level of RNAs or equivalent RNAs encoding one or more protein subunits, or activity of one or more protein subunits, such as viral or oncogenic protein subunit(s), is greater than that observed in the absence of the compounds or combination of compounds of the invention. For example, the expression of a gene, such as a viral or cancer related gene, can be increased in order to treat, prevent, ameliorate, or modulate a pathological condition caused or exacerbated by an absence or low level of gene expression.

By "modulate" is meant that the expression of the gene, or level of RNAs or equivalent RNAs encoding one or more protein subunits, or activity of one or more protein subunit(s) of a protein, for example a viral or cancer related protein is up-regulated or down-regulated, such that the expression, level, or activity is greater than or less than that observed in the absence of the compounds or combination of compounds of the invention.

The term "enzymatic nucleic acid molecule" as used herein refers to a nucleic acid molecule which has complementarity in a substrate binding region to a specified gene target, and also has an enzymatic activity which is active to specifically cleave target RNA. That is, the enzymatic nucleic acid molecule is able to intermolecularly cleave RNA and thereby inactivate a target RNA molecule. These complementary regions allow sufficient hybridization of the enzymatic nucleic acid molecule to the target RNA and thus permit cleavage. One hundred percent complementarity is preferred, but complementarity as low as 50-75% can also be useful in this invention (see for example Werner and Uhlenbeck, 1995, Nucleic Acids Research, 23, 2092-2096; Hammann et al., 1999, Antisense and Nucleic Acid Drug Dev., 9, 25-31). The nucleic acids can be modified at the base, sugar, and/or phosphate groups. The term enzymatic nucleic acid is used interchangeably with phrases such as ribozymes, catalytic RNA, enzymatic RNA, catalytic DNA, aptazyme or aptamer-binding ribozyme, regulatable ribozyme, catalytic oligonucleotides, nucleozyme, DNAzyme, RNA enzyme, endoribonuclease, endonuclease, minizyme, leadzyme, oligozyme or DNA enzyme. All of these terminologies describe nucleic acid molecules with enzymatic activity. The specific enzymatic nucleic acid molecules described in the instant application are not limiting in the invention and those skilled in the art will recognize that all that is important in an enzymatic nucleic acid molecule of this invention is that it has a specific substrate binding site which is complementary to one or more of the target nucleic acid regions, and that it have nucleotide sequences within or surrounding that substrate binding site which impart a nucleic acid cleaving and/or ligation activity to the molecule (Cech et al., U.S. Patent No. 4,987,071; Cech et al., 1988, 260 JAMA 3030).

The term "nucleic acid molecule" as used herein, refers to a molecule having nucleotides. The nucleic acid can be single, double, or multiple stranded and can comprise modified or unmodified nucleotides or non-nucleotides or various mixtures and combinations thereof.

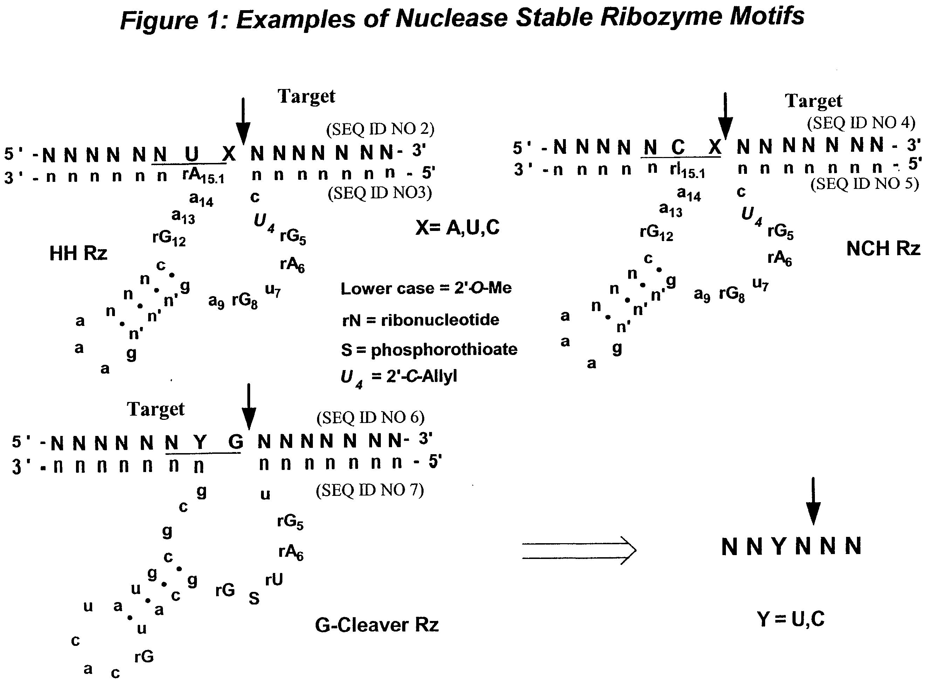

The term "enzymatic portion" or "catalytic domain" as used herein refers to that portion/region of the enzymatic nucleic acid molecule essential for cleavage of a nucleic acid substrate (for example see Figure 1).

The term "substrate binding arm" or "substrate binding domain" as used herein refers to that portion/region of a enzymatic nucleic acid which is able to interact, for example via complementarity (i.e., able to base-pair with), with a portion of its substrate. Preferably, such complementarity is 100%, but can be less if desired. For example, as few as 10 bases out of 14 can be base-paired (see for example Werner and Uhlenbeck, 1995, Nucleic Acids Research, 23, 2092-2096; Hammann et al., 1999, Antisense and Nucleic Acid Drug Dev., 9, 25-31). Examples of such arms are shown generally in Figures 1-4. That is, these arms contain sequences within a enzymatic nucleic acid which are intended to bring enzymatic nucleic acid and target RNA together through complementary base-pairing interactions. The enzymatic nucleic acid of the invention can have binding arms that are contiguous or non-contiguous and can be of varying lengths. The length of the binding arm(s) are preferably greater than or equal to four nucleotides and of sufficient length to stably interact with the target RNA; preferably 12-100 nucleotides; more preferably 14-24 nucleotides long (see for example Werner and Uhlenbeck, supra; Hamman et al., supra; Hampel et al., EP0360257; Berzal-Herrance et al., 1993, EMBO J., 12, 2567-73). If two binding arms are chosen, the design is such that the length of the binding arms are symmetrical (i.e., each of the binding arms is of the same length; e.g., five and five nucleotides, or six and six nucleotides, or seven and seven nucleotides long) or asymmetrical (i.e., the binding arms are of different length; e.g., six and three nucleotides; three and six nucleotides long; four and five nucleotides long; four and six nucleotides long; four and seven nucleotides long; and the like).

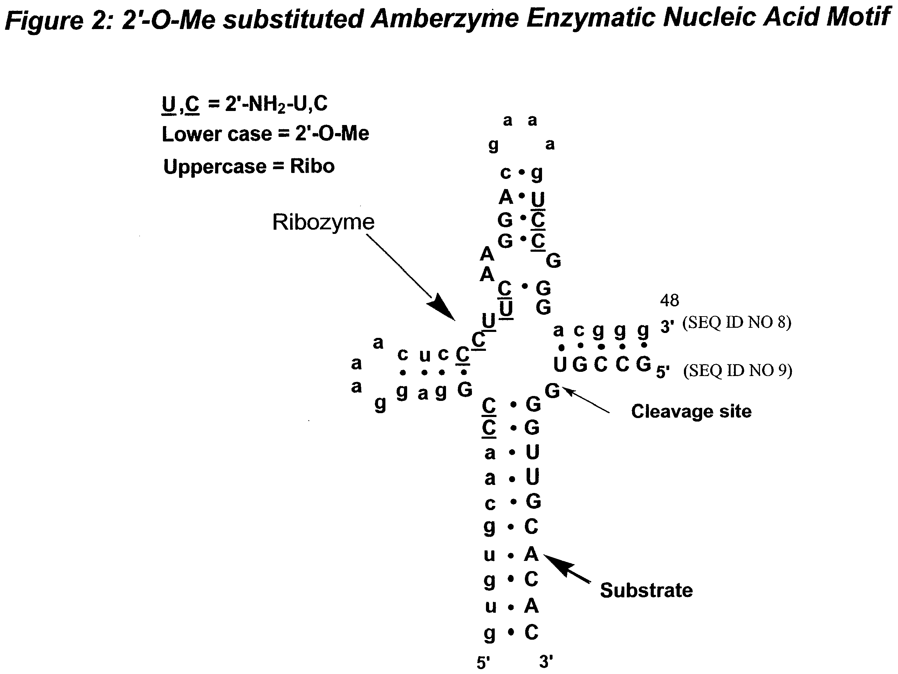

The term "Inozyme" or "NCH" motif as used herein, refers to an enzymatic nucleic acid molecule comprising a motif as is generally described as NCH Rz in Figure 1. Inozymes possess endonuclease activity to cleave RNA substrates having a cleavage triplet NCH/, where N is a nucleotide, C is cytidine and H is adenosine, uridine or cytidine, and / represents the cleavage site. H is used interchangeably with X. Inozymes can also possess endonuclease activity to cleave RNA substrates having a cleavage triplet NCN/, where N is a nucleotide, C is cytidine, and / represents the cleavage site. "I" in Figure 2 represents an Inosine nucleotide, preferably a ribo-Inosine or xylo-Inosine nucleoside.

The term "G-cleaver" motif as used herein, refers to an enzymatic nucleic acid molecule comprising a motif as is generally described as G-cleaver Rz in **Figure 1****.** G-cleavers possess endonuclease activity to cleave RNA substrates having a cleavage triplet NYN/, where N is a nucleotide, Y is uridine or cytidine and / represents the cleavage site. G-cleavers can be chemically modified as is generally shown in Figure 2.

The term "amberzyme" motif as used herein, refers to an enzymatic nucleic acid molecule comprising a motif as is generally described in **Figure 2****.** Amberzymes possess endonuclease activity to cleave RNA substrates having a cleavage triplet NG/N, where N is a nucleotide, G is guanosine, and / represents the cleavage site. Amberzymes can be chemically modified to increase nuclease stability through substitutions as are generally shown in Figure 3. In addition, differing nucleoside and/or non-nucleoside linkers can be used to substitute the 5'-gaaa-3' loops shown in the figure. Amberzymes represent a non-limiting example of an enzymatic nucleic acid molecule that does not require a ribonucleotide (2'-OH) group within its own nucleic acid sequence for activity.

The term "zinzyme" motif as used herein, refers to an enzymatic nucleic acid molecule comprising a motif as is generally described in **Figure 3****.** Zinzymes possess endonuclease activity to cleave RNA substrates having a cleavage triplet including but not limited to YG/Y, where Y is uridine or cytidine, and G is guanosine and / represents the cleavage site. Zinzymes can be chemically modified to increase nuclease stability through substitutions as are generally shown in **Figure 3****,** including substituting 2'-O-methyl guanosine nucleotides for guanosine nucleotides. In addition, differing nucleotide and/or non-nucleotide linkers can be used to substitute the 5'-gaaa-2' loop shown in the figure. Zinzymes represent a non-limiting example of an enzymatic nucleic acid molecule that does not require a ribonucleotide (2'-OH) group within its own nucleic acid sequence for activity.

The term 'DNAzyme' as used herein, refers to an enzymatic nucleic acid molecule that does not require the presence of a 2'-OH group for its activity. In particular embodiments the enzymatic nucleic acid molecule can have an attached linker(s) or other attached or associated groups, moieties, or chains containing one or more nucleotide with 2'-OH groups. DNAzymes can be synthesized chemically or expressed endogenously in vivo, by means of a single stranded DNA vector or equivalent thereof. An example of a DNAzyme is shown in **Figure 4** and is generally reviewed in Usman et al., International PCT Publication No. WO 95/11304; Chartrand et al., 1995, NAR 23, 4092; Breaker et al., 1995, Chem. Bio. 2, 655; Santoro et al., 1997, PNAS 94, 4262; Breaker, 1999, Nature Biotechnology, 17, 422-423; and Santoro et. al., 2000, J. Am. Chem. Soc., 122, 2433-39. Additional DNAzyme motifs can be selected for using techniques similar to those described in these references. The term "sufficient length" as used herein, refers to an oligonucleotide of length great enough to provide the intended function under the expected condition, i.e., greater than or equal to 3 nucleotides. For example, for binding arms of enzymatic nucleic acid "sufficient length" means that the binding arm sequence is long enough to provide stable binding to a target site under the expected binding conditions. Preferably, the binding arms are not so long as to prevent useful turnover of the nucleic acid molecule.

The term "stably interact" as used herein, refers to interaction of the oligonucleotides with target nucleic acid (e.g., by forming hydrogen bonds with complementary nucleotides in the target under physiological conditions) that is sufficient to the intended purpose (e.g., cleavage of target RNA by an enzyme).

The term "homology" as used herein, refers to the nucleotide sequence of two or more nucleic acid molecules is partially or completely identical.

The term "antisense nucleic acid", as used herein, refers to a non-enzymatic nucleic acid molecule that binds to target RNA by means of RNA-RNA or RNA-DNA or RNA-PNA (protein nucleic acid; Egholm et al., 1993 Nature 365, 566) interactions and alters the activity of the target RNA (for a review, see Stein and Cheng, 1993 Science 261, 1004 and Woolf et al., US patent No. 5,849,902). Typically, antisense molecules are complementary to a target sequence along a single contiguous sequence of the antisense molecule. However, in certain embodiments, an antisense molecule can bind to substrate such that the substrate molecule forms a loop, and/or an antisense molecule can bind such that the antisense molecule forms a loop. Thus, the antisense molecule can be complementary to two (or even more) non-contiguous substrate sequences or two (or even more) non-contiguous sequence portions of an antisense molecule can be complementary to a target sequence or both. For a review of current antisense strategies, see Schmajuk et al., 1999, J. Biol. Chem., 274, 21783-21789, Delihas et al., 1997, Nature, 15, 751-753, Stein et al., 1997, Antisense N. A. Drug Dev., 7, 151, Crooke, 2000, Methods Enzymol., 313, 3-45; Crooke, 1998, Biotech. Genet. Eng. Rev., 15, 121-157, Crooke, 1997, Ad. Pharmacol., 40, 1-49. In addition, antisense DNA can be used to target RNA by means of DNA-RNA interactions, thereby activating RNase H, which digests the target RNA in the duplex. The antisense oligonucleotides can comprise one or more RNAse H activating region, which is capable of activating RNAse H cleavage of a target RNA. Antisense DNA can be synthesized chemically or expressed via the use of a single stranded DNA expression vector or equivalent thereof.

The term "RNase H activating region" as used herein, refers to a region (generally greater than or equal to 4-25 nucleotides in length, preferably from 5-11 nucleotides in length) of a nucleic acid molecule capable of binding to a target RNA to form a noncovalent complex that is recognized by cellular RNase H enzyme (see for example Arrow et al., US 5,849,902; Arrow et al., US 5,989,912). The RNase H enzyme binds to the nucleic acid molecule-target RNA complex and cleaves the target RNA sequence. The RNase H activating region comprises, for example, phosphodiester, phosphorothioate (preferably at least four of the nucleotides are phosphorothiote substitutions; more specifically, 4-11 of the nucleotides are phosphorothiote substitutions); phosphorodithioate, 5'-thiophosphate, or methylphosphonate backbone chemistry or a combination thereof. In addition to one or more backbone chemistries described above, the RNase H activating region can also comprise a variety of sugar chemistries. For example, the RNase H activating region can comprise deoxyribose, arabino, fluoroarabino or a combination thereof, nucleotide sugar chemistry. Those skilled in the art will recognize that the foregoing are non-limiting examples and that any combination of phosphate, sugar and base chemistry of a nucleic acid that supports the activity of RNase H enzyme is within the scope of the definition of the RNase H activating region and the instant invention.

The term "2-5A antisense chimera" as used herein, refers to an antisense oligonucleotide containing a 5'-phosphorylated 2'-5'-linked adenylate residue. These chimeras bind to target RNA in a sequence-specific manner and activate a cellular 2-5A-dependent ribonuclease which, in turn, cleaves the target RNA (Torrence et al., 1993 Proc. Natl. Acad. Sci. USA 90, 1300; Silverman et al., 2000, Methods Enzymol., 313, 522-533; Player and Torrence, 1998, Pharmacol. Ther., 78, 55-113).

The term "triplex forming oligonucleotides" as used herein, refers to an oligonucleotide that can bind to a double-stranded DNA in a sequence-specific manner to form a triple-strand helix. Formation of such triple helix structure has been shown to inhibit transcription of the targeted gene (Duval-Valentin et al., 1992 Proc. Natl. Acad. Sci. USA 89, 504; Fox, 2000, Curr. Med. Chem., 7, 17-37; Praseuth et. al., 2000, Biochim. Biophys. Acta, 1489, 181-206).

The term "gene" it as used herein, refers to a nucleic acid that encodes an RNA, for example, nucleic acid sequences including structural genes encoding a polypeptide.

The term "pathogenic protein" as used herein, refers to endogenous or exongenous proteins that are associated with a disease state or condition, for example a particular cancer or viral infection.

The term "complementarity" refers to the ability of a nucleic acid to form hydrogen bond(s) with another RNA sequence by either traditional Watson-Crick or other non-traditional types. In reference to the nucleic molecules of the present invention, the binding free energy for a nucleic acid molecule with its target or complementary sequence is sufficient to allow the relevant function of the nucleic acid to proceed, e.g., enzymatic nucleic acid cleavage, antisense or triple helix inhibition. Determination of binding free energies for nucleic acid molecules is well known in the art (see, e.g., Turner et al., 1987, CSH Symp. Quant. Biol. LII pp. 123-133; Frier et al., 1986, Proc. Nat. Acad. Sci. USA 83:9373-9377; Turner et al., 1987, J. Am. Chem. Soc. 109:3783-3785). A percent complementarity indicates the percentage of contiguous residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence.

The term "RNA" as used herein, refers to a molecule comprising at least one ribonucleotide residue. By "ribonucleotide" or "2'-OH" is meant a nucleotide with a hydroxyl group at the 2' position of a β-D-ribo-furanose moiety.

The term "decoy RNA" as used herein, refers to a RNA molecule or aptamer that is designed to preferentially bind to a predetermined ligand. Such binding can result in the inhibition or activation of a target molecule. The decoy RNA or aptamer can compete with a naturally occurring binding target for the binding of a specific ligand. For example, it has been shown that over-expression of HIV trans-activation response (TAR) RNA can act as a "decoy" and efficiently binds HIV tat protein, thereby preventing it from binding to TAR sequences encoded in the HIV RNA (Sullenger et al., 1990, Cell, 63, 601-608). This is but a specific example and those in the art will recognize that other embodiments can be readily generated using techniques generally known in the art, see for example Gold et al., 1995, Annu. Rev. Biochem., 64, 763; Brody and Gold, 2000, J. Biotechnol., 74, 5; Sun, 2000, Curr. Opin. Mol. Ther., 2, 100; Kusser, 2000, J. Biotechnol., 74, 27; Hermann and Patel, 2000, Science, 287, 820; and Jayasena, 1999, Clinical Chemistry, 45, 1628. Similarly, a decoy RNA can be designed to bind to a receptor and block the binding of an effector molecule or a decoy RNA can be designed to bind to receptor of interest and prevent interaction with the receptor.

The term "single stranded RNA" (ssRNA) as used herein refers to a naturally occurring or synthetic ribonucleic acid molecule comprising a linear single strand, for example a ssRNA can be a messenger RNA (mRNA), transfer RNA (tRNA), ribosomal RNA (rRNA) etc. of a gene.

The term "single stranded DNA" (ssDNA) as used herein refers to a naturally occurring or synthetic deoxyribonucleic acid molecule comprising a linear single strand, for example, a ssDNA can be a sense or antisense gene sequence or EST (Expressed Sequence Tag).

The term "double stranded RNA" or "dsRNA" as used herein refers to a double stranded RNA molecule capable of RNA interference, including short interfering RNA (siNA).

The term "short interfering nucleic acid", "siNA", "short interfering RNA", "siRNA", "short interfering nucleic acid molecule", "short interfering oligonucleotide molecule", or "chemically-modified short interfering nucleic acid molecule" as used herein refers to any nucleic acid molecule capable of inhibiting or down regulating gene expression or viral replication, for example by mediating RNA interference "RNAi" or gene silencing in a sequence-specific manner; see for example Bass, 2001, Nature, 411, 428-429; Elbashir et al., 2001, Nature, 411, 494-498; and Kreutzer et al., International PCT Publication No. WO 00/44895; Zernicka-Goetz et al., International PCT Publication No. WO 01/36646; Fire, International PCT Publication No. WO 99/32619; Plaetinck et al., International PCT Publication No. WO 00/01846; Mello and Fire, International PCT Publication No. WO 01/29058; Deschamps-Depaillette, International PCT Publication No. WO 99/07409; and Li et al., International PCT Publication No. WO 00/44914; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237; Hutvagner and Zamore, 2002, Science, 297, 2056-60; McManus et al., 2002, RNA, 8, 842-850; Reinhart et al., 2002, Gene & Dev., 16, 1616-1626; and Reinhart & Bartel, 2002, Science, 297, 1831). Non limiting examples of siNA molecules of the invention are described in Haeberli et al., PCT/US03/05346 and McSwiggen et al., PCT/US03/05028, and in **Figures 34-42** herein. Chemical modifications described in Haeberli et al., PCT/US03/05346 and McSwiggen et al., PCT/US03/05028 and/or shown in **Table IV** can be applied to any siNA sequence of the invention. For example the siNA can be a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The siNA can be assembled from two separate oligonucleotides, where one strand is the sense strand and the other is the antisense strand, wherein the antisense and sense strands are self-complementary (i.e. each strand comprises nucleotide sequence that is complementary to nucleotide sequence in the other strand; such as where the antisense strand and sense strand form a duplex or double stranded structure, for example wherein the double stranded region is about 19 base pairs); the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof. Alternatively, the siNA is assembled from a single oligonucleotide, where the self-complementary sense and antisense regions of the siNA are linked by means of a nucleic acid based or non-nucleic acid-based linker(s). The siNA can be a polynucleotide with a hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof. The siNA can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siNA molecule capable of mediating RNAi. The siNA can also comprise a single stranded polynucleotide having nucleotide sequence complementary to nucleotide sequence in a target nucleic acid molecule or a portion thereof (for example, where such siNA molecule does not require the presence within the siNA molecule of nucleotide sequence corresponding to the target nucleic acid sequence or a portion thereof), wherein the single stranded polynucleotide can further comprise a terminal phosphate group, such as a 5'-phosphate (see for example Martinez et al., 2002, Cell., 110, 563-574 and Schwarz et al., 2002, Molecular Cell, 10, 537-568), or 5',3'-diphosphate. In certain embodiment, the siNA molecule of the invention comprises separate sense and antisense sequences or regions, wherein the sense and antisense regions are covalently linked by nucleotide or non-nucleotide linkers molecules as is known in the art, or are alternately non-covalently linked by ionic interactions, hydrogen bonding, van der waals interactions, hydrophobic interactions, and/or stacking interactions. In certain embodiments, the siNA molecules of the invention comprise nucleotide sequence that is complementary to nucleotide sequence of a target gene. In another embodiment, the siNA molecule of the invention interacts with nucleotide sequence of a target gene in a manner that causes inhibition of expression of the target gene. As used herein, siNA molecules need not be limited to those molecules containing only RNA, but further encompasses chemically-modified nucleotides and non-nucleotides. In certain embodiments, the short interfering nucleic acid molecules of the invention lack 2'-hydroxy (2'-OH) containing nucleotides. Applicant describes in certain embodiments short interfering nucleic acids that do not require the presence of nucleotides having a 2'-hydroxy group for mediating RNAi and as such, short interfering nucleic acid molecules of the invention optionally do not include any ribonucleotides (e.g., nucleotides having a 2'-OH group). Such siNA molecules that do not require the presence of ribonucleotides within the siNA molecule to support RNAi can however have an attached linker or linkers or other attached or associated groups, moieties, or chains containing one or more nucleotides with 2'-OH groups. Optionally, siNA molecules can comprise ribonucleotides at about 5, 10, 20, 30, 40, or 50% of the nucleotide positions. The modified short interfering nucleic acid molecules of the invention can also be referred to as short interfering modified oligonucleotides "siMON." As used herein, the term siNA is meant to be equivalent to other terms used to describe nucleic acid molecules that are capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), translational silencing, and others. In addition, as used herein, the term RNAi is meant to be equivalent to other terms used to describe sequence specific RNA interference, such as post transcriptional gene silencing, or epigenetics. For example, siNA molecules of the invention can be used to epigenetically silence genes at both the post-transcriptional level or the pre-transcriptional level. In a non-limiting example, epigenetic regulation of gene expression by siNA molecules of the invention can result from siNA mediated modification of chromatin structure to alter gene expression (see, for example, Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237).

The term "allozyme" as used herein refers to an allosteric enzymatic nucleic acid molecule, see for example see for example George et al., US Patent Nos. 5,834,186 and 5,741,679, Shih et al., US Patent No. 5,589,332, Nathan et al., US Patent No 5,871,914, Nathan and Ellington, International PCT publication No. WO 00/24931, Breaker et al., International PCT Publication Nos. WO 00/26226 and 98/27104, and Sullenger et al., International PCT publication No. WO 99/29842.

The term "cell" as used herein, refers to its usual biological sense, and does not refer to an entire multicellular organism. The cell can, for example, be in vitro, e.g., in cell culture, or present in a multicellular organism, including" e.g., birds, plants and mammals such as humans, cows, sheep, apes, monkeys, swine, dogs, and cats. The cell can be prokaryotic (e.g., bacterial cell) or eukaryotic (e.g., mammalian or plant cell).

The term "highly conserved sequence region" as used herein, refers to a nucleotide sequence of one or more regions in a target gene does not vary significantly from one generation to the other or from one biological system to the other.

The term "non-nucleotide" as used herein, refers to any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine.

The term "nucleotide" as used herein, refers to a heterocyclic nitrogenous base in N-glycosidic linkage with a phosphorylated sugar. Nucleotides are recognized in the art to include natural bases (standard), and modified bases well known in the art. Such bases are generally located at the 1' position of a nucleotide sugar moiety. Nucleotides generally comprise a base, sugar and a phosphate group. The nucleotides can be unmodified or modified at the sugar, phosphate and/or base moiety, (also referred to interchangeably as nucleotide analogs, modified nucleotides, non-natural nucleotides, non-standard nucleotides and other; see for example, Usman and McSwiggen, supra; Eckstein et al., International PCT Publication No. WO 92/07065; Usman et al., International PCT Publication No. WO 93/15187; Uhlman & Peyman). There are several examples of modified nucleic acid bases known in the art as summarized by Limbach et al., 1994, Nucleic Acids Res. 22, 2183. Some of the non-limiting examples of chemically modified and other natural nucleic acid bases that can be introduced into nucleic acids include, for example, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), propyne, quesosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine, beta-D-galactosylqueosine, 1-methyladenosine, 1-methylinosine, 2,2-dimethylguanosine, 3-methylcytidine, 2-methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueosine, uridine-5-oxyacetic acid, 2-thiocytidine, threonine derivatives and others (Burgin et al., 1996, Biochemistry, 35, 14090; Uhlman & Peyman, supra). By "modified bases" in this aspect is meant nucleotide bases other than adenine, guanine, cytosine and uracil at 1' position or their equivalents; such bases can be used at any position, for example, within the catalytic core of an enzymatic nucleic acid molecule and/or in the substrate-binding regions of the nucleic acid molecule.

The term "nucleoside" as used herein, refers to a heterocyclic nitrogenous base in N-glycosidic linkage with a sugar. Nucleosides are recognized in the art to include natural bases (standard), and modified bases well known in the art. Such bases are generally located at the 1' position of a nucleoside sugar moiety. Nucleosides generally comprise a base and sugar group. The nucleosides can be unmodified or modified at the sugar, and/or base moiety, (also referred to interchangeably as nucleoside analogs, modified nucleosides, non-natural nucleosides, non-standard nucleosides and other; see for example, Usman and McSwiggen, supra; Eckstein et al., International PCT Publication No. WO 92/07065; Usman et al., International PCT Publication No. WO 93/15187; Uhlman & Peyman). There are several examples of modified nucleic acid bases known in the art as summarized by Limbach ef al., 1994, Nucleic Acids Res. 22, 2183. Some of the non-limiting examples of chemically modified and other natural nucleic acid bases that can be introduced into nucleic acids include, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), propyne, quesosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine, beta-D-galactosylqueosine, 1-methyladenosine, 1-xnethylinosine, 2,2-dimethylguanosine, 3-methylcytidine, 2-methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueosine, uridine-5-oxyacetic acid, 2-thiocytidine, threonine derivatives and others (Burgin et al., 1996, Biochemistry, 35, 14090; Uhlman & Peyman, supra). By "modified bases" in this aspect is meant nucleoside bases other than adenine, guanine, cytosine and uracil at 1' position or their equivalents; such bases can be used at any position, for example, within the catalytic core of an enzymatic nucleic acid molecule and/or in the substrate-binding regions of the nucleic acid molecule.

The term "cap structure" as used herein, refers to chemical modifications, which have been incorporated at either terminus of the oligonucleotide (see for example Wincott et al., WO 97/26270). These terminal modifications protect the nucleic acid molecule from exonuclease degradation, and can help in delivery and/or localization within a cell. The cap can be present at the 5'-terminus (5'-cap) or at the 3'-terminus (3'-cap) or can be present on both terminus. In non-limiting examples, the 5'-cap includes inverted abasic residue (moiety), 4',5'-methylene nucleotide; 1-(beta-D-erythrofuranosyl) nucleotide, 4'-thio nucleotide, carbocyclic nucleotide; 1,5-anhydrohexitol nucleotide; L-nucleotides; alpha-nucleotides; modified base nucleotide; phosphorodithioate linkage; *threo*-pentofuranosyl nucleotide; acyclic 3',4'-seco nucleotide; acyclic 3,4-dihydroxybutyl nucleotide; acyclic 3,5-dihydroxypentyl nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety (for more details see Wincott et al., International PCT publication No. WO 97/26270).

The term "abasic" as used herein, refers to sugar moieties lacking a base or having other chemical groups in place of a base at the 1' position, for example a 3',3'-linked or 5',5'-linked deoxyabasic ribose derivative (for more details see Wincott et al., International PCT publication No. WO 97/26270).

The term "unmodified nucleoside" as used herein, refers to one of the bases adenine, cytosine, guanine, thymine, uracil joined to the 1' carbon of β-D-ribo-furanose.

The term "modified nucleoside" as used herein, refers to any nucleotide base which contains a modification in the chemical structure of an unmodified nucleotide base, sugar and/or phosphate.

The term "consists essentially of' as used herein, is meant that the active nucleic acid molecule of the invention, for example, an enzymatic nucleic acid molecule, contains an enzymatic center or core equivalent to those in the examples, and binding arms able to bind RNA such that cleavage at the target site occurs. Other sequences can be present which do not interfere with such cleavage. Thus, a core region can, for example, include one or more loop, stem-loop structure, or linker which does not prevent enzymatic activity. For example, a core sequence for a hammerhead enzymatic nucleic acid can comprise a conserved sequence, such as 5'-CUGAUGAG-3' and 5'-CGAA-3' connected by "X", where X is 5'-GCCGUUAGGC-3' (SEQ ID NO 1), or any other Stem II region known in the art, or a nucleotide and/or non-nucleotide linker. Similarly, for other nucleic acid molecules of the instant invention, such as Inozyme, G-cleaver, amberzyme, zinzyme, DNAzyme, antisense, 2-5A antisense, triplex forming nucleic acid, and decoy nucleic acids, other sequences or non-nucleotide linkers can be present that do not interfere with the function of the nucleic acid molecule.

Sequence X can be a linker of ≥ 2 nucleotides in length, preferably 3, 4, 5, 6, 7, 8, 9, 10, 15, 20, 26, 30, where the nucleotides can preferably be internally base-paired to form a stem of preferably ≥ 2 base pairs. In yet another embodiment, the nucleotide linker X can be a nucleic acid aptamer, such as an ATP aptamer, HIV Rev aptamer (RRE), HIV Tat aptamer (TAR) and others (for a review see Gold et al., 1995, Annu. Rev. Biochem., 64, 763; and Szostak & Ellington, 1993, in The RNA World, ed. Gesteland and Atkins, pp. 511, CSH Laboratory Press). A "nucleic acid aptamer" as used herein is meant to indicate a nucleic acid sequence capable of interacting with a ligand. The ligand can be any natural or a synthetic molecule, including but not limited to a resin, metabolites, nucleosides, nucleotides, drugs, toxins, transition state analogs, peptides, lipids, proteins, amino acids, nucleic acid molecules, hormones, carbohydrates, receptors, cells, viruses, bacteria and others.

Alternatively or in addition, sequence X can be a non-nucleotide linker. Non-nucleotides can include abasic nucleotide, polyether, polyamine, polyamide, peptide, carbohydrate, lipid, or polyhydrocarbon compounds. Specific examples include those described by Seela and Kaiser, Nucleic Acids Res. 1990, 18:6353 and Nucleic Acids Res. 1987, 15:3113; Cload and Schepartz, J. Am. Chem. Soc. 1991, 113:6324; Richardson and Schepartz, J. Am. Chem. Soc. 1991, 113:5109; Ma et al., Nucleic Acids Res. 1993, 21:2585 and Biochemistry 1993, 32:1751; Durand et al., Nucleic Acids Res. 1990, 18:6353; McCurdy et al., Nucleosides & Nucleotides 1991, 10:287; Jschke et al., Tetrahedron Lett. 1993, 34:301; Ono et al., Biochemistry 1991, 30:9914; Arnold et al., International Publication No. WO 89/02439; Usman et al., International Publication No. WO 95/06731; Dudycz et al., International Publication No. WO 95/11910 and Ferentz and Verdine, J. Am. Chem. Soc. 1991, 113:4000. A "non-nucleotide" further means any group or compound which can be incorporated into a nucleic acid chain in the place of one or more nucleotide units, including either sugar and/or phosphate substitutions, and allows the remaining bases to exhibit their enzymatic activity. The group or compound can be abasic in that it does not contain a commonly recognized nucleotide base, such as adenosine, guanine, cytosine, uracil or thymine. Thus, described herein is an enzymatic nucleic acid molecule having one or more non-nucleotide moieties, and having enzymatic activity to cleave an RNA or DNA molecule.

The term "patient" as used herein, refers to an organism, which is a donor or recipient of explanted cells or the cells themselves. "Patent" also refers to an organism to which the nucleic acid molecules of the invention can be administered. Preferably, a patient is a mammal or mammalian cells. More preferably, a patient is a human or human cells.

The term "enhanced enzymatic activity" as used herein, includes activity measured in cells and/or in vivo where the activity is a reflection of both the catalytic activity and the stability of the nucleic acid molecules of the invention. In this invention, the product of these properties can be increased in vivo compared to an all RNA enzymatic nucleic acid or all DNA enzyme. In some cases, the activity or stability of the nucleic acid molecule can be decreased (i.e., less than ten-fold), but the overall activity of the nucleic acid molecule is enhanced, in vivo.

By "comprising" is meant including, but not limited to, whatever follows the word "comprising". Thus, use of the term "comprising" indicates that the listed elements are required or mandatory, but that other elements are optional and can or can not be present. By "consisting of" is meant including, and limited to, whatever follows the phrase "consisting of". Thus, the phrase "consisting of' indicates that the listed elements are required or mandatory, and that no other elements can be present.

The term "negatively charged molecules" as used herein, refers to molecules such as nucleic acid molecules (e.g., RNA, DNA, oligonucleotides, mixed polymers, peptide nucleic acid, and the like), peptides (e.g., polyaminoacids, polypeptides, proteins and the
like), nucleotides, pharmaceutical and biological compositions, that have negatively charged groups that can ion-pair with the positively charged head group of the cationic lipids of the invention.

The term "coupling" as used herein, refers to a reaction, either chemical or enzymatic, in which one atom, moiety, group, compound or molecule is joined to another atom, moiety, group, compound or molecule.

The terms "deprotection" or "deprotecting" as used herein, refers to the removal of a protecting group.

The term "alkyl" as used herein refers to a saturated aliphatic hydrocarbon, including straight-chain, branched-chain "isoalkyl", and cyclic alkyl groups. The term "alkyl" also comprises alkoxy, alkyl-thio, alkyl-thio-alkyl, alkoxyalkyl, alkylamino, alkenyl, alkynyl, alkoxy, cycloalkenyl, cycloalkyl, cycloalkylalkyl, heterocycloalkyl, heteroaryl, C1-C6 hydrocarbyl, aryl or substituted aryl groups. Preferably, the alkyl group has 1 to 12 carbons. More preferably it is a lower alkyl of from about 1 to about 7 carbons, more preferably about 1 to about 4 carbons. The alkyl group can be substituted or unsubstituted. When substituted the substituted group(s) preferably comprise hydroxy, oxy, thio, amino, nitro, cyano, alkoxy, alkyl-thio, alkyl-thio-alkyl, alkoxyalkyl, alkylamino, silyl, alkenyl, alkynyl, alkoxy, cycloalkenyl, cycloalkyl, cycloalkylalkyl, heterocycloalkyl, heteroaryl, C1-C6 hydrocarbyl, aryl or substituted aryl groups. The term "alkyl" also includes alkenyl groups containing at least one carbon-carbon double bond, including straight-chain, branched-chain, and cyclic groups. Preferably, the alkenyl group has about 2 to about 12 carbons. More preferably it is a lower alkenyl of from about 2 to about 7 carbons, more preferably about 2 to about 4 carbons. The alkenyl group can be substituted or unsubstituted. When substituted the substituted group(s) preferably comprise hydroxy, oxy, thio, amino, nitro, cyano, alkoxy, alkyl-thio, alkyl-thio-alkyl, alkoxyalkyl, alkylamino, silyl, alkenyl, alkynyl, alkoxy, cycloalkenyl, cycloalkyl, cycloalkylalkyl, heterocycloalkyl, heteroaryl, C1-C6 hydrocarbyl, aryl or substituted aryl groups. The term "alkyl" also includes alkynyl groups containing at least one carbon-carbon triple bond, including straight-chain, branched-chain, and cyclic groups. Preferably, the alkynyl group has about 2 to about 12 carbons. More preferably it is a lower alkynyl of from about 2 to about 7 carbons, more preferably about 2 to about 4 carbons. The alkynyl group can be substituted or unsubstituted. When substituted the substituted group(s) preferably comprise hydroxy, oxy, thio, amino, nitro, cyano, alkoxy, alkyl-thio, alkyl-thio-alkyl, alkoxyalkyl, alkylamino, silyl, alkenyl, alkynyl, alkoxy, cycloalkenyl, cycloalkyl, cycloalkylalkyl, heterocycloalkyl, heteroaryl, C1-C6 hydrocarbyl, aryl or substituted aryl groups. Alkyl groups or moieties of the invention can also include aryl, alkylaryl, carbocyclic aryl, heterocyclic aryl, amide and ester groups. The preferred substituent(s) of aryl groups are halogen, trihalomethyl, hydroxyl, SH, OH, cyano, alkoxy, alkyl, alkenyl, alkynyl, and amino groups. An "alkylaryl" group refers to an alkyl group (as described above) covalently joined to an aryl group (as described above). Carbocyclic aryl groups are groups wherein the ring atoms on the aromatic ring are all carbon atoms. The carbon atoms are optionally substituted. Heterocyclic aryl groups are groups having from about 1 to about 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, N-lower alkyl pyrrolo, pyrimidyl, pyrazinyl, imidazolyl and the like, all optionally substituted. An "amide" refers to an -C(O)-NH-R, where R is either alkyl, aryl, alkylaryl or hydrogen. An "ester" refers to an -C(O)-OR', where R is either alkyl, aryl, alkylaryl or hydrogen.

The term "alkoxyalkyl" as used herein refers to an alkyl-O-alkyl ether, for example, methoxyethyl or ethoxymethyl.

The term "alkyl-thio-alkyl" as used herein refers to an alkyl-S-alkyl thioether, for example, methylthiomethyl or methylthioethyl.

The term "amino" as used herein refers to a nitrogen containing group as is known in the art derived from ammonia by the replacement of one or more hydrogen radicals by organic radicals. For example, the terms "aminoacyl" and "aminoalkyl" refer to specific N-substituted organic radicals with acyl and alkyl substituent groups respectively.

The term "amination" as used herein refers to a process in which an amino group or substituted amine is introduced into an organic molecule.

The term "exocyclic amine protecting moiety" as used herein refers to a nucleobase amino protecting group compatible with oligonucleotide synthesis, for example, an acyl or amide group.

The term "alkenyl" as used herein refers to a straight or branched hydrocarbon of a designed number of carbon atoms containing at least one carbon-carbon double bond. Examples of "alkenyl" include vinyl, allyl, and 2-methyl-3-heptene.

The term "alkoxy" as used herein refers to an alkyl group of indicated number of carbon atoms attached to the parent molecular moiety through an oxygen bridge. Examples of alkoxy groups include, for example, methoxy, ethoxy, propoxy and isopropoxy.

The term "alkynyl" as used herein refers to a straight or branched hydrocarbon of a designed number of carbon atoms containing at least one carbon-carbon triple bond. Examples of "alkynyl" include propargyl, propyne, and 3-hexyne.

The term "aryl" as used herein refers to an aromatic hydrocarbon ring system containing at least one aromatic ring. The aromatic ring can optionally be fused or otherwise attached to other aromatic hydrocarbon rings or non-aromatic hydrocarbon rings. Examples of aryl groups include, for example, phenyl, naphthyl, 1,2,3,4-tetrahydronaphthalene and biphenyl. Preferred examples of aryl groups include phenyl and naphthyl.

The term "cycloalkenyl" as used herein refers to a C3-C8 cyclic hydrocarbon containing at least one carbon-carbon double bond. Examples of cycloalkenyl include cyclopropenyl, cyclobutenyl, cyclopentenyl, cyclopentadiene, cyclohexenyl, 1,3-cyclohexadiene, cycloheptenyl, cycloheptatrienyl, and cyclooctenyl.

The term "cycloalkyl" as used herein refers to a C3-C8 cyclic hydrocarbon. Examples of cycloalkyl include cyclopropyl, cyclobutyl, cyclopentyl, cyclohexyl, cycloheptyl and cyclooctyl.

The term "cycloalkylalkyl," as used herein, refers to a C3-C7 cycloalkyl group attached to the parent molecular moiety through an alkyl group, as defined above. Examples of cycloalkylalkyl groups include cyclopropylmethyl and cyclopentylethyl.

The terms "halogen" or "halo" as used herein refers to indicate fluorine, chlorine, bromine, and iodine.

The term "heterocycloalkyl," as used herein refers to a non-aromatic ring system containing at least one heteroatom selected from nitrogen, oxygen, and sulfur. The heterocycloalkyl ring can be optionally fused to or otherwise attached to other heterocycloalkyl rings and/or non-aromatic hydrocarbon rings. Preferred heterocycloalkyl groups have from 3 to 7 members. Examples of heterocycloalkyl groups include, for example, piperazine, morpholine, piperidine, tetrahydrofuran, pyrrolidine, and pyrazole. Preferred heterocycloalkyl groups include piperidinyl, piperazinyl, rnorpholinyl, and pyrolidinyl.

The term "heteroaryl" as used herein refers to an aromatic ring system containing at least one heteroatom selected from nitrogen, oxygen, and sulfur. The heteroaryl ring can be fused or otherwise attached to one or more heteroaryl rings, aromatic or non-aromatic hydrocarbon rings or heterocycloalkyl rings. Examples of heteroaryl groups include, for example, pyridine, furan, thiophene, 5,6,7,8-tetrahydroisoquinoline and pyrimidine. Preferred examples of heteroaryl groups include thienyl, benzothienyl, pyridyl, quinolyl, pyrazinyl, pyrimidyl, imidazolyl, benzimidazolyl, furanyl, benzofuranyl, thiazolyl, benzothiazolyl, isoxazolyl, oxadiazolyl, isothiazolyl, benzisothiazolyl, triazolyl, tetrazolyl, pyrrolyl, indolyl, pyrazolyl, and benzopyrazolyl.

The term "C1-C6 hydrocarbyl" as used herein refers to straight, branched, or cyclic alkyl groups having 1-6 carbon atoms, optionally containing one or more carbon-carbon double or triple bonds. Examples of hydrocarbyl groups include, for example, methyl, ethyl, propyl, isopropyl, n-butyl, sec-butyl, tert-butyl, pentyl, 2-pentyl, isopentyl, neopentyl, hexyl, 2-hexyl, 3-hexyl, 3-methylpentyl, vinyl, 2-pentene, cyclopropylmethyl, cyclopropyl, cyclohexylmethyl, cyclohexyl and propargyl. When reference is made herein to C1-C6 hydrocarbyl containing one or two double or triple bonds it is understood that at least two carbons are present in the alkyl for one double or triple bond, and at least four carbons for two double or triple bonds.

The term "protecting group" as used herein, refers to groups known in the art that are readily introduced and removed from an atom, for example O, N, P, or S. Protecting groups are used to prevent undesirable reactions from taking place that can compete with the formation of a specific compound or intermediate of interest. See also "Protective Groups in Organic Synthesis", 3rd Ed., 1999, Greene, T. W. and related publications.

The term "nitrogen protecting group," as used herein, refers to groups known in the art that are readily introduced on to and removed from a nitrogen. Examples of nitrogen protecting groups include Boc, Cbz, benzoyl, and benzyl. See also "Protective Groups in Organic Synthesis", 3rd Ed., 1999, Greene, T. W. and related publications.

The term "hydroxy protecting group," or "hydroxy protection" as used herein, refers to groups known in the art that are readily introduced on to and removed from an oxygen, specifically an -OH group. Examples of hyroxy protecting groups include trityl or substituted trityl goups, such as monomethoxytrityl and dimethoxytrityl, or substituted silyl groups, such as tert-butyldimethyl, trimethylsilyl, or tert-butyldiphenyl silyl groups. See also "Protective Groups in Organic Synthesis", 3rd Ed., 1999, Greene, T. W. and related publications.

The term "acyl" as used herein refers to -C(O)R groups, wherein R is an alkyl or aryl.

The term "phosphorus containing group" as used herein, refers to a chemical group containing a phosphorus atom. The phosphorus atom can be trivalent or pentavalent, and can be substituted with O, H, N, S, C or halogen atoms. Examples of phosphorus containing groups of the instant invention include but are not limited to phosphorus atoms substituted with O, H, N, S, C or halogen atoms, comprising phosphonate, alkylphosphonate, phosphate, diphosphate, triphosphate, pyrophosphate, phosphorothioate, phosphorodithioate, phosphoramidate, phosphoramidite groups, nucleotides and nucleic acid molecules.

The term "phosphine" or "phosphite" as used herein refers to a trivalent phosphorus species, for example compounds having Formula 97: 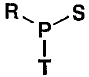 wherein R can include the groups: 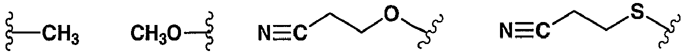 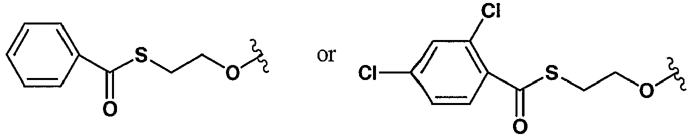 and wherein S and T independently include the groups:

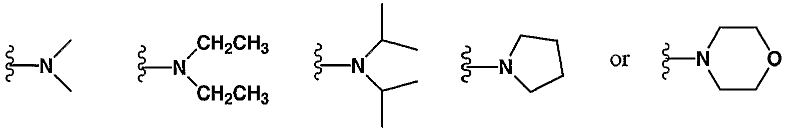

The term "phosphate" as used herein refers to a pentavalent phosphorus species, for example a compound having Formula 98: 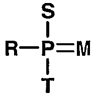 wherein R includes the groups: 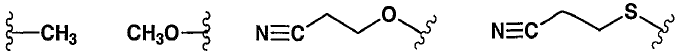 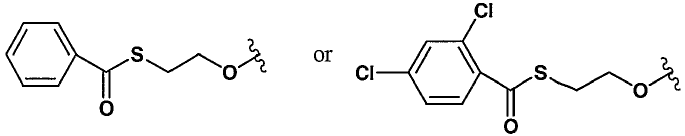 and wherein S and T each independently can be a sulfur or oxygen atom or a group which can include: 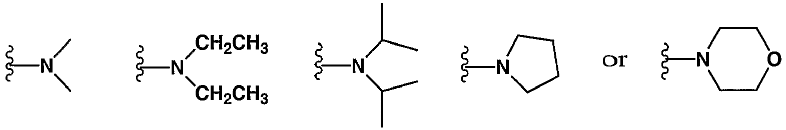 and wherein M comprises a sulfur or oxygen atom. The phosphate of the invention can comprise a nucleotide phosphate, wherein any R, S, or T in Formula 98 comprises a linkage to a nucleic acid or nucleoside.

The term "cationic salt" as used herein refers to any organic or inorganic salt having a net positive charge, for example a triethylammonium (TEA) salt.

The term "degradable linker" as used herein, refers to linker moieties that are capable of cleavage under various conditions. Conditions suitable for cleavage can include but are not limited to pH, UV irradiation, enzymatic activity, temperature, hydrolysis, elimination, and substitution reactions, and thermodynamic properties of the linkage.

The term "photolabile linker" as used herein, refers to linker moieties as are known in the art, that are selectively cleaved under particular UV wavelengths. Compounds of the invention containing photolabile linkers can be used to deliver compounds to a target cell or tissue of interest, and can be subsequently released in the presence of a UV source.

The term "nucleic acid conjugates" as used herein, refers to nucleoside, nucleotide and oligonucleotide conjugates.

The term "lipid" as used herein, refers to any lipophilic compound. Non-limiting examples of lipid compounds include fatty acids and their derivatives, including straight chain, branched chain, saturated and unsaturated fatty acids, carotenoids, terpenes, bile acids, and steroids, including cholesterol and derivatives or analogs thereof.

The term "folate" as used herein, refers to analogs and derivatives of folic acid, for example antifolates, dihydrofloates, tetrahydrofolates, tetrahydorpterins, folinic acid, pteropolyglutamic acid, 1-deza, 3-deaza, 5-deaza, 8-deaza, 10-deaza, 1,5-deaza, 5,10 dideaza, 8,10-dideaza, and 5,8-dideaza folates, antifolates, and pteroic acid derivatives.

The term "compounds with neutral charge" as used herein, refers to compositions which are neutral or uncharged at neutral or physiological pH. Examples of such compounds are cholesterol and other steroids, cholesteryl hemisuccinate (CHEMS), dioleoyl phosphatidyl choline, distearoylphosphotidyl choline (DSPC), fatty acids such as oleic acid, phosphatidic acid and its derivatives, phosphatidyl serine, polyethylene glycol -conjugated phosphatidylamine, phosphatidylcholine, phosphatidylethanolamine and related variants, prenylated compounds including farnesol, polyprenols, tocopherol, and their modified forms, diacylsuccinyl glycerols, fusogenic or pore forming peptides, dioleoylphosphotidylethanolamine (DOPE), ceramide and the like.

The term "lipid aggregate" as used herein refers to a lipid-containing composition wherein the lipid is in the form of a liposome, micelle (non-lamellar phase) or other aggregates with one or more lipids.

The term "biological system" as used herein, refers to a eukaryotic system or a prokaryotic system, can be a bacterial cell, plant cell or a mammalian cell, or can be of plant origin, mammalian origin, yeast origin, Drosophila origin, or archebacterial origin.

The term "systemic administration" as used herein refers to the in vivo systemic absorption or accumulation of drugs in the blood stream followed by distribution throughout the entire body. Administration routes which lead to systemic absorption include, without limitations: intravenous, subcutaneous, intraperitoneal, inhalation, oral, intrapulmonary and intramuscular. Each of these administration routes expose the desired negatively charged polymers, e.g., nucleic acids, to an accessible diseased tissue. The rate of entry of a drug into the circulation has been shown to be a function of molecular weight or size. The use of a liposome or other drug carrier comprising the compounds of the instant invention can potentially localize the drug, for example, in certain tissue types, such as the tissues of the reticular endothelial system (RES). A liposome formulation which can facilitate the association of drug with the surface of cells, such as, lymphocytes and macrophages is also useful. This approach can provide enhanced delivery of the drug to target cells by taking advantage of the specificity of macrophage and lymphocyte immune recognition of abnormal cells, such as the cancer cells.

The term "pharmacological composition" or "pharmaceutical formulation" refers to a composition or formulation in a form suitable for administration, for example, systemic administration, into a cell or patient, preferably a human. Suitable forms, in part, depend upon the use or the route of entry, for example oral, transdermal, or by injection. Such forms should not prevent the composition or formulation to reach a target cell (i.e., a cell to which the negatively charged polymer is targeted).

Other features and advantages of the invention will be apparent from the following description of the preferred embodiments thereof, and from the claims.

### Description of the Preferred Embodiments

The drawings will be first described briefly.

### Drawings:

**Figure 1** shows examples of chemically stabilized ribozyme motifs. HH Rz, represents hammerhead ribozyme motif (Usman et al., 1996, Curr. Op. Struct. Bio., 1, 527); NCH Rz represents the NCH ribozyme motif (Ludwig & Sproat, International PCT Publication No. WO 98/58058); G-Cleaver, represents G-cleaver ribozyme motif (Kore et al., 1998, Nucleic Acids Research 26, 4116-4120, Eckstein et al., International PCT publication No. WO 99/16871). N or n, represent independently a nucleotide which can be same or different and have complementarity to each other; rI, represents ribo-Inosine nucleotide; arrow indicates the site of cleavage within the target. Position 4 of the HH Rz and the NCH Rz is shown as having 2'-C-allyl modification, but those skilled in the art will recognize that this position can be modified with other modifications well known in the art, so long as such modifications do not significantly inhibit the activity of the ribozyme.
**Figure 2** shows an example of the Amberzyme ribozyme motif that is chemically stabilized (see for example Beigelman et al., International PCT publication No. WO 99/55857).
**Figure 3** shows an example of the Zinzyme A ribozyme motif that is chemically stabilized (see for example Beigelman et al., Beigelman et al., International PCT publication No. WO 99/55857).
**Figure 4** shows an example of a DNAzyme motif described by Santoro et al., 1997, PNAS, 94,4262.
**Figure 5** shows a synthetic scheme for the synthesis of a folate conjugate of the instant invention.

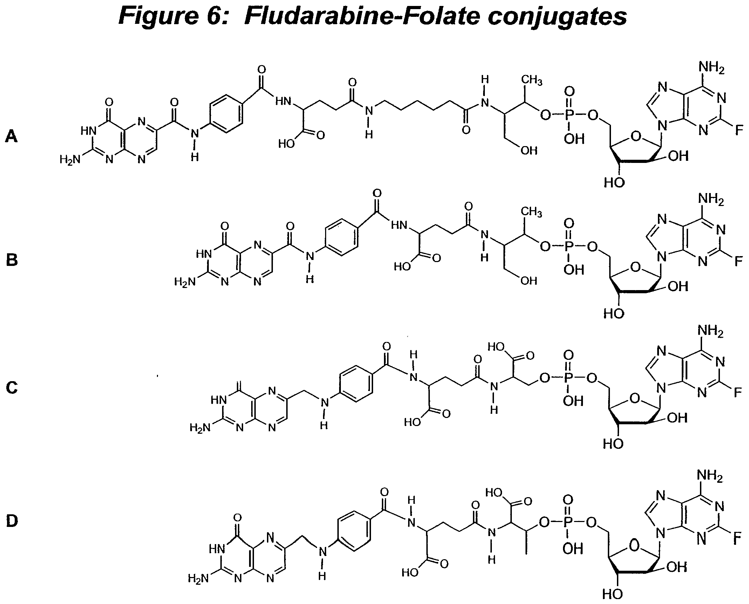

**Figure 6** shows representative examples of fludarabine-folate conjugate molecules of the invention.

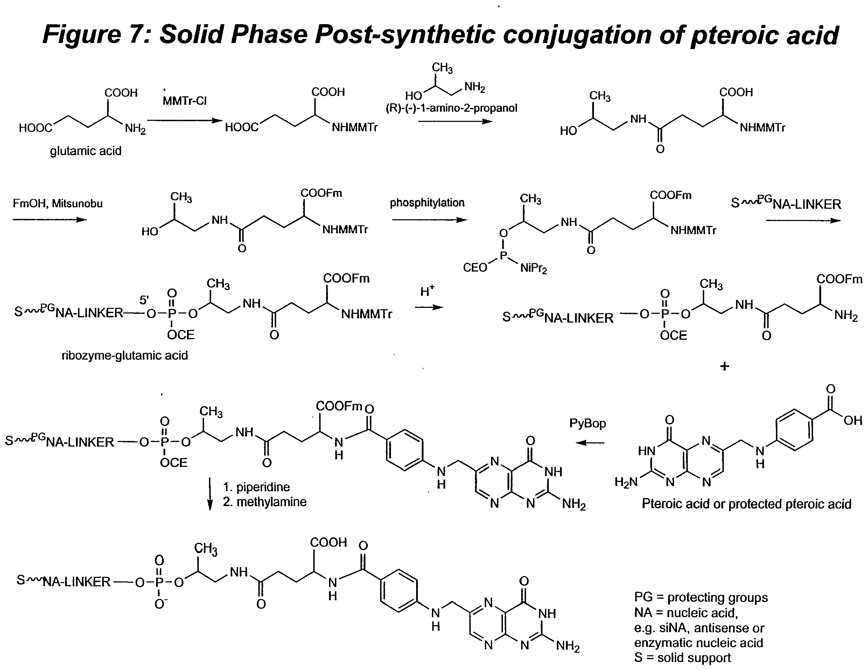

**Figure 7** shows a synthetic scheme for post-synthetic modification of a nucleic acid molecule to produce a folate conjugate.

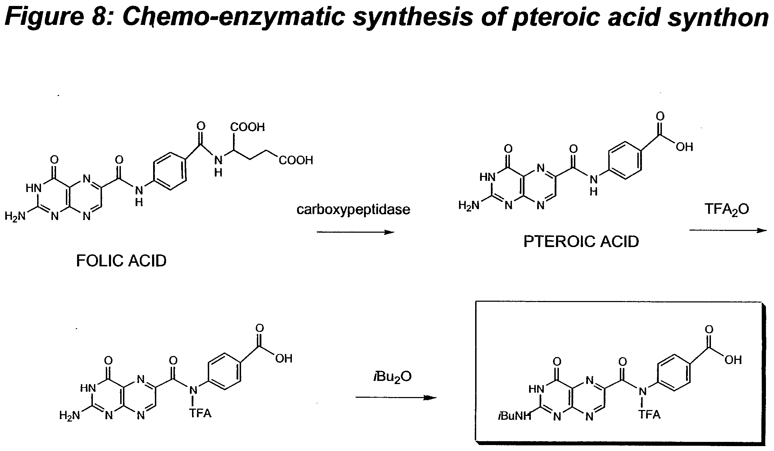

**Figure 8** shows a synthetic scheme for generating a protected pteroic acid synthon of the invention.
**Figure 9** shows a synthetic scheme for generating a 2-dithiopyridyl activated folic acid synthon of the invention.
**Figure 10** shows a synthetic scheme for generating an oligonucleotide or nucleic acid-folate conjugate.
**Figure 11** shows an alternative synthetic scheme for generating an oligonucleotide or nucleic acid-folate conjugate.

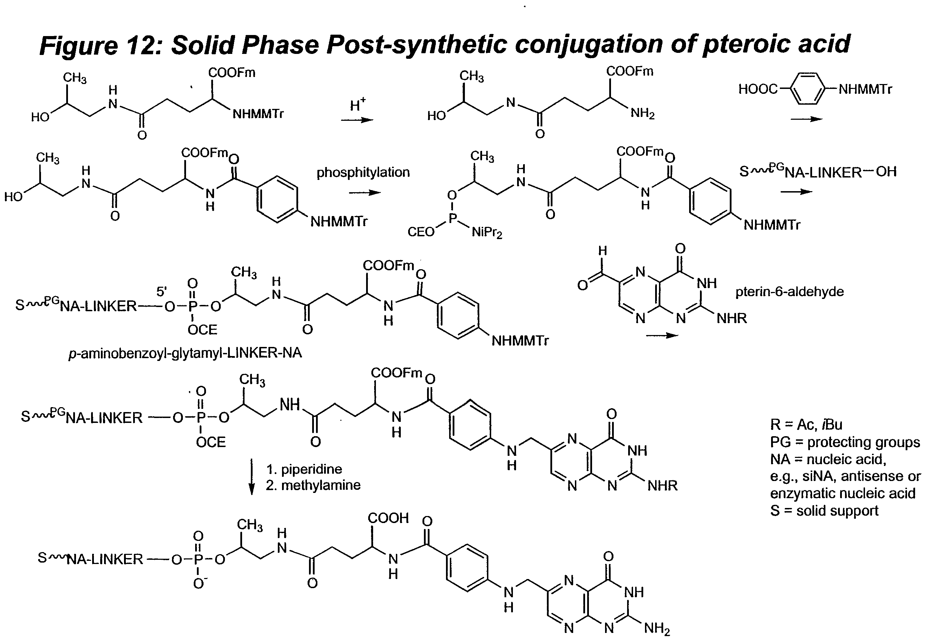

**Figure 12** shows an alternative synthetic scheme for post-synthetic modification of a nucleic acid molecule to produce a folate conjugate.
**Figure 13** shows an example of a synthetic scheme for the synthesis of a N-acetyl-D-galactosamine-2'-aminouridine phosphoramidite conjugate of the invention.
**Figure 14** shows an example of a synthetic scheme for the synthesis of a N-acetyl-D-galactosamine-D-threoninol phosphoramidite conjugate of the invention.
**Figure 15** shows an example of a N-acetyl-D-galactosamine siNA nucleic acid conjugate and a N-acetyl-D-galactosamine enzymatic nucleic acid conjugate of the invention. **W** shown in the example refers to a biodegradable linker, for example a nucleic acid dimer, trimer, or tetramer comprising ribonucleotides and/or deoxyribonucleotides. The siNA can be conjugated at the 3', 5' or both 3' and 5' ends of the sense strand of a double stranded siNA and/or the 3'-end of the antisense strand of the siNA. A single stranded siNA molecule can be conjugated at the 3'-end of the siNA.
**Figure 16** shows an example of a synthetic scheme for the synthesis of a dodecanoic acid derived conjugate linker of the invention.
**Figure 17** shows an example of a synthetic scheme for the synthesis of an oxime linked nucleic acid/peptide conjugate of the invention.
**Figure 18** shows examples of phospholipid derived nucleic acid conjugates of the invention. **W** shown in the examples refers to a biodegradable linker, for example a nucleic acid dimer, trimer, or tetramer comprising ribonucleotides and/or deoxyribonucleotides. The siNA can be conjugated at the 3', 5' or both 3' and 5' ends of the sense strand of a double stranded siNA and/or the 3'-end of the antisense strand of the siNA. A single stranded siNA molecule can be conjugated at the 3'-end of the siNA.
**Figure 19** shows an example of a synthetic scheme for preparing a phospholipid derived siNA conjugates of the invention.
**Figure 20** shows an example of a synthetic scheme for preparing a polyethylene glycol (PEG) derived enzymatic nucleic acid conjugates of the invention.
**Figure 21** shows PK data of a 40K PEG conjugated enzymatic nucleic acid molecule compared to the corresponding non-conjugated enzymatic nucleic acid molecule. The graph is a time course of serum concentration in mice dosed with 30 mg/kg of Angiozyme™ or 40-kDa-PEG-Angiozyme™. The hybridization method was used to quantitate Angiozyme™ levels.
**Figure 22** shows PK data of a phospholipid conjugated enzymatic nucleic acid molecule compared to the corresponding non-conjugated enzymatic nucleic acid molecule.

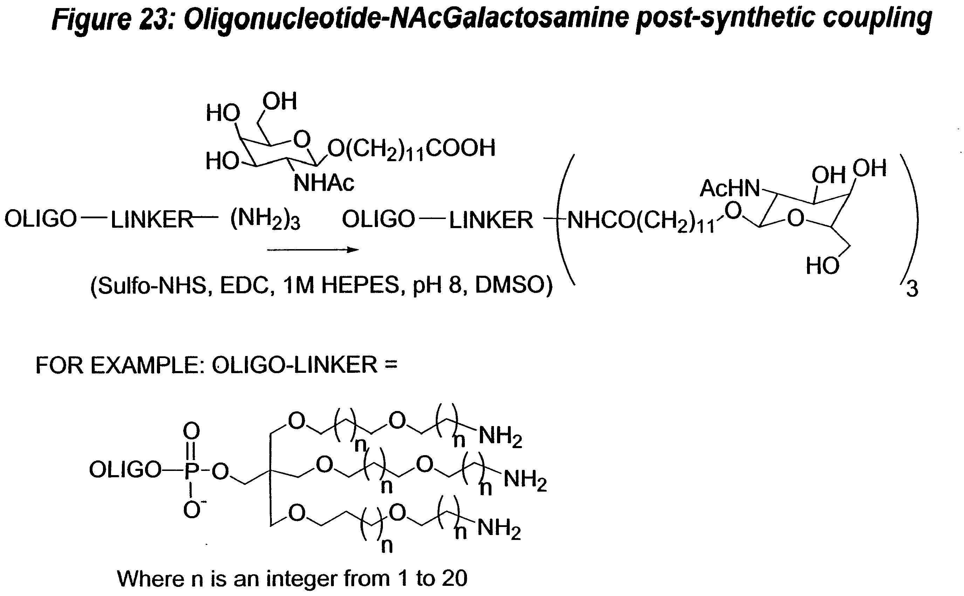

**Figure 23** shows an example of a synthetic scheme for preparing a poly-N-acetyl-D-galactosamine nucleic acid conjugate of the invention.
**Figure 24a****-b** shows an example of a synthetic approach for synthesizing peptide or protein conjugates to PEG utilizing a biodegradable linker using oxime and morpholino linkages.
**Figure 25** shows an example of a synthetic approach for synthesizing peptide or protein conjugates to PEG utilizing a biodegradable linker using oxime and phosphoramidate linkages.
**Figure 26a****-b** shows an example of a synthetic approach for synthesizing peptide or protein conjugates to PEG utilizing a biodegradable linker using phosphoramidate linkages.

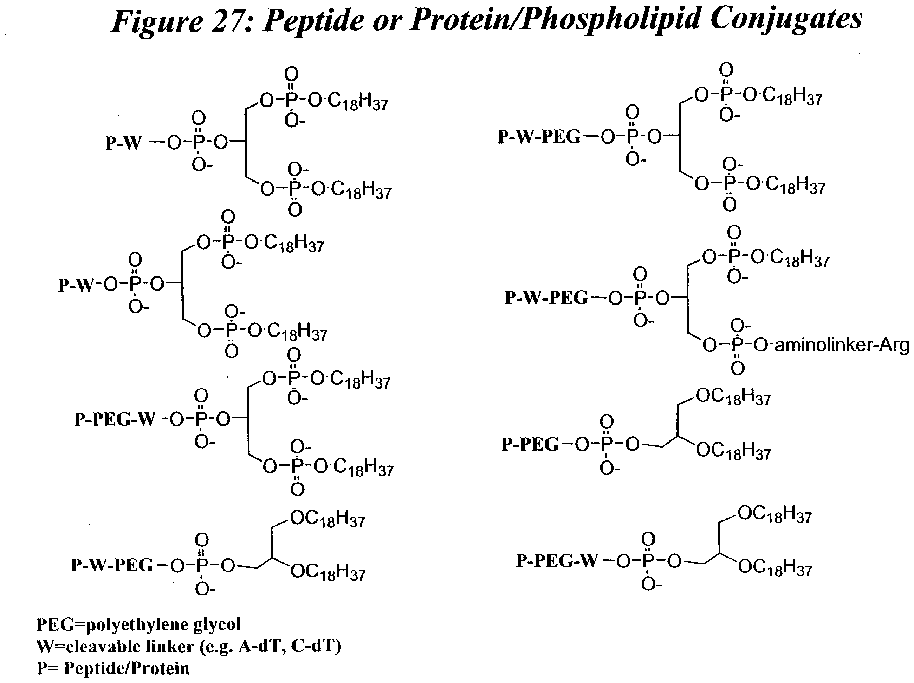

**Figure 27** shows examples of phospholipid derived protein/peptide conjugates of the invention. **W** shown in the examples refers to a biodegradable linker, for example a nucleic acid dimer, trimer, or tetramer comprising ribonucleotides and/or deoxyribonucleotides.

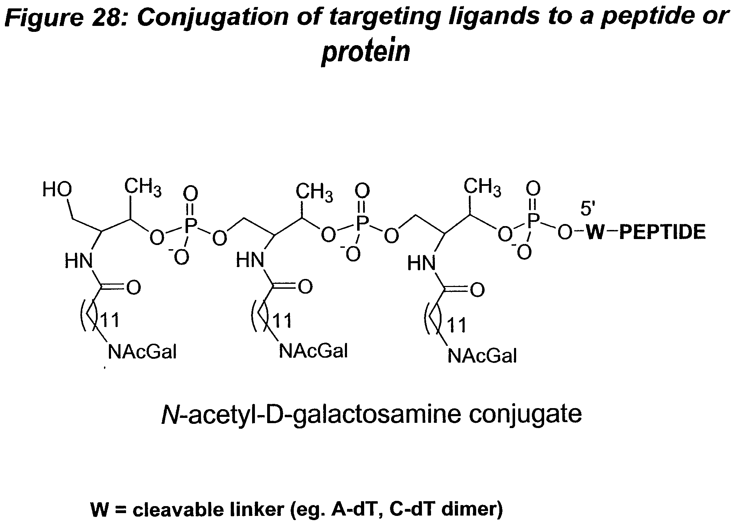

**Figure 28** shows an example of an N-acetyl-D-galactosamine peptide/protein conjugate of the invention, the example shown is with a peptide. **W** shown in the example refers to a biodegradable linker, for example a nucleic acid dimer, trimer, or tetramer comprising ribonucleotides and/or deoxyribonucleotides.
**Figure 29** shows an example of a synthetic approach for synthesizing peptide or protein conjugates to PEG utilizing a biodegradable linker using phosphoramidate linkages via coupling a protein phosphoramidite to a PEG conjugated nucleic acid linker.
**Figure 30** shows an example of the synthesis of siNA cholesterol conjugates of the invention using a phosphoramidite approach.

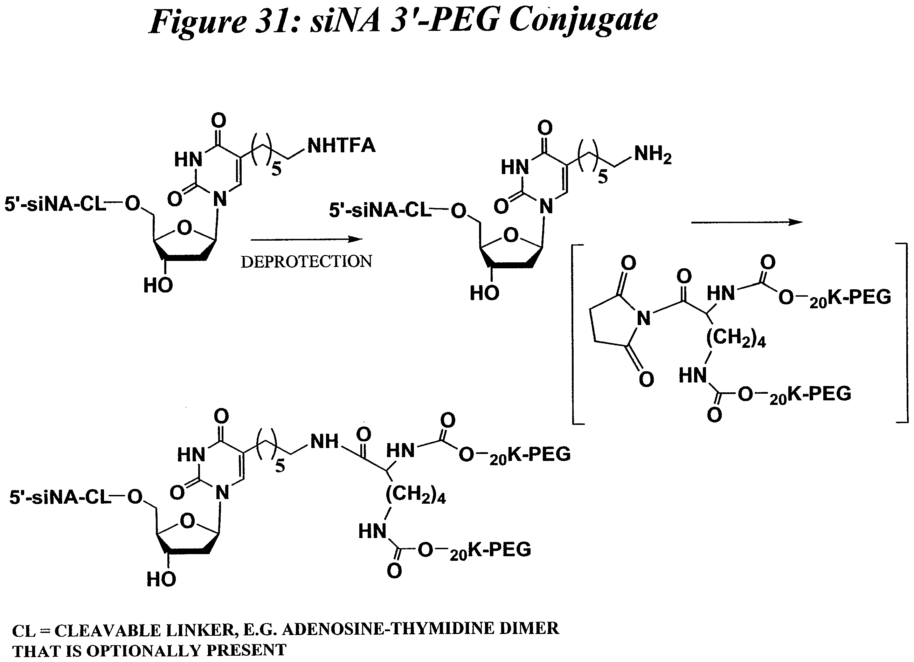

**Figure 31** shows an example of the synthesis of siNA PEG conjugates of the invention using NHS ester coupling.

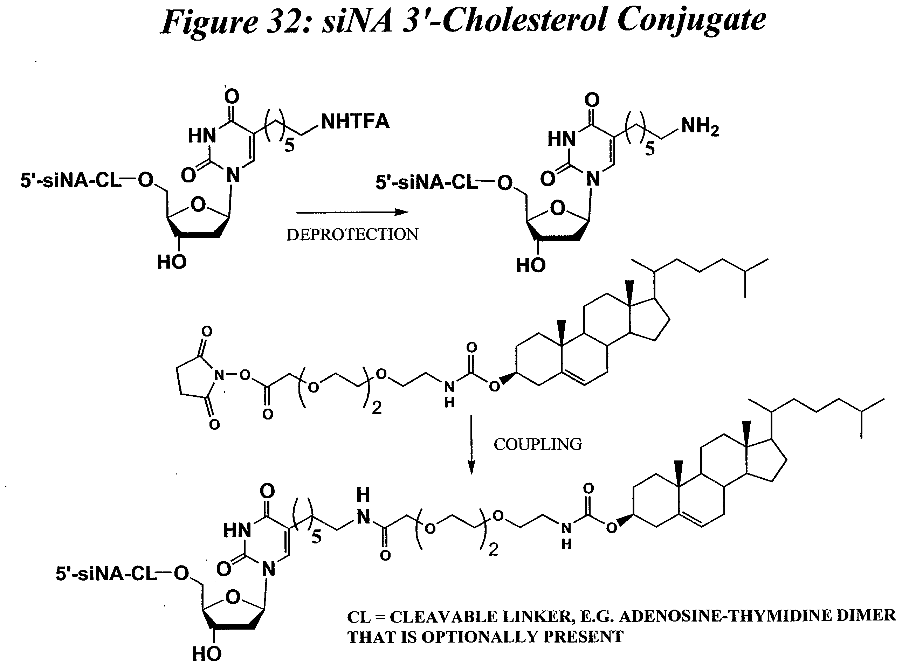

**Figure 32** shows an example of the synthesis of siNA cholesterol conjugates of the invention using NHS ester coupling.

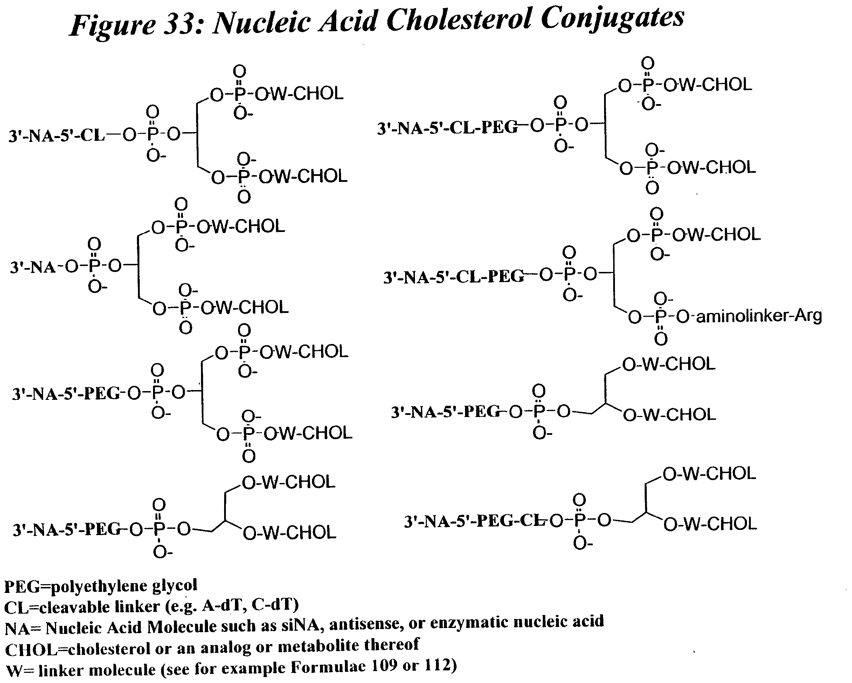

**Figure 33** shows an example of various siNA cholesterol conjugates of the invention.

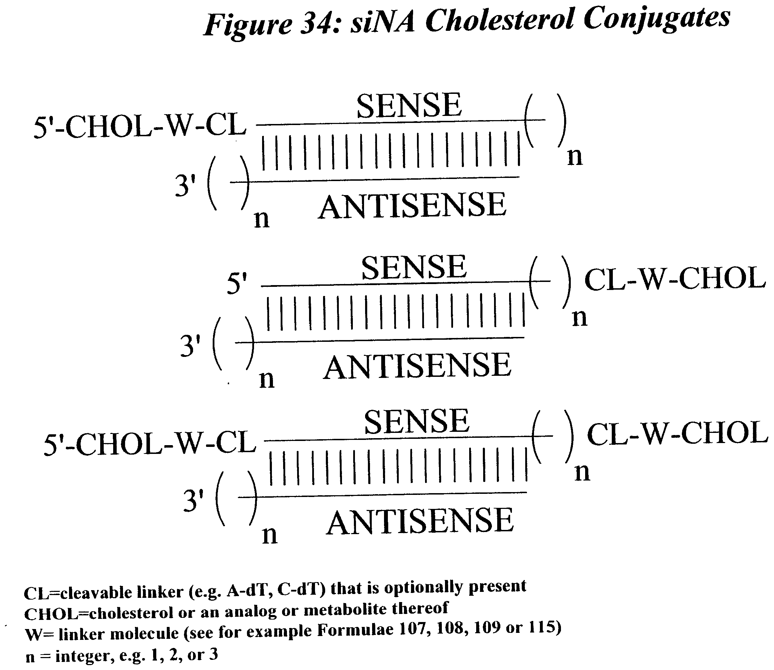

**Figure 34** shows an example of various siNA cholesterol conjugates of the invention in which various linker chemistries and/or cleavable linkers can be utilized at different positions of a double stranded siNA molecule.

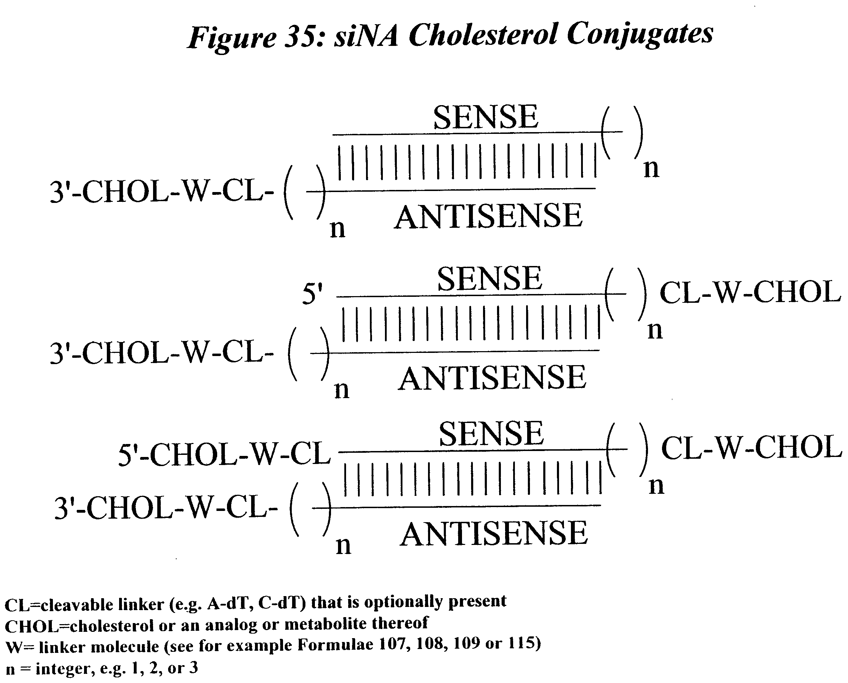

**Figure 35** shows an example of various siNA cholesterol conjugates of the invention in which various linker chemistries and/or cleavable linkers can be utilized at different positions of a double stranded siNA molecule.

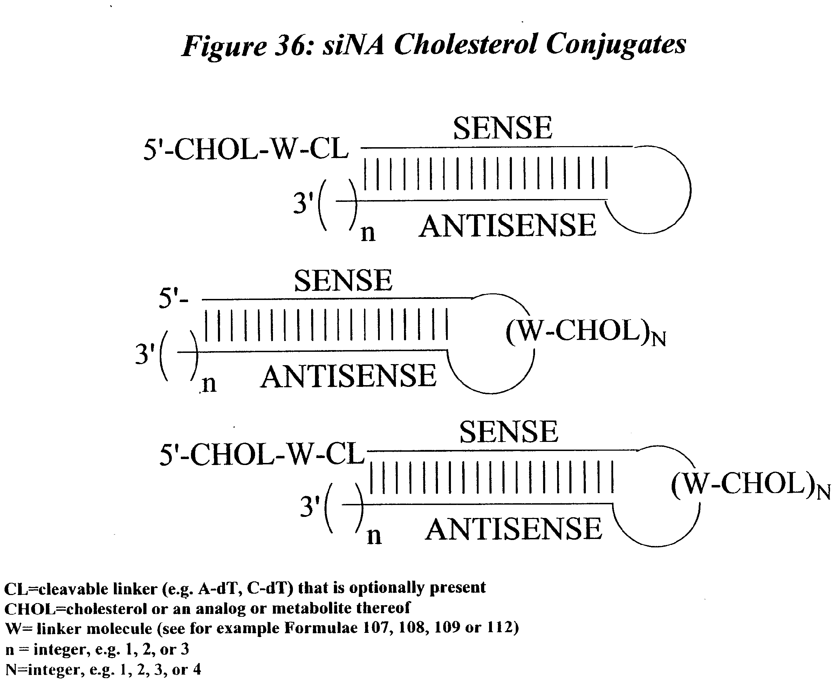

**Figure 36** shows an example of various siNA cholesterol conjugates of the invention in which various linker chemistries and/or cleavable linkers can be utilized at different positions of a single stranded siNA molecule.

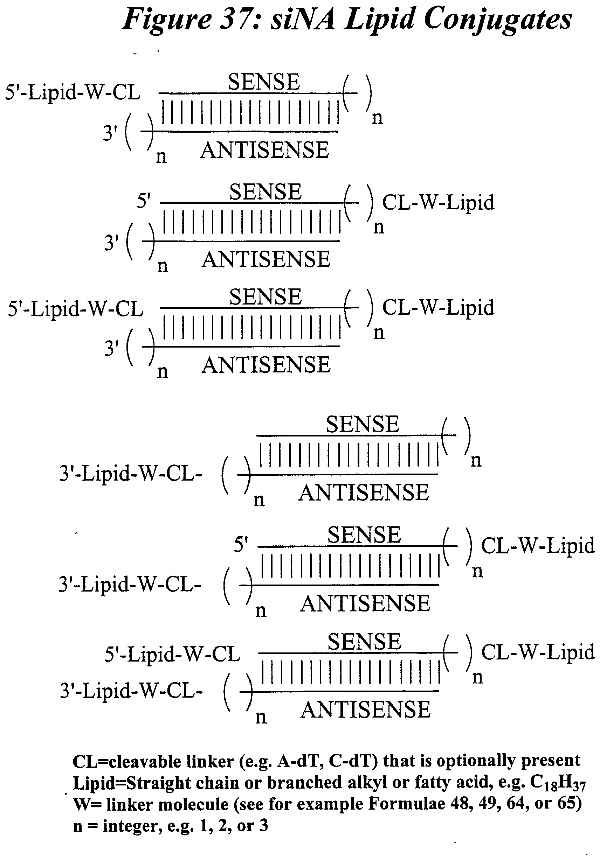

**Figure 37** shows an example of various siNA phospholipid conjugates of the invention in which various linker chemistries and/or cleavable linkers can be utilized at different positions of a double stranded siNA molecule.

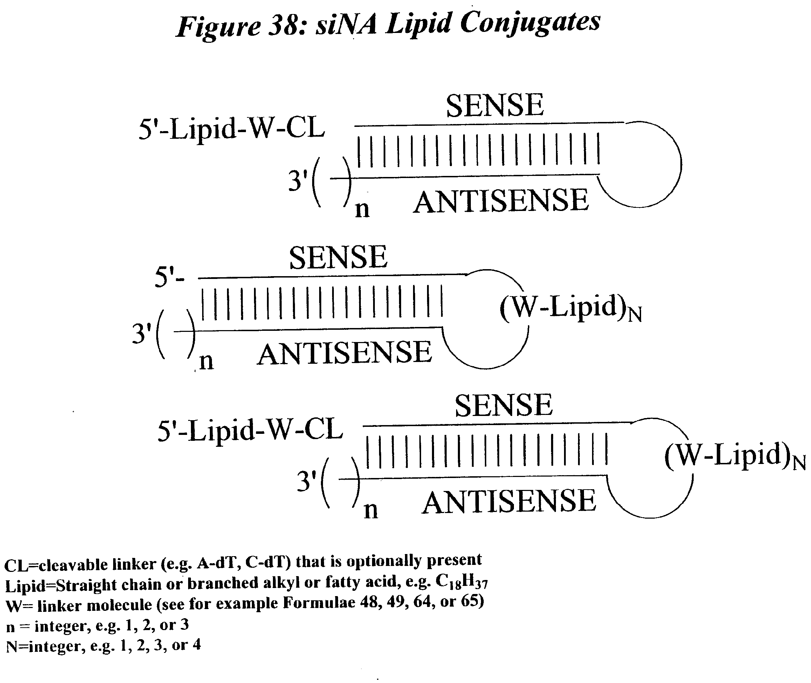

**Figure 38** shows an example of various siNA phospholipid conjugates of the invention in which various linker chemistries and/or cleavable linkers can be utilized at different positions of a single stranded siNA molecule.

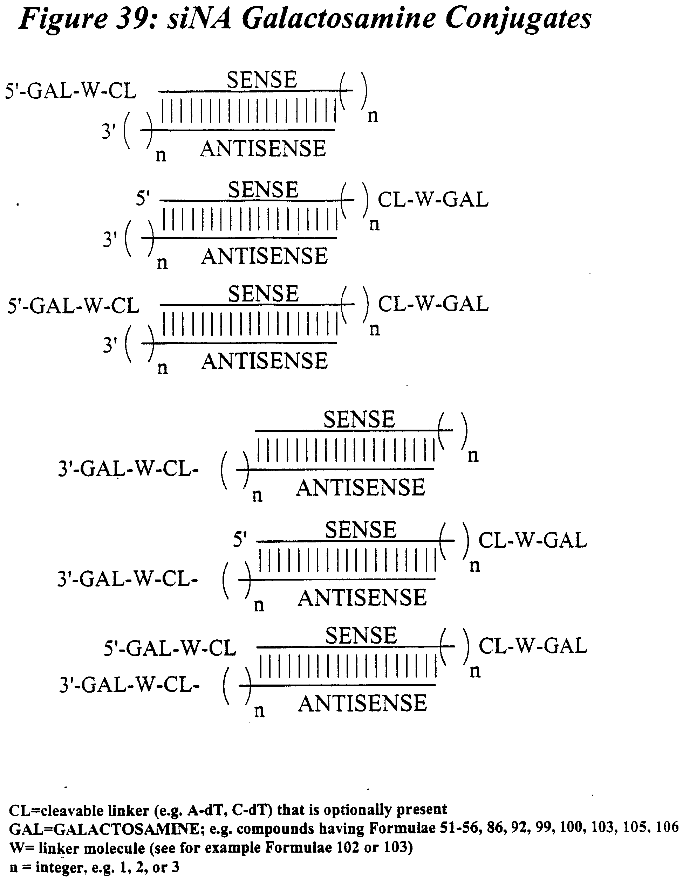

**Figure 39** shows an example of various siNA galactosamine conjugates of the invention in which various linker chemistries and/or cleavable linkers can be utilized at different positions of a double stranded siNA molecule.

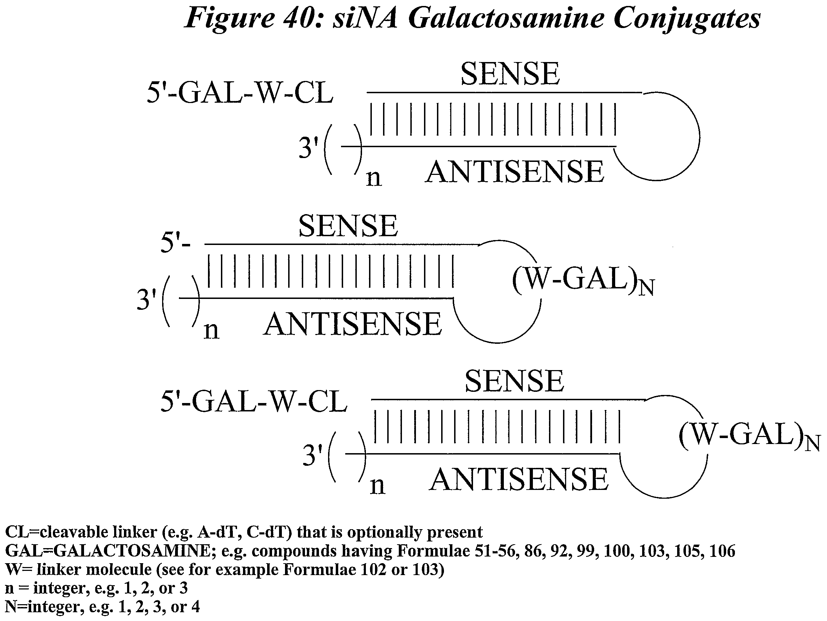

**Figure 40** shows an example of various siNA galactosamine conjugates of the invention in which various linker chemistries and/or cleavable linkers can be utilized at different positions of a single stranded siNA molecule.
**Figure 41** shows an example of various generalized siNA conjugates of the invention in which various linker chemistries and/or cleavable linkers can be utilized at different positions of a double stranded siNA molecule. CONJ in the figure refers to any biologically active compound or any other conjugate compound as described herein and in the Formulae herein.
**Figure 42** shows an example of various generalized siNA conjugates of the invention in which various linker chemistries and/or cleavable linkers can be utilized at different positions of a single stranded siNA molecule. CONJ in the figure refers to any biologically active compound or any other conjugate compound as described herein and in the Formulae herein.
**Figure 43** shows an example of the pharmacokinetic distribution of intact siNA in liver after administration of conjugated or unconjugated siNA molecules in mice.
**Figure 44** shows an example of the activity of conjugated siNA constructs compared to matched chemistry unconjugated siNA constructs in an HBV cell culture system without the use of transfection lipid. As shown in the Figure, siNA conjugates provide efficacy in cell culture without the need for transfection reagent.

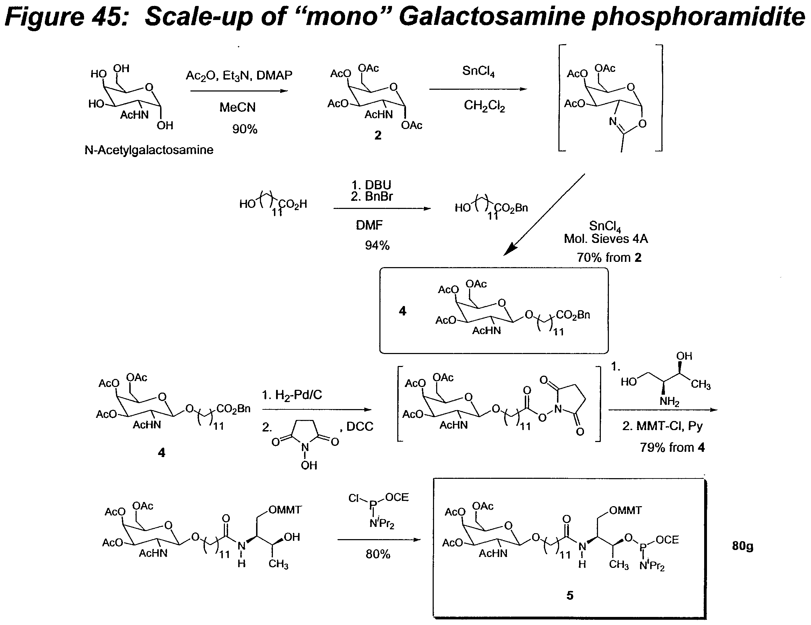

**Figure 45** shows a non-limiting example of a scheme for the synthesis of a mono-galactosamine phosphoramidite of the invention that can be used to generate galactosamine conjugated nucleic acid molecules.

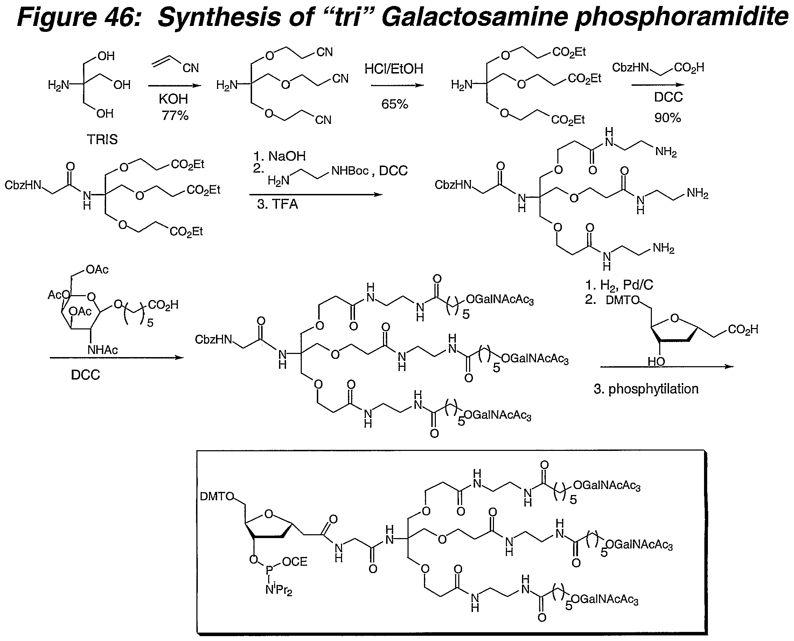

**Figure 46** shows a non-limiting example of a scheme for the synthesis of a tri-galactosamine phosphoramidite of the invention that can be used to generate tri-galactosamine conjugated nucleic acid molecules.

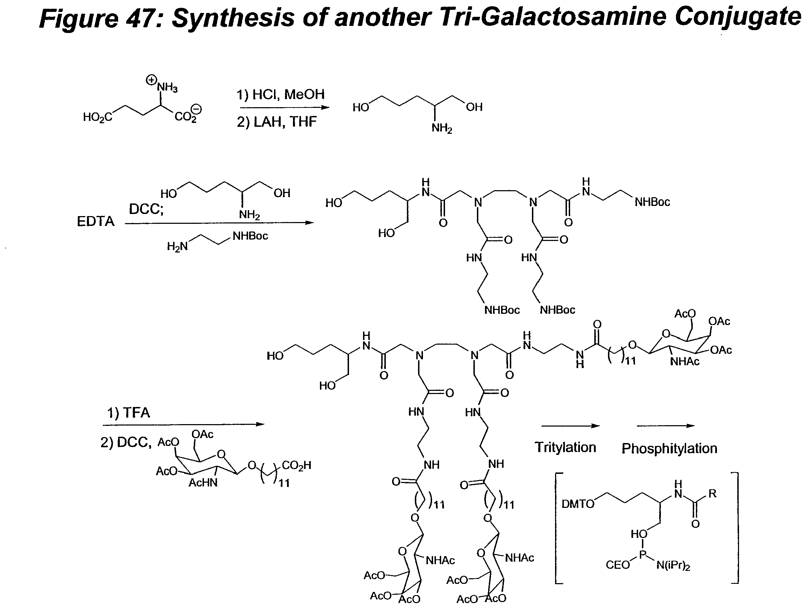

**Figure 47** shows a non-limiting example of a scheme for the synthesis of another tri-galactosamine phosphoramidite of the invention that can be used to generate tri-galactosamine conjugated nucleic acid molecules.

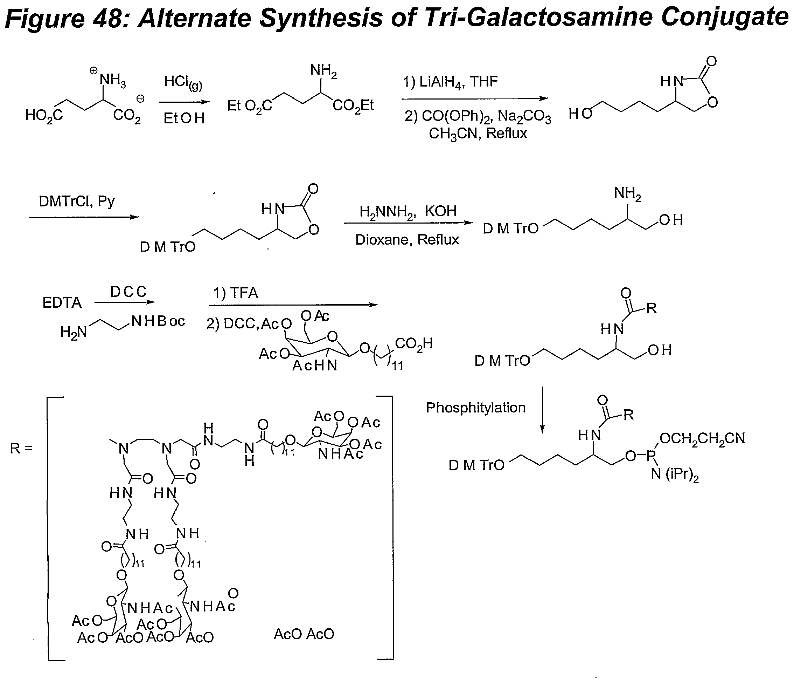

**Figure 48** shows a non-limiting example of an alternate scheme for the synthesis of a tri-galactosamine phosphoramidite of the invention that can be used to generate tri-galactosamine conjugated nucleic acid molecules.

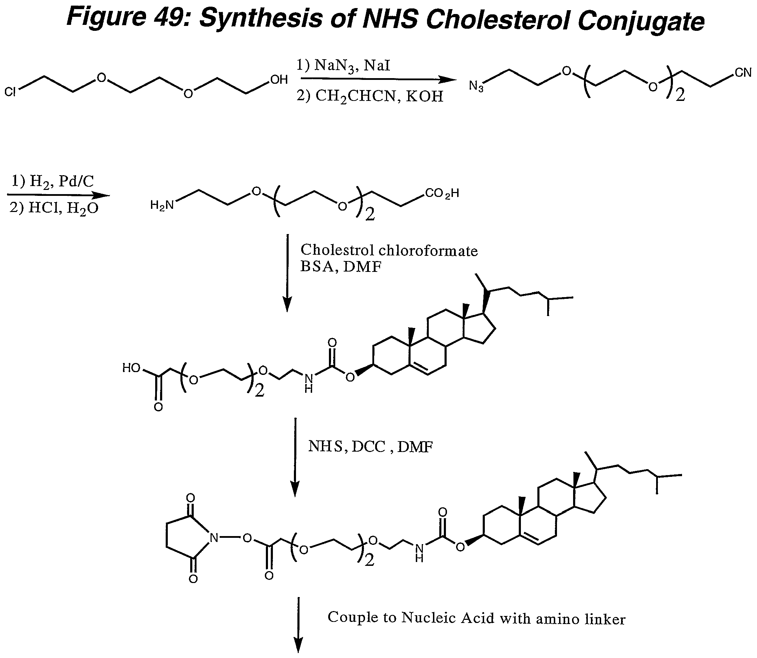

**Figure 49** shows a non-limiting example of a scheme for the synthesis of a cholesterol NHS ester of the invention that can be used to generate cholesterol conjugated nucleic acid molecules.

### Method of Use

The compositions and conjugates of the instant invention can be used to administer pharmaceutical agents. Pharmaceutical agents prevent, inhibit the occurrence, or treat (alleviate a symptom to some extent, preferably all of the symptoms) of a disease state in a patient.

Generally, the compounds of the instant invention are introduced by any standard means, with or without stabilizers, buffers, and the like, to form a pharmaceutical composition. For use of a liposome delivery mechanism, standard protocols for formation of liposomes can be followed. The compositions of the present invention can also be formulated and used as tablets, capsules or elixirs for oral administration; suppositories for rectal administration; sterile solutions; suspensions for injectable administration; and the like.

Described herein are pharmaceutically acceptable formulations of the compounds described above, preferably in combination with the molecule(s) to be delivered. These formulations include salts of the above compounds, e.g., acid addition salts, for example, salts of hydrochloric, hydrobromic, acetic acid, and benzene sulfonic acid.

Described herein is the use of the compounds of the invention in a composition comprising surface-modified liposomes containing poly (ethylene glycol) lipids (PEG-modified, or long-circulating liposomes or stealth liposomes). Described herein is the use of compounds of the invention covalently attached to polyethylene glycol. These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al., Chem. Rev. 1995, 95, 2601-2627; Ishiwata et al., Chem. Pharm. Bull. 1995, 43, 1005-1011). Such compositions have been shown to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating compositions enhance the pharmacokinetics and pharmacodynamics of therapeutic compounds, such as DNA and RNA, particularly compared to conventional cationic liposomes which are known to accumulate in tissues of the MPS (Liu et al., J. Biol. Chem. 1995, 42, 24864-24870; Choi et al., International PCT Publication No. WO 96/10391; Ansell et al., International PCT Publication No. WO 96/10390; Holland et al., International PCT Publication No. WO 96/10392). Long-circulating compositions are also likely to protect drugs from nuclease degradation to a greater extent compared to cationic liposomes, based on their ability to avoid accumulation in metabolically aggressive MPS tissues such as the liver and spleen.

Described herein are composition(s) prepared for storage or administration that includes a pharmaceutically effective amount of the desired compound(s) in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A.R. Gennaro edit. 1985). For example, preservatives, stabilizers, dyes and flavoring agents can be included in the composition. Examples of such agents include but are not limited to sodium benzoate, sorbic acid and esters of p-hydroxybenzoic acid. In addition, antioxidants and suspending agents can be included in the composition.

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the
physical characteristics of the specific mammal under consideration, concurrent medication, and other factors which those skilled in the medical arts will recognize. Generally, an amount between 0.1 mg/kg and 100 mg/kg body weight/day of active ingredients is administered dependent upon potency of the negatively charged polymer. Furthermore, the compounds of the invention and formulations thereof can be administered to a fetus via administration to the mother of a fetus.

The compounds of the invention and formulations thereof can be administered orally, topically, parenterally, by inhalation or spray or rectally in dosage unit formulations containing conventional non-toxic pharmaceutically acceptable carriers, adjuvants and vehicles. The term parenteral as used herein includes percutaneous, subcutaneous, intravascular (e.g., intravenous), intramuscular, or intrathecal injection or infusion techniques and the like. In addition, there is provided a pharmaceutical formulation comprising a nucleic acid molecule of the invention and a pharmaceutically acceptable carrier. One or more nucleic acid molecules of the invention can be present in association with one or more non-toxic pharmaceutically acceptable carriers and/or diluents and/or adjuvants, and if desired other active ingredients. The pharmaceutical compositions containing nucleic acid molecules of the invention can be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to any method known to the art for the manufacture of pharmaceutical compositions and such compositions can contain one or more such sweetening agents, flavoring agents, coloring agents or preservative agents in order to provide pharmaceutically elegant and palatable preparations. Tablets contain the active ingredient in admixture with non-toxic pharmaceutically acceptable excipients that are suitable for the manufacture of tablets. These excipients can be, for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets can be uncoated or they can be coated by known techniques. In some cases such coatings can be prepared by known techniques to delay disintegration and absorption in the gastrointestinal tract and thereby provide a sustained action over a longer period. For example, a time delay material such as glyceryl monosterate or glyceryl distearate can be employed.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavoring agents can be added to provide palatable oral preparations. These compositions can be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, can also be present.

Pharmaceutical compositions described herein can also be in the form of oil-in-water emulsions. The oily phase can be a vegetable oil or a mineral oil or mixtures of these. Suitable emulsifying agents can be naturally-occurring gums, for example gum acacia or gum tragacanth, naturally-occurring phosphatides, for example soy bean,
lecithin, and esters or partial esters derived from fatty acids and hexitol, anhydrides, for example, sorbitan monooleate, and condensation products of the said partial esters with ethylene oxide, for example polyoxyethylene sorbitan monooleate. The emulsions can also contain sweetening and flavoring agents.

Syrups and elixirs can be formulated with sweetening agents, for example glycerol, propylene glycol, sorbitol, glucose or sucrose. Such formulations can also contain a demulcent, a preservative and flavoring and coloring agents. The pharmaceutical compositions can be in the form of a sterile injectable aqueous or oleaginous suspension. This suspension can be formulated according to the known art using those suitable dispersing or wetting agents and suspending agents that have been mentioned above. The sterile injectable preparation can also be a sterile injectable solution or suspension in a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils are conventionally employed as a solvent or suspending medium. For this purpose any bland fixed oil can be employed including synthetic mono-or diglycerides. In addition, fatty acids such as oleic acid find use in the preparation of injectables.

The compounds of the invention can also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Compounds of the invention can be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels of the order of from about 0.1 mg to about 140 mg per kilogram of body weight per day are useful in the treatment of the above-indicated conditions (about 0.5 mg to about 7 g per patient per day). The amount of active ingredient that can be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition can also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

The compounds of the present invention can also be administered to a patient in combination with other therapeutic compounds to increase the overall therapeutic effect. The use of multiple compounds to treat an indication can increase the beneficial effects while reducing the presence of side effects.

### Synthesis of Nucleic acid Molecules

Synthesis of nucleic acids greater than 100 nucleotides in length is difficult using automated methods, and the therapeutic cost of such molecules is prohibitive. In this invention, small nucleic acid motifs ("small refers to nucleic acid motifs less than about 100 nucleotides in length, preferably less than about 80 nucleotides in length, and more preferably less than about 50 nucleotides in length; *e.g*., antisense oligonucleotides, hammerhead or the NCH ribozymes) are preferably used for exogenous delivery. The simple structure of these molecules increases the ability of the nucleic acid to invade targeted regions of RNA structure. Exemplary molecules of the instant invention are chemically synthesized, and others can similarly be synthesized.

Oligonucleotides (eg; antisense GeneBlocs) are synthesized using protocols known in the art as described in Caruthers et al., 1992, Methods in Enzymology 211, 3-19, Thompson et al., International PCT Publication No. WO 99/54459, Wincott et al., 1995, Nucleic Acids Res. 23, 2677-2684, Wincott et al., 1997, Methods Mol. Bio., 74, 59, Brennan et al., 1998, Biotechnol Bioeng., 61, 33-45, and Brennan, US patent No. 6,001,311. The synthesis of oligonucleotides makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 *µ*mol scale protocol with a 2.5 min coupling step for 2'-O-methylated nucleotides and a 45 sec coupling step for 2'-deoxy nucleotides. **Table II** outlines the amounts and the contact times of the reagents used in the synthesis cycle. Alternatively, syntheses at the 0.2 *µ*mol scale can be performed on a 96-well plate synthesizer, such as the instrument produced by Protogene (Palo Alto, CA) with minimal modification to the cycle. In a non-limiting example, a 33-fold excess (60 *µ*L of 0.11 M = 6.6 *µ*mol) of 2'-O-methyl phosphoramidite and a 105-fold excess of S-ethyl tetrazole (60 *µ*L of 0.25 M = 15 *µ*mol) can be used in each coupling cycle of 2'-O-methyl residues relative to polymer-bound 5'-hydroxyl. In a non-limiting example, a 22-fold excess (40 *µ*L of 0.11 M = 4.4 *µ*mol) of deoxy phosphoramidite and a 70-fold excess of S-ethyl tetrazole (40 *µ*L of 0.25 M = 10 *µ*mol) can be used in each coupling cycle of deoxy residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer includebut are not limited to; detritylation solution is 3% TCA in methylene chloride (ABI); capping is performed with 16% N-methyl imidazole in THF (ABI) and 10% acetic anhydride/10% 2,6-lutidine in THF (ABI); and oxidation solution is 16.9 mM I₂, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide, 0.05 M in acetonitrile) is used.

Deprotection of the antisense oligonucleotides is performed as follows: the polymer-bound trityl-on oligoribonucleotide is transferred to a 4 mL glass screw top vial and suspended in a solution of 40% aq. methylamine (1 mL) at 65 °C for 10 min. After cooling to -20 °C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder. Standard drying or lyophilization methods known to those skilled in the art can be used.

The method of synthesis used for normal RNA including certain enzymatic nucleic acid molecules follows the procedure as described in Usman et al., 1987, J. Am. Chem. Soc., 109, 7845; Scaringe et al., 1990, Nucleic Acids Res., 18, 5433; and Wincott et al., 1995, Nucleic Acids Res. 23, 2677-2684 Wincott et al., 1997, Methods Mol. Bio., 74, 59, and makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 *µ*mol scale protocol with a 7.5 min coupling step for alkylsilyl protected nucleotides and a 2.5 min coupling step for 2'-O-methylated nucleotides. **Table II** outlines the amounts and the contact times of the reagents used in the synthesis cycle. Alternatively, syntheses at the 0.2 *µ*mol scale can be done on a 96-well plate synthesizer, such as the instrument produced by Protogene (Palo Alto, CA) with minimal modification to the cycle. A 33-fold excess (60 *µ*L of 0.11 M = 6.6 *µ*mol) of 2'-O-methyl phosphoramidite and a 75-fold excess of S-ethyl tetrazole (60 *µ*L of 0.25 M = 15 *µ*mol) can be used in each coupling cycle of 2'-O-methyl residues relative to polymer-bound 5'-hydroxyl. A 66-fold excess (120 *µ*L of 0.11 M = 13.2 *µ*mol) of alkylsilyl (ribo) protected phosphoramidite and a 150-fold excess of S-ethyl tetrazole (120 *µ*L of 0.25 M = 30 *µ*mol) can be used in each coupling cycle of ribo residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include; detritylation solution is 3% TCA in methylene chloride (ABI); capping is performed with 16% *N*-methyl imidazole in THF (ABI) and 10% acetic anhydride/10% 2,6-lutidine in THF (ABI); oxidation solution is 16.9 mM I₂, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide0.05 M in acetonitrile) is used.

Deprotection of the RNA is performed using either a two-pot or one-pot protocol. For the two-pot protocol, the polymer-bound trityl-on oligoribonucleotide is transferred to a 4 mL glass screw top vial and suspended in a solution of 40% aq. methylamine (1 mL) at 65 °C for 10 min. After cooling to -20 °C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder. The base deprotected oligoribonucleotide is resuspended in anhydrous TEA/HF/NMP solution (300 *µ*L of a solution of 1.5 mL N-methylpyrrolidinone, 750 *µ*L TEA and 1 mL TEA•3HF to provide a 1.4 M HF concentration) and heated to 65 °C. After 1.5 h, the oligomer is quenched with 1.5 M NH₄HCO₃.

Alternatively, for the one-pot protocol, the polymer-bound trityl-on oligoribonucleotide is transferred to a 4 mL glass screw top vial and suspended in a solution of 33% ethanolic methylamine/DMSO: 1/1 (0.8 mL) at 65 °C for 15 min. The vial is brought to r.t. TEA•3HF (0.1 mL) is added and the vial is heated at 65 °C for 15 min. The sample is cooled at -20 °C and then quenched with 1.5 M NH₄HCO₃.

For purification of the trityl-on oligomers, the quenched NH₄HCO₃ solution is loaded onto a C-18 containing cartridge that had been prewashed with acetonitrile followed by 50 mM TEAA. After washing the loaded cartridge with water, the RNA is detritylated with 0.5% TFA for 13 min. The cartridge is then washed again with water, salt exchanged with 1 M NaCl and washed with water again. The oligonucleotide is then eluted with 30% acetonitrile.

Inactive hammerhead ribozymes or binding attenuated control ((BAC) oligonucleotides) are synthesized by substituting a U for G₅ and a U for A₁₄ (numbering from Hertel, K. J., et al., 1992, Nucleic Acids Res., 20, 3252). Similarly, one or more nucleotide substitutions can be introduced in other enzymatic nucleic acid molecules to inactivate the molecule and such molecules can serve as a negative control.

The average stepwise coupling yields are typically >98% (Wincott et al., 1995 Nucleic Acids Res. 23, 2677-2684). Those of ordinary skill in the art will recognize that the scale of synthesis can be adapted to be larger or smaller than the example described above including, but not limited to, 96 well format, with the ratio of chemicals used in the reaction being adjusted accordingly.

Alternatively, the nucleic acid molecules of the present invention can be synthesized separately and joined together post-synthetically, for example by ligation (Moore et al., 1992, Science 256, 9923; Draper et al., International PCT publication No. WO 93/23569; Shabarova et al., 1991, Nucleic Acids Research 19, 4247; Bellon et al., 1997, Nucleosides & Nucleotides, 16, 951; Bellon et al., 1997, Bioconjugate Chem. 8, 204).

The nucleic acid molecules of the present invention are modified extensively to enhance stability by modification with nuclease resistant groups, for example, 2'-amino, 2'-C-allyl, 2'-flouro, 2'-*O*-methyl, 2'-H (for a review see Usman and Cedergren, 1992, TIBS 17, 34; Usman et al., 1994, Nucleic Acids Symp. Ser. 31, 163). Nucleic acid conjugaes of the invention can purified by gel electrophoresis using general methods or are purified by high pressure liquid chromatography (HPLC; See Wincott *et al*., Supra) or hydrophobic interaction chromatography and are re-suspended in water.

### Optimizing Activity of the nucleic acid molecule of the invention.

Chemically synthesizing nucleic acid molecules with modifications (base, sugar and/or phosphate) that prevent their degradation by serum ribonucleases can increase their potency (see *e.g.,* Eckstein et al., International Publication No. WO 92/07065; Perrault et al., 1990 Nature 344, 565; Pieken et al., 1991, Science 253, 314; Usman and Cedergren, 1992, Trends in Biochem. Sci. 17, 334; *Usman et al*., International Publication No. WO 93/15187; and *Rossi et al*., International Publication No. WO 91/03162; Sproat, US Patent No. 5,334,711; and *Burgin et al*., *supra;* all of these describe various chemical modifications that can be made to the base, phosphate and/or sugar moieties of the nucleic acid molecules herein). Modifications which enhance their efficacy in cells, and removal of bases from nucleic acid molecules to shorten oligonucleotide synthesis times and reduce chemical requirements are desired.

There are several examples in the art describing sugar, base and phosphate modifications that can be introduced into nucleic acid molecules with significant enhancement in their nuclease stability and efficacy. For example, oligonucleotides are modified to enhance stability and/or enhance biological activity by modification with nuclease resistant groups, for example, 2'-amino, 2'-C-allyl, 2'-flouro, 2'-*O*-methyl, 2'-H, nucleotide base modifications (for a review see Usman and Cedergren, 1992, TIBS. 17, 34; Usman et al., 1994, Nucleic Acids Symp. Ser. 31, 163; Burgin et al., 1996, Biochemistry , 35, 14090). Sugar modification of nucleic acid molecules have been extensively described in the art (see Eckstein et al., International Publication PCT No. WO 92/07065; Perrault et al. Nature, 1990, 344, 565-568; Pieken et al. Science, 1991, 253, 314-317; Usman and Cedergren, Trends in Biochem. Sci. , 1992, 17, 334-339; Usman et al. International Publication PCT No. WO 93/15187; Sproat, US Patent No. 5,334,711 and Beigelman et al., 1995, J. Biol. Chem., 270, 25702; Beigelman et al., International PCT publication No. WO 97/26270; Beigelman et al., US Patent No. 5,716,824; Usman et al., US patent No. 5,627,053; Woolf et al., International PCT Publication No. WO 98/13526; Thompson *et al*., USSN 60/082,404 which was filed on April 20, 1998; Karpeisky et al., 1998, Tetrahedron Lett., 39, 1131; Earnshaw and Gait, 1998, Biopolymers (Nucleic acid Sciences), 48, 39-55; Verma and Eckstein, 1998, Annu. Rev. Biochem., 67, 99-134; and Burlina et al., 1997, Bioorg. Med. Chem., 5, 1999-2010). Such publications describe general methods and strategies to determine the location of incorporation of sugar, base and/or phosphate modifications and the like into ribozymes without inhibiting catalysis. In view of such teachings, similar modifications can be used as described herein to modify the nucleic acid molecules of the instant invention.

While chemical modification of oligonucleotide internucleotide linkages with phosphorothioate, phosphorothioate, and/or 5'-methylphosphonate linkages improves stability, too many of these modifications may cause some toxicity. Therefore, when designing nucleic acid molecules the amount of these internucleotide linkages should be minimized. Without being bound by any particular theory, the reduction in the concentration of these linkages should lower toxicity resulting in increased efficacy and higher specificity of these molecules.

Nucleic acid molecules having chemical modifications that maintain or enhance activity are provided. Such nucleic acid is also generally more resistant to nucleases than unmodified nucleic acid. Thus, in a cell and/or *in vivo* the activity can not be significantly lowered. Therapeutic nucleic acid molecules (e.g., enzymatic nucleic acid molecules and antisense nucleic acid molecules) delivered exogenously are optimally stable within cells until translation of the target RNA has been inhibited long enough to reduce the levels of the undesirable protein. This period of time varies between hours to days depending upon the disease state. The nucleic acid molecules should be resistant to nucleases in order to function as effective intracellular therapeutic agents. Improvements in the chemical synthesis of RNA and DNA (Wincott et al., 1995 Nucleic Acids Res. 23, 2677; Caruthers et al., 1992, Methods in Enzymology 211,3-19 have expanded the ability to modify nucleic acid molecules by introducing nucleotide modifications to enhance their nuclease stability as described above.

Use of the nucleic acid-based molecules of the invention can lead to better treatment of the disease progression by affording the possibility of combination therapies (e.g., multiple antisense or enzymatic nucleic acid molecules targeted to different genes, nucleic acid molecules coupled with known small molecule inhibitors, or intermittent treatment with combinations of molecules (including different motifs) and/or other chemical or biological molecules). The treatment of patients with nucleic acid molecules can also include combinations of different types of nucleic acid molecules.

In another embodiment, nucleic acid catalysts having chemical modifications that maintain or enhance enzymatic activity are provided. Such nucleic acids are also generally more resistant to nucleases than unmodified nucleic acid. Thus, in a cell and/or *in vivo* the activity of the nucleic acid can not be significantly lowered. As exemplified herein such enzymatic nucleic acids are useful in a cell and/or *in vivo* even if activity over all is reduced 10 fold (Burgin et al., 1996, Biochemistry, 35, 14090). Such enzymatic nucleic acids herein are said to "maintain" the enzymatic activity of an all RNA ribozyme or all DNA DNAzyme.

In another aspect the nucleic acid molecules comprise a 5' and/or a 3'- cap structure.

In another embodiment the 3'-cap includes, for example 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; *threo*-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety ; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (for more details see Beaucage and lyer, 1993, Tetrahedron 49, 1925).

Described herein are modified enzymatic nucleic acid molecules with phosphate backbone modifications comprising one or more phosphorothioate, phosphorodithioate, methylphosphonate, morpholino, amidate carbamate, carboxymethyl, acetamidate, polyamide, sulfonate, sulfonamide, sulfamate, formacetal, thioformacetal, and/or alkylsilyl substitutions. For a review of oligonucleotide backbone modifications see Hunziker and Leumann, 1995, Nucleic Acid Analogues: Synthesis and Properties, in Modern Synthetic Methods, VCH, 331-417, and Mesmaeker et al., 1994, Novel Backbone Replacements for Oligonucleotides, in Carbohydrate Modifications in Antisense Research, ACS, 24-39.

In connection with 2'-modified nucleotides as described for the invention, by "amino" is meant 2'-NH2 or 2'-O-NH₂, which can be modified or unmodified. Such modified groups are described, for example, in Eckstein et al., U.S. Patent 5,672,695 and Matulic-Adamic et al., WO 98/28317, respectively.

Various modifications to nucleic acid (e.g., antisense and ribozyme) structure can be made to enhance the utility of these molecules. For example, such modifications can enhance shelf-life, half-life *in vitro*, stability, and ease of introduction of such oligonucleotides to the target site, including e.g., enhancing penetration of cellular membranes and conferring the ability to recognize and bind to targeted cells.

Use of these molecules can lead to better treatment of disease progression by affording the possibility of combination therapies (e.g., multiple enzymatic nucleic acid molecules targeted to different genes, enzymatic nucleic acid molecules coupled with
known small molecule inhibitors, or intermittent treatment with combinations of enzymatic nucleic acid molecules (including different enzymatic nucleic acid molecule motifs) and/or other chemical or biological molecules). The treatment of patients with nucleic acid molecules can also include combinations of different types of nucleic acid molecules. Therapies can be devised which include a mixture of enzymatic nucleic acid molecules (including different enzymatic nucleic acid molecule motifs), antisense and/or 2-5A chimera molecules to one or more targets to alleviate symptoms of a disease.

### Indications

Particular disease states that can be treated using compounds and compositions of the invention include, but are not limited to, cancers and cancerous conditions such as breast, lung, prostate, colorectal, brain, esophageal, stomach, bladder, pancreatic, cervical, hepatocellular, head and neck, and ovarian cancer, melanoma, lymphoma, glioma, multidrug resistant cancers; ocular conditions such as macular degeneration and diabetic retinopathy, and/or viral infections including HIV, HBV, HCV, CMV, RSV, HSV, poliovirus, influenza, rhinovirus, west nile virus, severe acute respiratory syndrome (SARS) virus, Ebola virus, foot and mouth virus, and papilloma virus infection.

The molecules of the invention can be used in conjunction with other known methods, therapies, or drugs. For example, the use of monoclonal antibodies (eg; mAb IMC C225, mAB ABX-EGF) treatment, tyrosine kinase inhibitors (TKIs), for example OSI-774 and ZD1839, chemotherapy, and/or radiation therapy, are all non-limiting examples of a methods that can be combined with or used in conjunction with the compounds of the instant invention. Common chemotherapies that can be combined with nucleic acid molecules of the instant invention include various combinations of cytotoxic drugs to kill the cancer cells. These drugs include, but are not limited to, paclitaxel (Taxol), docetaxel, cisplatin, methotrexate, cyclophosphamide, doxorubin, fluorouracil carboplatin, edatrexate, gemcitabine, vinorelbine etc. Those skilled in the art will recognize that other drug compounds and therapies can be similarly be readily combined with the compounds of the instant invention are hence within the scope of the instant invention.

### Diagnostic uses

The compounds of this invention, for example, nucleic acid conjugate molecules, can be used as diagnostic tools to examine genetic drift and mutations within diseased cells or to detect the presence of a disease related RNA in a cell. The close relationship between, for example, enzymatic nucleic acid molecule activity and the structure of the target RNA allows the detection of mutations in any region of the molecule which alters the base-pairing and three-dimensional structure of the target RNA. By using multiple enzymatic nucleic acid molecules conjugates of the invention, one can map nucleotide changes which are important to RNA structure and function in vitro, as well as in cells and tissues. Cleavage of target RNAs with enzymatic nucleic acid molecules can be used to inhibit gene expression and define the role (essentially) of specified gene products in the progression of disease. In this manner, other genetic targets can be defined as important mediators of the disease. These experiments can lead to better treatment of the disease progression by affording the possibility of combinational therapies (e.g., multiple enzymatic nucleic acid molecules targeted to different genes, enzymatic nucleic acid molecules coupled with known small molecule inhibitors, or intermittent treatment with combinations of enzymatic nucleic acid molecules and/or other chemical or biological molecules). Other in vitro uses of enzymatic nucleic acid molecules of this invention are well known in the art, and include detection of the presence of mRNAs associated with a disease-related condition. Such RNA is detected by determining the presence of a cleavage product after treatment with an enzymatic nucleic acid molecule using standard methodology.

In a specific example, enzymatic nucleic acid molecules that are delivered to cells as conjugates and which cleave only wild-type or mutant forms of the target RNA are used for the assay. The first enzymatic nucleic acid molecule is used to identify wild-type RNA present in the sample and the second enzymatic nucleic acid molecule is used to identify mutant RNA in the sample. As reaction controls, synthetic substrates of both wild-type and mutant RNA are cleaved by both enzymatic nucleic acid molecules to demonstrate the relative enzymatic nucleic acid molecule efficiencies in the reactions and the absence of cleavage of the "non-targeted" RNA species. The cleavage products from the synthetic substrates also serve to generate size markers for the analysis of wild-type and mutant RNAs in the sample population. Thus each analysis requires two enzymatic nucleic acid molecules, two substrates and one unknown sample which is combined into six reactions. The presence of cleavage products is determined using an RNAse protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of the phenotype is adequate to establish risk. If probes of comparable specific activity are used for both transcripts, then a qualitative comparison of RNA levels will be adequate and will decrease the cost of the initial diagnosis. Higher mutant form to wild-type ratios are correlated with higher risk whether RNA levels are compared qualitatively or quantitatively. The use of enzymatic nucleic acid molecules in diagnostic applications contemplated by the instant invention is more fully described in George et al., US Patent Nos. 5,834,186 and 5,741,679, Shih et al., US Patent No. 5,589,332, Nathan et al., US Patent No 5,871,914, Nathan and Ellington, International PCT publication No. WO 00/24931, Breaker et al., International PCT Publication Nos. WO 00/26226 and 98/27104, and Sullenger et al., International PCT publication No. WO 99/29842.

### Additional Uses

Potential uses of sequence-specific enzymatic nucleic acid molecules that are delivered to cells as conjugates can have many of the same applications for the study of RNA that DNA restriction endonucleases have for the study of DNA (Nathans et al., 1975 Ann. Rev. Biochem. 44:273). For example, the pattern of restriction fragments can be used to establish sequence relationships between two related RNAs, and large RNAs can be specifically cleaved to fragments of a size more useful for study. The ability to engineer sequence specificity of the enzymatic nucleic acid molecule is ideal for cleavage of RNAs of unknown sequence. Applicant has described the use of nucleic acid molecules to down-regulate gene expression of target genes in bacterial, microbial, fungal, viral, and eukaryotic systems including plant, or mammalian cells.

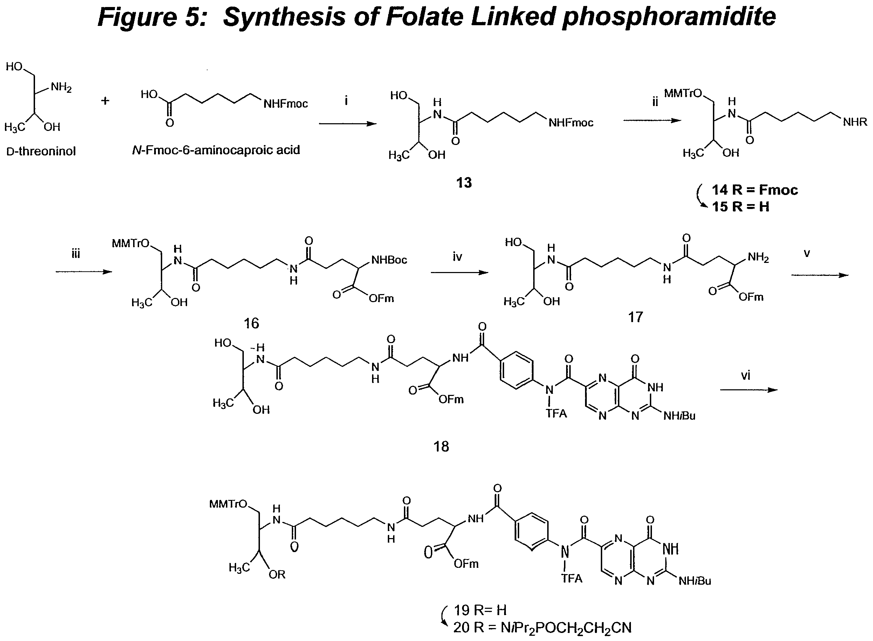

### Example 1: Synthesis of O¹-(4-monomethoxytrityl)-N-(6-(N-(α-OFm-L-glutamyl)aminocaproyl))-D-threoninol-N²-iBu-N¹⁰-TFA-pteroic acid conjugate 3'-O-(2-cyanoethyl-N,N-diisopropylphosphor-amiditel (20) (Figure 5)

**General.** All reactions were carried out under a positive pressure of argon in anhydrous solvents. Commercially available reagents and anhydrous solvents were used without further purification. ¹H (400.035 MHz) and ³¹P (161.947 MHz) NMR spectra were recorded in CDCl₃, unless stated otherwise, and chemical shifts in ppm refer to TMS and H₃PO₄, respectively. Analytical thin-layer chromatography (TLC) was performed with Merck Art.5554 Kieselgel 60 F₂₅₄ plates and flash column chromatography using Merck 0.040-0.063 mm silica gel 60.

***N*-(*N*-Fmoc-6-aminocaproyl)-D-threoninol (13).** *N*-Fmoc-6-aminocaproic acid (10 g, 28.30 mmol) was dissolved in DMF (50 ml) and *N*-hydroxysuccinimide (3.26 g, 28.30 mmol) and 1,3-dicyclohexylcarbodiimide (5.84 g, 28.3 mmol) were added to the solution. The reaction mixture was stirred at RT (about 23°C) overnight and the precipitated 1,3-dicyclohexylurea filtered off. To the filtrate D-threoninol (2.98 g, 28.30 mmol) was added and the reaction mixture stirred at RT overnight. The solution was reduced to *ca* half the volume *in vacuo,* the residue diluted with about m ml of ethyl acetate and extracted with about x ml of 5% NaHCO₃, followed by washing with brine. The organic layer was dried (Na₂SO₄), evaporated to a syrup and chromatographed by silica gel column chromatography using 1-10% gradient of methanol in ethyl acetate. Fractions containing the product were pooled and evaporated to a white solid (9.94 g, 80%). ¹H-NMR (DMSO-d₆-D₂O) δ7.97-7.30 (m, 8H, aromatic), 4.34 (d, J=6.80, 2H, Fm), 4.26 (t, J=6.80, 1H, Fm), 3.9 (m, 1H, H3 Thr), 3.69 (m, 1H, H2 Thr), 3.49 (dd, J=10.6, J=7.0, 1H, H1 Thr), 3.35 (dd, J=10.6, J=6.2, 1H, H1' Thr), 3.01 (m, 2H, *CH₂*CO Acp), 2.17 (m, 2H, *CH*₂NH Acp), 1.54 (m, 2H, CH₂ Acp), 1.45 (m, 2H, CH₂ Acp), 1.27 (m, 2H, CH₂ Acp), 1.04 (d, J=6.4, 3H, CH₃). MS/ESI⁺ m/z 441.0 (M+H)⁺.

***O*¹-(4-Monomethoxytrityl)-*N*-(*N*-Fmoc-6-aminocaproyl)-D-threoninol (14).** To the solution of **13** (6 g, 13.62 mmol) in dry pyridine (80 ml) *p*-anisylchlorodiphenylmethane (6 g, 19.43 mmol) was added and the reaction mixture stirred at RT overnight. Methanol was added (20 ml) and the solution concentrated *in vacuo.* The residual syrup was partitioned between about x ml of dichloromethane and about x ml of 5% NaHCO₃, the organic layer was washed with brine, dried (Na₂SO₄) and evaporated to dryness. Flash column chromatography using 1-3% gradient of methanol in dichloromethane afforded **14** as a white foam (6 g, 62%). ¹H-NMR (DMSO) δ7.97-6.94 (m, 22H, aromatic), 4.58 (d, 1H, J=5.2, OH), 4.35 (d, J=6.8, 2H, Fm), 4.27 (t, J=6.8, 1H, Fm), 3.97 (m, 2H, H2,H3 Thr), 3.80 (s, 3H, OCH₃), 3.13 (dd, J=8.4, J=5.6, 1H, H1 Thr), 3.01 (m, 2H, *CH*₂CO Acp), 2.92 (m, dd, J=8.4, J=6.4, 1H, H1' Thr), 2.21 (m, 2H, *CH*₂NH Acp), 1.57 (m, 2H, CH₂ Acp), 1.46 (m, 2H, CH₂ Acp), 1.30 (m, 2H, CH₂ Acp), 1.02 (d, J=5.6, 3H, CH₃). MS/ESI⁺ m/z 735.5 (M+Na)⁺.

***O*¹-(4-Monomethoxytrityl)-*N*-(6-aminocaproyl)-D-threoninol (15). 14** (9.1 g, 12.77 mmol) was dissolved in DMF (100 ml) containing piperidine (10 ml) and the reaction mixture was kept at RT for about 1 hour. The solvents were removed *in vacuo* and the residue purified by silica gel column chromatography using 1-10% gradient of methanol in dichloromethane to afford **15** as a syrup (4.46 g, 71%). ¹H-NMR δ7.48-6.92 (m, 14H, aromatic), 6.16 (d, J=8.8, 1H, NH), 4.17 (m, 1H, H3 Thr), 4.02 (m, 1H, H2 Thr), 3.86 (s, 3H, OCH₃), 3.50 (dd, J=9.7, J=4.4, 1H, H1 Thr), 3.37 (dd, J=9.7, J=3.4, 1H, H1' Thr), 2.78 (t, J=6.8, 2H, *CH₂*CO Acp), 2.33 (t, J=7.6, 2H, *CH*₂NH Acp), 1.76 (m, 2H, CH₂ Acp), 1.56 (m, 2H, CH₂ Acp), 1.50 (m, 2H, CH₂ Acp), 1.21 (d, J=6.4, 3H, CH₃). MS/EST⁺ m/z 491.5 (M+H)⁺.

***O*¹-(4-Monomethoxytrityl)-*N*-(6-(*N*-(*N*-Boc-α-OFm-L-glutamyl) aminocaproyl))-D-threoninol (16).** To the solution of *N*-Boc-α-OFm-glutamic acid (Bachem) (1.91 g, 4.48 mmol) in DMF (10 ml) *N*-hydroxysuccinimide (518 mg, 4.50 mmol) and 1,3-dicyclohexylcarbodiimide (928 mg, 4.50 mmol) was added and the reaction mixture was stirred at RT overnight. 1,3-Dicyclohexylurea was filtered off and to the filtrate **15** (2 g, 4.08 mmol) and pyridine (2 ml) were added. The reaction mixture was stirred at RT for 3 hours and than concentrated *in vacuo.* The residue was partitioned between ethyl acetate and 5% Na₂HCO₃, the organic layer extracted with brine as previously described, dried (Na₂SO₄) and evaporated to a syrup. Column chromatography using 2-10% gradient of methanol in dichlotomethane afforded **16** as a white foam (3.4 g, 93%). ¹H-NMR δ 7.86-6.91 (m, 22H, aromatic), 6.13 (d, J=8.8, 1H, NH), 5.93 (br s, 1H, NH), 5.43 (d, J=8.4, 1H, NH), 4.63 (dd, J=10.6, J=6.4, 1H, Fm), 4.54 (dd, J=10.6, J=6.4, 1H, Fm), 4.38 (m, 1H, Glu), 4.3 (t, J=6.4, 1H, Fm), 4.18 (m, 1H, H3 Thr), 4.01 (m, 1H, H2 Thr), 3.88 (s, 3H, OCH₃), 3.49 (dd, J=9.5, J=4.4, 1H, H1 Thr), 3.37 (dd, J=9.5, J=3.8, 1H, H1' Thr), 3.32 (m, 2H, *CH₂*CO Acp), 3.09 (br s, 1H, OH), 2.32 (m, 2H, *CH*₂NH Acp), 2.17 (m, 3H, Glu), 1.97 (m, 1H, Glu), 1.77 (m, 2H, CH₂ Acp), 1.61 (m, 2H, CH₂ Acp), 1.52 (s, 9H, t-Bu), 1.21 (d, J=6.4, 3H, CH₃). MS/ESI⁺ m/z 920.5 (M+Na)⁺.

***N***-**(*6***-**(*N***-***α***-**OFm**-**L**-**glutamyl**)**aminocaproyl))**-**D**-**threoninol hydrochloride (17). 16** (2 g, 2.23 mmol) was dissolved in methanol (30 ml) containing anisole (10 ml) and to this solution x ml of 4M HCl in dioxane was added. The reaction mixture was stirred for 3 hours at RT and then concentrated *in vacuo.* The residue was dissolved in ethanol and the product precipitated by addition of x ml of ether. The precipitate was washed with ether and dried to give **17** as a colorless foam (1 g, 80%). ¹H-NMR (DMSO-d₆-D₂O) δ7.97-7.40 (m, 8H, aromatic), 4.70 (m, 1H, Fm), 4.55 (m, 1H, Fm), 4.40 (t, J=6.4, 1H, Fm), 4.14 (t, J=6.6, 1H, Glu), 3.90 (dd, J=2.8, J=6.4, 1H, H3 Thr), 3.68 (m, 1H, H2 Thr), 3.49 (dd, J=10.6, J=7.0, 1H, H1 Thr), 3.36 (dd, J=10.6, J=6.2, 1H, H1' Thr), 3.07 (m, 2H, *CH*₂CO Acp), 2.17 m, 3H), 1.93 (m, 2H), 1.45 (m, 2H), 1.27 (m, 2H), 1.04 (d, J=6.4, 3H Thr). MS/ESI⁺ m/z 526.5 (M+H)⁺.

***N-(6-(N-α*-OFm-L-glutamyl)aminocaproyl))-D-threoninol-*N*²-*i*Bu-*N*¹⁰-TFA-pteroic acid conjugate (18).** To the solution of *N*²-*i*Bu-*N*¹⁰-TFA-pteroic acid¹ (480 mg, 1 mmol) in DMF (5 ml) 1-hydroxybenzotriazole (203 mg, 1.50 mmol), EDCI (288 mg, 1.50 mmol) and **17** (free base, 631 mg, 1.2 mmol) are added. The reaction mixture is stirred at RT for 2 hours, then concentrated to *ca* 3 ml and loaded on the column of silica gel. Elution with dichloromethane, followed by 1-20% gradient of methanol in dichloromethane afforded **18** (0.5 g, 51%). ¹H-NMR (DMSO-d₆-D₂O) δ 9.09 (d, J=6.8, 1H, NH) 8.96 (s, 1H, H7 pteroic acid), 8.02-7.19 (m, 13H, aromatic, NH), 5.30 ( s, 2H, pteroic acid), 4.50 (m, 1H, Glu), 4.41 (d, J=6.8, 2H, Fm), 4.29 (t, J=6.8, 1H, Fm), 3.89 (dd, J=6.2, J=2.8, 1H, H3 Thr), 3.68 (m, 1H, H2 Thr), 3.48 (dd, J=10.4, J=7.0, 1H, H1 Thr), 3.36 (dd, J=10.4, J=6.2, 1H H1' Thr), 3.06 (m, 2H, *CH₂*CO Acp), 2.84 (m, 1H, *i*Bu), 2.25 (m, 2H, *CH*₂NH Acp), 2.16 (m, 3H, Glu), 1.99 (m, 1H, Glu), 1.52 (m, 2H Acp), 1.42 (m, 2H Acp), 1.27 (m, 2H Acp), 1.20 (s, 3H *i*Bu), 1.19 (s, 3H, *i*Bu), 1.03 (d, J=6.2, 3H Thr). MS/ESI⁻ m/z 984.5 (M-H)⁻.

***O*¹-(4-monomethoxytrityl)-*N-(6-(N-α-*OFm-L-glutamyl)aminocaproyl))-D-threoninol-*N*²-*i*Bu-*N*¹⁰-TFA-pteroic acid conjugate (19).** To the solution of conjugate **18** (1 g, 1.01 mmol) in dry pyridine (15 ml) *p*-anisylchlorodiphenylmethane (405 mg) was added and the reaction mixture was stirred, protected from moisture, at RT overnight. Methanol (3 ml) was added and the reaction mixture concentrated to a syrup *in vacuo.* The residue was partitioned between dichloromethane and 5% NaHCO₃, the organic layer washed with brine, dried (Na₂SO₄) and evaporated to dryness. Column chromatography using 0.5-10% gradient of methanol in dichloromethane afforded **19** as a colorless foam (0.5 g, 39%. ¹H-NMR (DMSO-d₆-D₂O δ9.09 (d, J=6.8, 1H, NH) 8.94 (s, 1H, H7 pteroic acid), 8.00-6.93 (m, 27H, aromatic, NH), 5.30 ( s, 2H, pteroic acid), 4.50 (m, 1H, Glu), 4.40 (d, J=6.8, 2H, Fm), 4.29 (t, J=6.8, 1H, Fm), 3.94 (m, 2H, H3,H2 Thr), 3.79 (s, 3H, OCH₃) 3.11 (dd, J=8.6, J=5.8, 1H, H1 Thr), 3.04 (m, 2H, *CH₂*CO Acp), 2.91 (dd, J=8.6, J=6.4, 1H, H1' Thr), 2.85 (m, 1H, *i*Bu), 2.25 (m, 2H, *CH*₂NH Acp), 2.19 (m, 2H, Glu), 2.13 (m, 1H, Glu), 1.98 (m, 1H, Glu), 1.55 (m, 2H Acp), 1.42 (m, 2H Acp), 1.29(m, 2H Acp), 1.20 (s, 3H *i*Bu), 1.18 (s, 3H, *i*Bu), 1.00 (d, J=6.4, 3H Thr). MS/EST m/z 1257.0 (M-H)⁻.

***O*¹-(4-monomethoxytrityl)-*N*-(6-(*N*-a-OFm-L-glutamyl)aminocaproyl))-D-threoninol-*N*²-*i*Bu-*N*¹⁰-TFA-pteroic acid conjugate 3'-*O*-(2-cyanoethyl-*N*,*N-*diisopropylphosphor-amidite) (20).** To the solution of **19** (500 mg, 0.40 mmol) in dichloromethane (2 ml) 2-cyanoethyl tetraisopropylphosphordiamidite (152 µL, 0.48 mmol) was added followed by pyridinium trifluoroacetate (93 mg, 0.48 mmol). The reaction mixture was stirred at RT for 1 hour and than loaded on the column of silica gel in hexanes. Elution using ethyl acetate-hexanes 1:1, followed by ethyl acetate and ethyl acetate-acetone 1:1 in the presence of 1% pyridine afforded **20** as a colorless foam (480 mg, 83%). ³¹P NMR δ 149.4 (s), 149.0 (s).

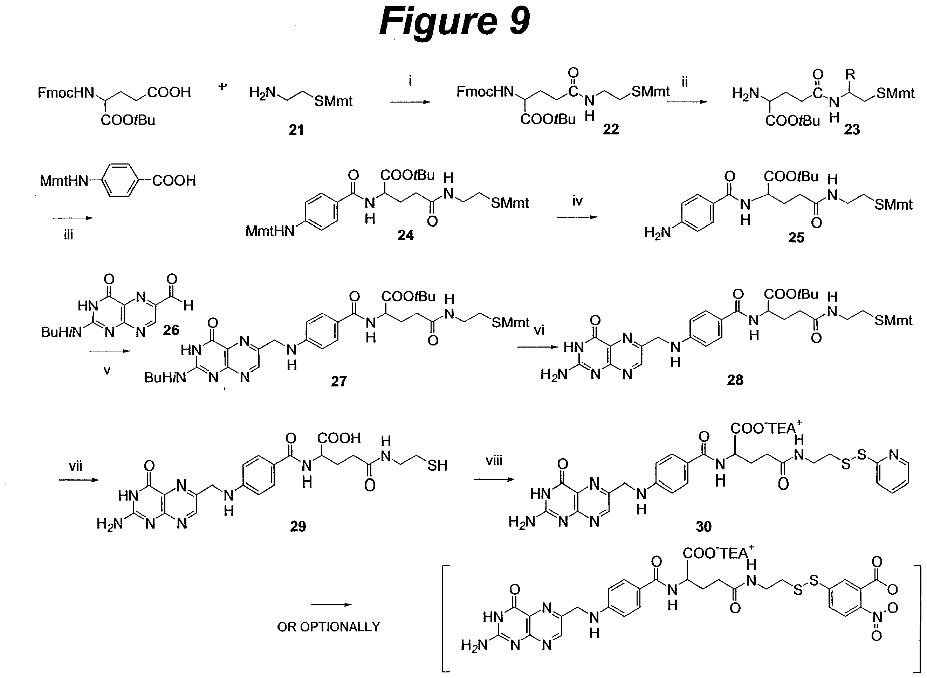

### Example 2: Synthesis of 2-dithiopyridyl activated folic acid (30) (Figure 9)

Synthesis of the cysteamine modified folate **30** is presented in Fig. 9. Monomethoxytrityl cysteamine **21** was prepared by selective tritylation of the thiol group of cysteamine with 4-methoxytrityl alcohol in trifluoroacetic acid. Peptide coupling of 21 with Fmoc-Glu-O*t*Bu (Bachem Bioscience Inc., King of Prussia, PA) in the presence of PyBOP yielded **22** in a high yield. *N*-Fmoc group was removed smoothly with piperidine to give **23.** Condensation of **23** with *p*-(4-methoxytrityl)aminobenzoic acid, prepared by reaction of *p*-aminobenzoic acid with 4-methoxytrityl chloride in pyridine, afforded the fully protected conjugate **24.** Selective cleavage of *N*-MMTr group with acetic acid afforded **25** in quantitative yield. Shiff base formation between **25** and N²-*i*Bu-6-formylpterin **26**,⁹ followed by reduction with borane-pyridine complex proceeded with a good yield to give fully protected cysteamine-folate adduct **27**.¹² The consecutive cleavage of protecting groups of **27** with base and acid yielded thiol derivative **29.** The thiol exchange reaction of **29** with 2,2-dipyridyl disulfide afforded the desired *S*-pyridyl activated synthon **30** as a yellow powder; Isolated as a TEA⁺ salt: ¹H NMR spectrum for **10** in D₂O: δ 8.68 (s, 1 H, H-7), 8.10 (d, J = 3.6, 1 H, pyr), 7.61 (d, J = 8.8, 2 H, PABA), 7.43 (m, 1 H, pyr), 7.04 (d, J = 7.6, 1 H, pyr), 6.93 (m, 1 H, pyr), 6.82 (d, J = 8.8, 1 H, PABA), 4.60 (s, 2 H, 6-CH₂), 4.28 (m, 1 H, Glu), 3.30-3.08 (m, 2 H, cysteamine), 3.05 (m, 6 H, TEA), 2.37 (m, 2 H, cysteamine), 2.10 (m, 4 H, Glu), 1.20 (m, 9 H, TEA). MS/ESI⁻ m/z 608.02 [M-H]⁻. It is worth noting that the isolation of **30** as its TEA⁺ or Na⁺ salt made it soluble in DMSO and/or water, which is an important requirement for its use in conjugation reactions.

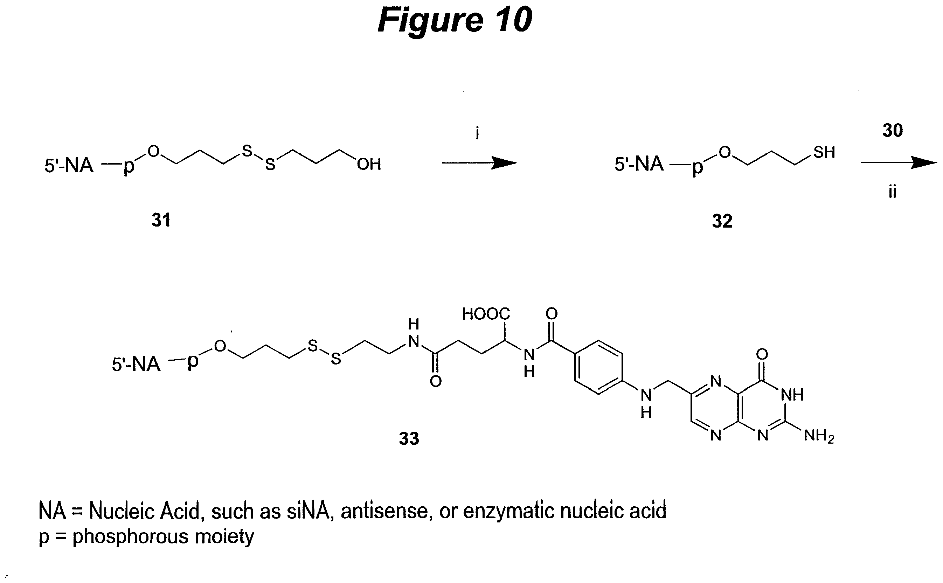

### Example 3: Post synthetic conjugation of enzymatic nucleic acid to form nucleic acid-folate conjugate (33) (Figure 10)

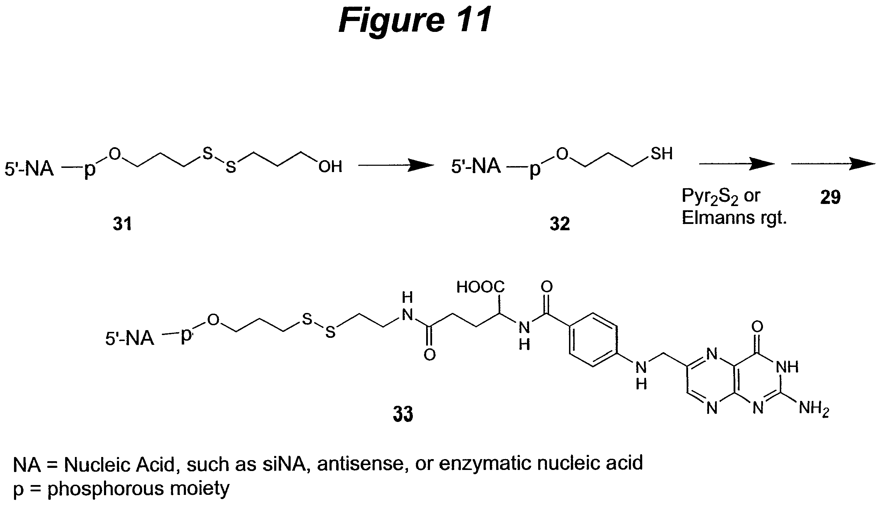

Oligonucleotide synthesis, deprotection and purification was performed as described herein. 5'-Thiol-Modifier C6 (Glen Research, Sterling, Virginia) was coupled as the last phosphoramidite to the 5'-end of a growing oligonucleotide chain. After cleavage from the solid support and base deprotection, the disulfide modified enzymatic nucleic acid molecule **31** (Fig. 10) was purified using ion exchange chromatography. The thiol group was unmasked by reduction with dithiothreitol (DTT) to afford **32** which was purified by gel filtration and immediately conjugated with **30.** The resulting conjugate **33** was separated from the excess folate by gel filtration and then purified by RP HPLC using gradient of acetonitrile in 50 mM triethylammonium acetate (TEAA). Desalting was performed by RP HPLC. Reactions were conducted on 400 mg of disulfide modified enzymatic nucleic acid molecule **31** to afford 200-250 mg (50-60% yield) of conjugate 33. MALDI TOF MS confirmed the structure: **13** [M-H]⁻ 12084.74 (calc.12083.82). An alternative approach to this synthesis is shown in **Figure 11****.**

As shown in Examples 2 and 3, a folate-cysteamine adduct can be prepared by a scaleable solution phase synthesis in a good overall yield. Disulfide conjugation of this novel targeting ligand to the thiol-modified oligonucleotide is suitable for the multi-gram scale synthesis. The 9-atom spacer provides a useful spatial separation between folate and attached oligonucleotide cargo. Importantly, conjugation of folate to the oligonucleotide through a disulfide bond should permit intermolecular separation which was suggested to be required for the functional cytosolic entry of a protein drug.

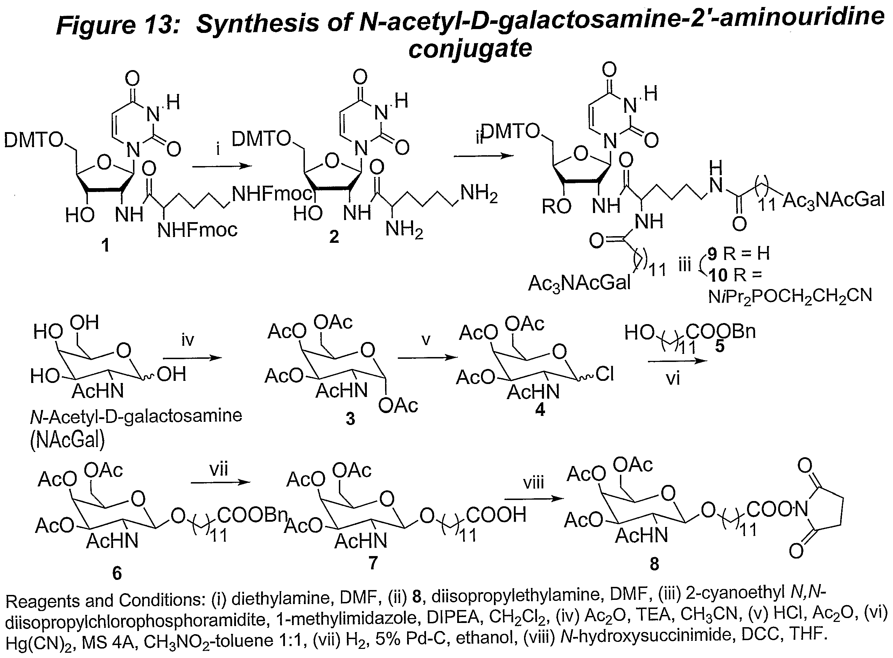

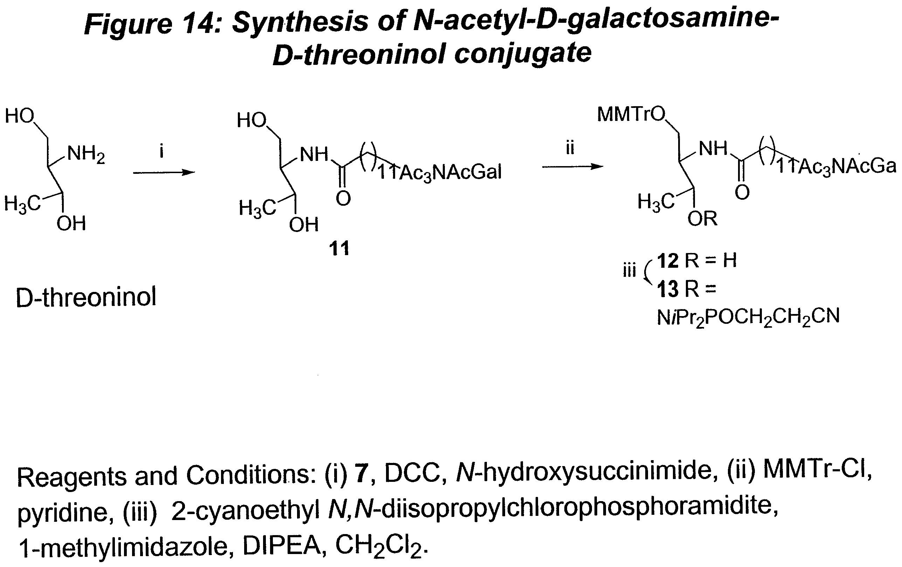

### Example 4: Synthesis of Galactose and N-acetyl-Galactosamine conjugates (Figures 13, 14, and 15)

Applicant has designed both nucleoside and non-nucleoside-*N*-acetyl-D-galactosamine conjugates suitable for incorporation at any desired position of an oligonucleotide. Multiple incorporations of these monomers could result in a "glycoside cluster effect".

All reactions were carried out under a positive pressure of argon in anhydrous solvents. Commercially available reagents and anhydrous solvents were used without further purification. *N*-acetyl-D-galactosamine was purchased from Pfanstiel (Waukegan, IL), folic acid from Sigma (St. Louis, MO), D-threoninol from Aldrich (Milwaukee, WI) and *N*-Boc-α-OFm glutamic acid from Bachem. ¹H (400.035 MHz) and ³¹P (161.947 MHz) NMR spectra were recorded in CDCl₃, unless stated otherwise, and chemical shifts in ppm refer to TMS and H3PO4, respectively. Analytical thin-layer chromatography (TLC) was performed with Merck Art.5554 Kieselgel 60 F₂₅₄ plates and flash column chromatography using Merck 0.040-0.063 mm silica gel 60. The general procedures for RNA synthesis, deprotection and purification are described herein. MALDI-TOF mass spectra were determined on PerSeptive Biosystems Voyager spectrometer. Electrospray mass spectrometry was run on the PE/Sciex API365 instrument.

**2'-(*N*-L-lysyl)amino-5'-*O*-4,4'-dimethoxytrityl-2'-deoxyuridine (2).** 2'-(*N*-α,ε-bis-Fmoc-L-lysyl)amino-5'-*O*-4,4'-dimethoxytrityl-2'-deoxyuridine **(1)** (4 g, 3.58 mmol) was dissolved in anhydrous DMF (30 ml) and diethylamine (4 ml) was added. The reaction mixture was stirred at rt for 5 hours and than concentrated (oil pump) to a syrup. The residue was dissolved in ethanol and ether was added to precipitate the product (1.8 g, 75%). ¹H-NMR (DMSO-d₆-D₂O) δ 7.70 (d, J6,5=8.4, 1H, H6), 7.48-6.95 (m, 13H, aromatic), 5.93 (d, J1',2'=8.4, 1H, H1'), 5.41 (d, J_{5,6}=8.4, 1H, H5), 4,62 (m, 1H, H2'), 4.19 (d, 1H, J_{3',2'}=6.0, H3'), 3.81 (s, 6H, 2xOMe), 3.30 (m, 4H, 2H5', CH₂), 1.60-1.20 (m, 6H, 3×CH₂). MS/ESI⁺ m/z 674.0 (M+H)⁺.

***N*-Acetyl-1,4,6-tri-*O*-acetyl-2-amino-2-deoxy-β-D-galactospyranose (3).** *N*-Acetyl-D-galac-tosamine (6.77 g, 30.60 mmol) was suspended in acetonitrile (200 ml) and triethylamine (50 ml, 359 mmol) was added. The mixture was cooled in an ice-bath and acetic anhydride (50 ml, 530 mmol)) was added dropwise under cooling. The suspension slowly cleared and was then stirred at rt for 2 hours. It was than cooled in an ice-bath and methanol (60 ml) was added and the stirring continued for 15 min. The mixture was concentrated under reduced pressure and the residue partitioned between dichloromethane and 1 N HCl. Organic layer was washed twice with 5% NaHCO₃, followed by brine, dried (Na2SO4) and evaporated to dryness to afford 10 g (84%) of **3** as a colorless foam. ¹H NMR was in agreement with published data (Findeis, 1994, Int. J. Peptide Protein Res., 43, 477-485.

**2-Acetamido-3,4,6-tetra-*O*-acetyl-1-chloro-D-galactospyranose (4).** This compound was prepared from **3** as described by Findeis *supra.*

**Benzyl 12-Hydroxydodecanoate (5).** To a cooled (0 °C) and stirred solution of 12-hydroxydodecanoic acid (10.65 g, 49.2 mmol) in DMF (70 ml) DBU (8.2 ml, 54.1 mmol) was added, followed by benzyl bromide (6.44 ml, 54.1 mmol). The mixture was left overnight at rt, than concentrated under reduced pressure and partitioned between 1 N HCl and ether. Organic phase was washed with saturated NaHCO₃, dried over Na₂SO₄ and evaporated. Flash chromatography using 20-30% gradient of ethyl acetate in hexanes afforded benzyl ester as a white powder (14.1 g, 93.4%). ¹H-NMR spectral data were in accordance with the published values.³³

**12'-Benzyl hydroxydodecanoyl-2-acetamido-3,4,6-tri-*O*-acetyl-2-deoxy-β-D-galactopyrano-se (6).** 1-Chloro sugar **4** (4.26 g, 11.67 mmol)and benzyl 12-hydroxydodecanoate **(5)** (4.3 g, 13.03 mmol) were dissolved in nitromethane-toluene 1:1 (122 ml) under argon and Hg(CN)₂ (3.51 g, 13.89 mmol) and powdered molecular sieves 4A (1.26 g) were added. The mixture was stirred at rt for 24 h, filtered and the filtrate concentrated under reduced pressure. The residue was partitioned between dichloromethane and brine, organic layer was washed with brine, followed by 0.5 M KBr, dried (Na₂SO₄) and evaporated to a syrup. Flash silica gel column chromatography using 15-30% gradient of acetone in hexanes yielded product **6** as a colorless foam (6 g, 81%). ¹H-NMR δ 7.43 (m, 5H, phenyl), 5.60 (d, 1H, J_{NH,2}=8.8, NH), 5.44 (d, J_{4,3}=3.2, 1H, H4), 5.40 (dd, J_{3,4}=3.2, J_{3,2}=10.8, 1H, H3), 5.19 (s, 2H, *CH*₂Ph), 4.80 (d, J_{1,2}=8.0, 1H, H1), 4.23 (m, 2H, CH₂), 3.99 (m, 3H, H2, H6), 3.56 (m, 1H, H5), 2.43 (t, J=7.2, 2H,CH₂), 2.22 (s, 3H, Ac), 2.12 (s, 3H, Ac), 2.08 (s, 3H, Ac), 2.03 (s, 3H, Ac), 1.64 (m, 4H, 2×CH₂), 1.33 (br m, 14H, 7×CH₂). MS/ESI⁻ m/z 634.5 (M-H)⁻.

### 12'-Hydroxydodecanoyl-2-acetamido-3,4,6-tri-O-acetyl-2-deoxy-β-D-galactopyranose (7).

Conjugate **6** (2 g, 3.14 mmol)) was dissolved in ethanol (50 ml) and 5% Pd-C (0.3 g) was added. The reaction mixture was hydrogenated overnight at 45 psi H₂, the catalyst was filtered off and the filtrate evaporated to dryness to afford pure 7 (1.7 g, quantitative) as a white foam. ¹H-NMR δ 5.73 (d, 1H, J_{NH,2}=8.4, NH), 5.44 (d, J_{4,3}=3.0, 1H, H4), 5.40 (dd, ^{J}_{3,4}=3.0, J_{3,2}=11.2,1H, H3), 4.78 (d, J_{1,2}=8.8, 1H, H1), 4.21(m, 2H, CH₂), 4.02 (m, 3H, H2, H6), 3.55 (m, 1H, H5), 2.42 (m, 2H, CH₂), 2.23(s, 3H, Ac), 2.13 (s, 3H, Ac), 2.09 (s, 3H, Ac), 2.04 (s, 3H, Ac), 1.69 (m, 4H, 2×CH₂), 1.36 (br m, 14H, 7×CH₂). MS/ESI⁻ m/z 544.0 (M-H)⁻.

**2'-(*N*-α,ε-bis-(12'-Hydroxydodecanoyl-2-acetamido-3,4,6-tri-*O*-acetyl-2-deoxy-β-D-galac-topyranose)-L-lysyl)amino-2'-deoxy-5'-*O*-4,4'-dimethoxytrityl uridine (9). 7** (1.05 g, 1.92 mmol) was dissolved in anhydrous THF and *N*-hydroxysuccinimide (0.27 g, 2.35 mmol) and 1,3-dicyclohexylcarbodiimide (0.55 g, 2.67 mmol) were added. The reaction mixture was stirred at rt overnight, then filtered through Celite pad and the filtrate concentrated under reduced pressure. The crude NHSu ester **8** was dissolved in dry DMF (13 ml) containing diisopropylethylamine (0.67 ml, 3.85 mmol) and to this solution nucleoside **2** (0.64 g, 0.95 mmol was added). The reaction mixture was stirred at rt overnight and than concentrated under reduced pressure. The residue was partitioned between water and dichloromethane, the aqueous layer extracted with dichloromethane, the organic layers combined, dried (Na₂SO₄) and evaporated to a syrup. Flash silica gel column chromatography using 2-3% gradient of methanol in ethyl acetate yielded **9** as a colorless foam (1.04 g, 63%). ¹H-NMR ^{δ} 7.42 (d, J_{6,5}=8.4, 1H, H6 Urd), 7.53-6.97 (m, 13H, aromatic), 6.12 (d, J_{1',2'}=8.0, 1H, H-1'), 5.41 (m, 3H, H5 Urd, H4 NAcGal), 5.15 (dd, J_{3,4}=3.6, J_{3,2}=11.2, 2H, H3 NAcGal), 4.87 (dd, J_{2',3'}=5.6, J_{2',1},=8.0, 1H, H2'), 4.63 (d, J_{1,2}=8.0, 2H, H1 NAcGal), 4.42 (d, J_{3',2'}=5.6, 1H, H3'), 4.29-4.04 (m, 9H, H4', H2 NAcGal, H5 NacGal, CH₂), 3.95-3.82 (m, 8H, H6 NAcGal, 2xOMe), 3.62-3.42 (m, 4H, H5', H6 NAcGal), 3.26 (m, 2H, CH₂), 2.40-1.97 (m, 28H, CH₂, Ac), 1.95-1.30 (m, 50H, CH₂). MS/ESI⁻ m/z 1727.0 (M-H)⁻.

**2'-(*N*-α,ε-bis-(12'-Hydroxydodecanoyl-2-acetamido-3,4,6-tri-*O*-acetyl-2-deoxy-β-D-galac-topyranose)-L-lysyl)amino-2'-deoxy-5'-*O*-4,4'-dimethoxytrityl uridine 3'-*O-*(2-cyanoethyl *N*,*N*-diisopropylphosphoramidite) (10).** Conjugate **9** (0.87 g, 0.50 mmol) was dissolved in dry dichloromethane (10 ml) under argon and diisopropylethylamine (0.36 ml, 2.07 mmol) and 1-methylimidazole (21 µL, 0.26 mmol) were added. The solution was cooled to 0 °C and 2-cyanoethyl diisopropylchlorophosphoramidite (0.19 ml, 0.85 mmol) was added. The reaction mixtures was stirred at rt for 1 hour, than cooled to 0 °C and quenched with anhydrous ethanol (0.5 ml). After stirring for 10 min the solution was concentrated under reduced pressure (40 °C) and the residue dissolved in dichloromethane and chromatographed on the column of silica gel using hexanes-ethyl acetate 1:1, followed by ethyl acetate and finally ethyl acetate-acetone 1:1 (1% triethylamine was added to solvents) to afford the phosphoramidite **10** (680 mg, 69%). ³¹P-NMR δ 152.0 (s), 149.3 (s). MS/ESI⁻ m/z 1928.0 (M-H)⁻.

***N*-(12'-Hydroxydodecanoyl-2-acetamido-3,4,6-tri-*O*-acetyl-2-deoxy-β-D-galactopyranose)-D-threoninol (11).** 12'-Hydroxydodecanoyl-2-acetamido-3,4,6-tri-*O-*acetyl-2-deoxy-β-D-galac-topyranose **7** (850 mg, 1.56 mmol) was dissolved in DMF (5 ml) and to the solution *N*-hydroxysuccinimide (215 mg, 1.87 mmol) and 1,3-dicyclohexylcarbodimide (386 mg, 1.87 mmol) were added. The reaction mixture was stirred at rt overnight, the precipitate was filtered off and to the filtrate D-threoninol (197 mg, 1.87 mmol) was added. The mixture was stirred at rt overnight and concentrated in vacuo. The residue was partitioned between dichloromethane and 5% NaHCO₃, the organic layer was washed with brine, dried (Na₂SO₄) and evaporated to a syrup. Silica gel column chromatography using 1-10% gradient of methanol in dichloromethane afforded **11** as a colorless oil (0.7 g, 71%). ¹H-NMR δ 6.35 (d, J=7.6, 1H, NH), 5.77 (d, **J**=8.0, 1H, NH), 5.44 (d, J_{4,3}=3.6, 1H, H4), 5.37 (dd, J_{3,4}=3.6, J_{3,2}=11.2, 1H, H3), 4.77 (d, **J**_{1,2}=8.0, 1H, H1), 4.28-4.18 (m, 3H, CH₂, CH), 4.07-3.87 (m, 6H), 3.55 (m, 1H, H5), 3.09 (d, J=3.2, 1H, OH), 3.02 (t, J=4.6, 1H, OH), 2.34 (t, J=7.4 2H, CH₂), 2.23 (s, 3H, Ac), 2.10 (s, 3H, Ac), 2.04 (s, 3H, Ac), 1.76-1.61 (m, 2×CH₂), 1.35 (m, 14H, 7×CH₂), **1**.29 (d, J=6.4, 3H, CH₃). MS/ESI⁻ m/z (M-H)⁻.

**1-*O*-(4-Monomethoxytrityl)-*N*-(12'-hydroxydodecanoyl-2-acetamido-3,4,6-tri-*O-*acetyl-2-deoxy-β-D-galactopyranose)-D-threoninol (12).** To the solution of **11** (680 mg, 1.1 mmol) in dry pyridine (10 ml) *p*-anisylchlorotriphenylmethane (430 mg, 1.39 mmol) was added and the rection mixture was stirred, protected from moisture, overnight. Methanol (3 ml) was added and the solution stirred for 15 min and evaporated *in vacuo.* The residue was partitioned between dichloromethane and 5% NaHCO₃, the organic layer was washed with brine, dried (Na₂SO₄) and evaporated to a syrup. Silica gel column chromatography using 1-3% gradient of methanol in dichloromethane afforded **12** as a white foam (0.75 g, 77%). ¹H-NMR δ 7.48-6.92 (m, 14 H, aromatic), 6.15 (d, J=8.8, 1H, NH), 5.56 (d, J=8.0, 1H, NH), 5.45 (d, J_{4,3}=3.2, 1H, H4), 5.40 (dd, J_{3,4}=3.2, J_{3,2}=11.2, 1H, H3), 4.80 (d, J_{1,2}=8.0, 1H, H1), 4.3-4.13 (m, 3H, CH₂, CH), 4.25-3.92 (m, 4H, H6, H2, CH), 3.89 (s, 3H, OMe), 3.54 (m, 2H, H5, CH), 3.36 (dd, J=3.4, J=9.8, 1H, CH), 3.12 (d, J=2.8, 1H, OH), 2.31 (t, J=7.6, 2H, CH₂), 2.22 (s, 3H, Ac), 2.13 (s, 3H, Ac), 2.03 (s, 3H, Ac), 1.80-1.55 (m, 2×CH₂), 1.37 (m, 14H, 7×CH₂), 1.21 (d, J=6.4, 3H, CH₃). MS/ESI⁻ m/z 903.5 (M-H)⁻.

**1-*O*-(4-Monomethoxytrityl)-*N*-(12'-hydroxydodecanoyl-2-acetamido-3,4,6-tri-*O-*acetyl-2-deoxy-β-D-galactopyranose)-D-threoninol 3-*O*-(2-cyanoethyl *N,N-*diisopropylphosphorami-dite) (13).** Conjugate **12** (1.2 g, 1.33 mmol) was dissolved in dry dichloromethane (15 ml) under argon and diisopropylethylamine (0.94 ml, 5.40 mmol) and 1-methylimidazole (55 µL, 0.69 mmol) were added. The solution was cooled to 0 °C and 2-cyanoethyl *N*,*N*-diisopropyl-chlorophosphoramidite (0.51 ml, 2.29 mmol) was added. The reaction mixture was stirred at rt for 2 hours, than cooled to 0 °C and quenched with anhydrous ethanol (0.5 ml). After stirring for 10 min. the solution was concentrated under reduced pressure (40 °C) and the residue dissolved in dichloromethane and chromatographed on the column of silica gel using 50-80% gradient of ethyl acetate in hexanes (1% triethylamine) to afford the phosphoramidite **13** (1.2 g, 82%). ³¹P-NMR δ 149.41 (s), 149.23 (s).

### Oligonucleotide synthesis

Phosphoramidites **10,** and **13,** were used along with standard 2'-*O*-TBDMS and 2'-*O-*methyl nucleoside phosphoramidites. Synthesis were conducted on a 394 (ABI) synthesizer using modified 2.5 µmol scale protocol with a 5 min coupling step for 2'-*O-*TBDMS protected nucleotides and 2.5 min coupling step for 2'-O-methyl nucleosides. Coupling efficiency for the phosphoramidite **10** was lower than 50% while coupling efficiencies for phosphoramidite **13** was typically greater than 95% based on the measurement of released trityl cations. Once the synthesis was completed, the oligonucleotides were deprotected. The 5'-trityl groups were left attached to the oligomers to assist purification. Cleavage from the solid support and the removal of the protecting groups was performed as described herein with the exception of using 20% piperidine in DMF for 15 min for the removal of Fm protection prior methylamine treatment. The 5'-tritylated oligomers were separated from shorter (trityl-off) failure sequences using a short column of SEP-PAK C-18 adsorbent. The bound, tritylated oligomers were detritylated on the column by treatment with 1% trifluoroacetic acid, neutralized with triethylammonium acetate buffer, and than eluted. Further purification was achieved by reverse-phase HPLC. An example of a N-acetyl-D-galactosamine conjugate that can be synthesized using phosphoramidite **13** is shown in **Figure 15****.**

Structures of the ribozyme conjugates were confirmed by MALDI-TOF MS.

### Monomer synthesis

**2'-Amino-2'-deoxyuridine-*N*-acetyl-D-galactosamine conjugate.** The bis-Fmoc protected lysine linker was attached to the 2'-amino group of 2'-amino-2'-deoxyuridine using the EEDQ catalyzed peptide coupling. The 5'-OH was protected with 4,4'-dimethoxytrityl group to give **1,** followed by the cleavage of *N*-Fmoc groups with diethylamine to afford synthon **2** in the high overall yield.

2-acetamido-3,4,6-tetra-*O*-acetyl-1-chloro-D-galactopyranose **4** was synthesized with minor modifications according to the reported procedure (Findeis *supra*). Mercury salt catalyzed glycosylation of **4** with the benzyl ester of 12-hydroxydodecanoic acid **5** afforded glycoside **6** in 81 % yield. Hydrogenolysis of benzyl protecting group yielded **7** in a quantitative yield. The coupling of the sugar derivative with the nucleoside synthon was achieved through preactivation of the carboxylic function of **7** as *N-*hydroxysuccinimide ester **8,** followed by coupling to lysyl-2'-aminouridine conjugate **2.** The final conjugate **9** was than phosphitylated under standard conditions to afford the phosphoramidite **10** in 69% yield.

**D-Threoninol-*N*-acetyl-D-galactosamine conjugate** Using the similar strategy as described above, D-threoninol was coupled to **7** to afford conjugate **11** in a good yield. Monomethoxytritylation, followed by phosphitylation yielded the desired phosphoramidite **13.**

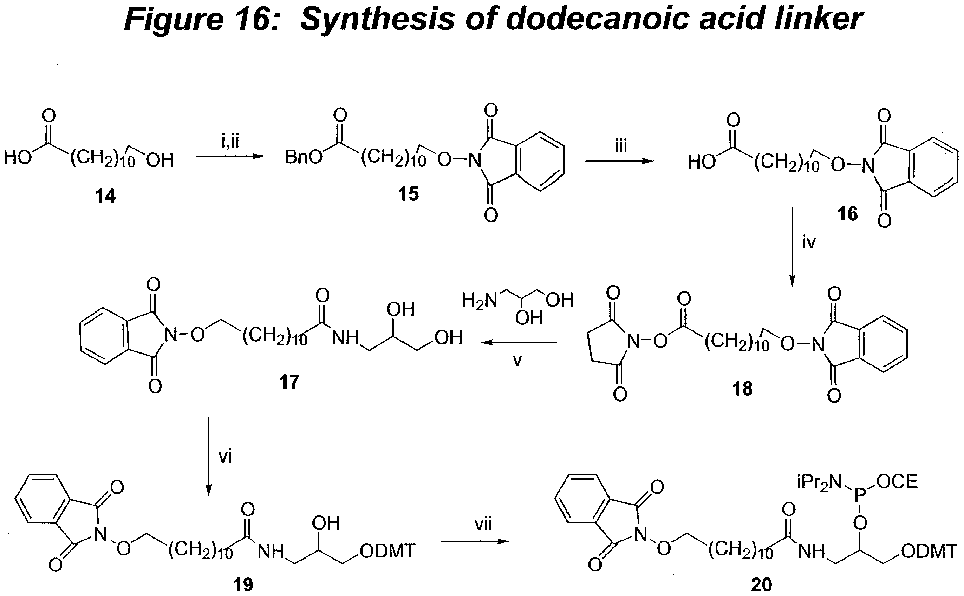

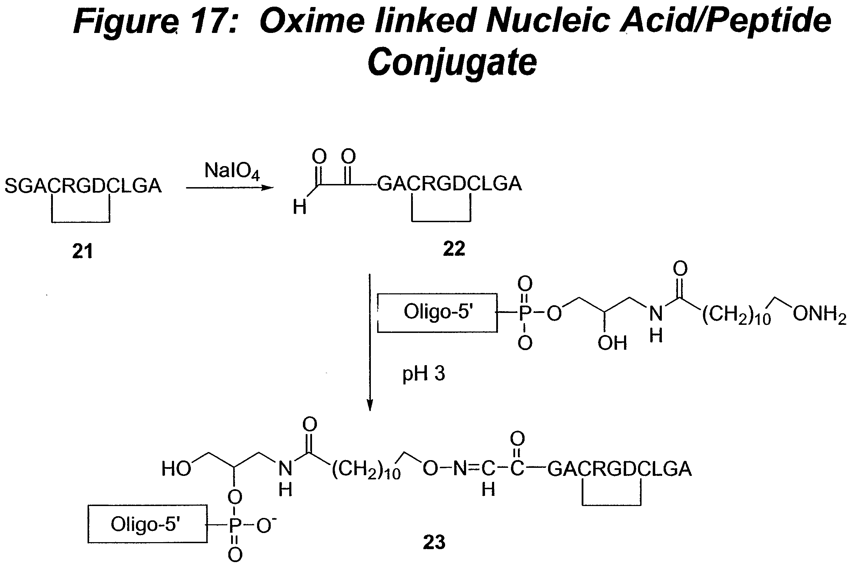

### Example 2: Synthesis of Oxime linked nucleic acid/peptide conjugates (Figures 16 and 17)

**12-Hydroxydodecanoic acid benzyl ester** Benzyl bromide (10.28 ml, 86.45 mmol) was added dropwise to a solution of 12-hydroxydodecanoic acid (17 g, 78.59 mmol) and DBU (12.93 ml, 86.45 mmol) in absolute DMF (120 ml) under vigorous stirring at 0°C. After completeion of the addition reaction mixture was warmed to a room temperature and left overnight under stirring. TLC (hexane-ethylacetate 3:1) indicated complete transformation of the starting material. DMF was removed under reduced pressure and the residue was partitioned between ethyl ether and 1N HCl. Organic phase was separated, washed with saturated aq sodium bicarbonate and dried over sodium sulfate. Sodium sulfate was filtered off, filtrate was evaporated to dryness. The residue was crystallized from hexane to give 21.15 g (92%) of the title compound as a white powder.

**12-O-N-Phthaloyl-dodecanoic acid benzyl ester (15).** Diethylazodicarboxylate (DEAD, 16.96 ml, 107.7 mmol) was added dropwise to the mixture of 12-Hydroxydodecanoic acid benzyl ester (21g, 71.8 mmol), triphenylphosphine (28.29 g, 107.7 mmol) and N-hydroxyphthalimide (12.88 g, 78.98 mmol) in absolute THF (250 ml) at -20° - -30°C under stirring. The reaction mixture was stirred at this temperature for additional 2-3h, after which time TLC (hexane-ethylacetate 3:1) indicated reaction completion. The solvent was removed in *vacuo* and the residue was treated ether (250 ml). Formed precipitate of triphenylphosphine oxide was filtered off, mother liquor was evaporated to dryness and the residue was dissolved in methylene chloride and purified by flash chromatography on silica gel in hexane-ethyl acetate (7:3). Appropriate fractions were pooled and evaporated to dryness to afford 26.5 g(84.4%) of compound **15.**

**12-O-N-Phthaloyl-dodecanoic acid (16).** Compound **15** (26.2 g, 59.9 mmol) was dissolved in 225 ml of ethanol-ethylacetate (3.5:1) mixture and 10% Pd/C (2.6 g) was added. The reaction mixture was hydrogenated in Parr apparatus for 3 hours. Reaction mixture was filtered through celite and evaporated to dryness. The residue was crystallized from methanol to provide 15.64 g (75%) of compound **16.**

**12-O-N-Phthaloyl-dodecanoic acid 2,3-di-hydroxy-propylamide (18)** The mixture of compound **16** (15.03 g, 44.04 mmol), dicyclohexylcarbodiimide (10.9 g, 52.85 mmol) and N-hydroxysuccinimide (6.08 g, 52.85 mmol) in absolute DMF (150 ml) was stirred at room temperature overnight. TLC (methylene chloride -methanol 9:1) indicated complete conversion of the starting material and formation of NHS ester **17.** Then aminopropanediol (4.01 g, 44 mmol) was added and the reaction mixture was stirred at room temperature for another 2 h. The formed precipitate of dicyclohexylurea was removed by filtration, filtrate was evaporated under reduced pressure. The residue was partitioned between ethyl acetate and saturated aq sodium bicarbonate. The whole mixture was filtered to remove any insoluble material and clear layers were separated. Organic phase was concentrated in vacuo until formation of crystalline material. The precipitate was filtered off and washed with cold ethylacetate to produce 10.86 g of compound **17.** Combined mother liquor and washings were evaporated to dryness and crystallized from ethylacetate to afford 3.21 g of compound **18.** Combined yield - 14.07 g (73.5%).

**12-O-N-Phthaloyl-dodecanoic acid 2-hydroxy,3-dimethoxytrityloxy-propylamide (19)**_Dimethoxytrityl chloride (12.07 g, 35.62 mmol) was added to a stirred solution of compound **18** (14.07 g, 32.38 mmol) in absolute pyridine (130 ml) at 0°C. The reaction solution was kept at 0°C overnight. Then it was quenched with MeOH (10 ml) and evaporated to dryness. The residue was dissolved in methylene chloride and washed with saturated aq sodium bicarbonate. Organic phase was separated, dried over sodium sulfate and evaporated to dryness. The residue was purified by flash chromatography on silica gel using step gradient of acetone in hexanes (3:7 to 1:1) as an eluent. Appropriate fractions were pooled and evaporated to provide 14.73g (62%) of compound **19,** as a colorless oil.

**12-O-N-Phthaloyl-dodecanoic acid 2-O-(cyanoethyl-N.N-diisopropylamino-phosphoramidite),3-dimethoxytrityloxy-propylamide (20).** Phosphitylated according to Sanghvi, et al., 2000, Organic Process Research and Development, 4, 175-81.

Purified by flash chromatography on silica gel using step gradient of acetone in hexanes (1:4 to 3:7) containing 0.5% of triethylamine. Yield - 82%, colourless oil.

### Oxidation of peptides

Peptide (3.3 mg, 3.3 µmol) was dissolved in 10mM AcONa and 2 eq of sodium periodate (100mM soln in water) was added. Final reaction volume - 0.5 ml. After 10 minutes reaction mixture was purified using analytical HPLC on Phenomenex Jupiter 5u C18 300A (150x4.6 mm) column; solvent A: 50mM KH₂PO₄ (pH 3); solvent B: 30% of solvent A in MeCN; gradient B over 30 min. Appropriate fractions were pooled and concentrated on a SpeedVac to dryness. Yield: quantitative.

### Conjugation reaction of Herzyme-ONH2-linker with N-glyoxyl peptide (Figure 17)

Herzyme (SEQ ID NO: 13) with a 5'-terminal linker (100 OD) was mixed with oxidized peptide (3-5 eq) in 50 mM KH2PO4 (pH3, reaction volume 1 ml) and kept at room temperature for 24-48h. The reaction mixture was purified using analytical HPLC on a Phenomenex Jupiter 5u C18 300A (150x4.6 mm) column; solvent A: 10mM TEAA; solvent B: 10mM TEAA/MeCN. Appropriate fractions were pooled and concentrated on a SpeedVac to dryness to provide desired conjugate. ESMS: calculated: 12699, determined: 12698.

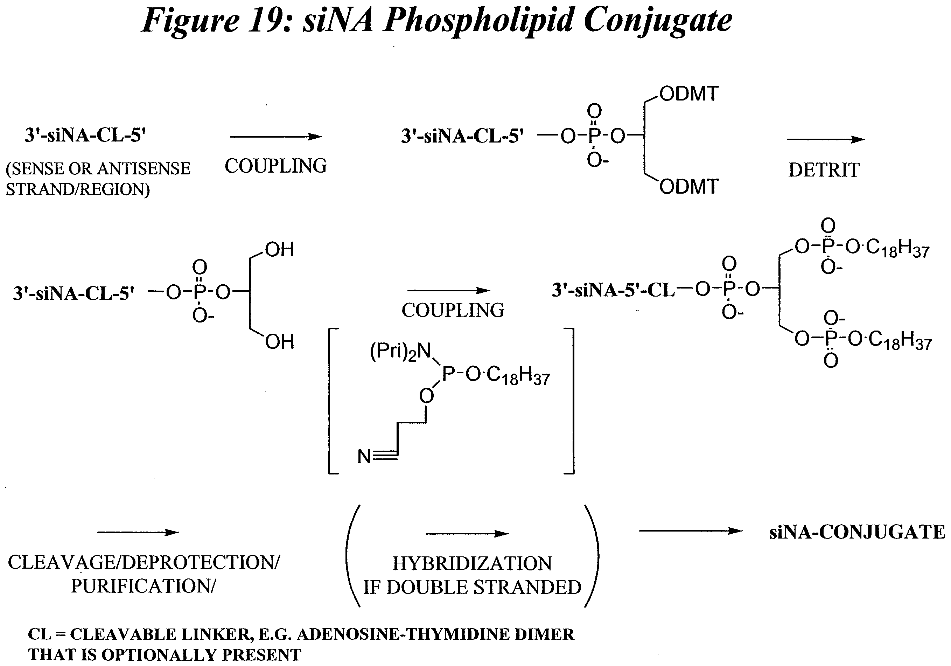

### Example 5: Synthesis of Phospholipid enzymatic nucleic acid conjugates (Figure 19)

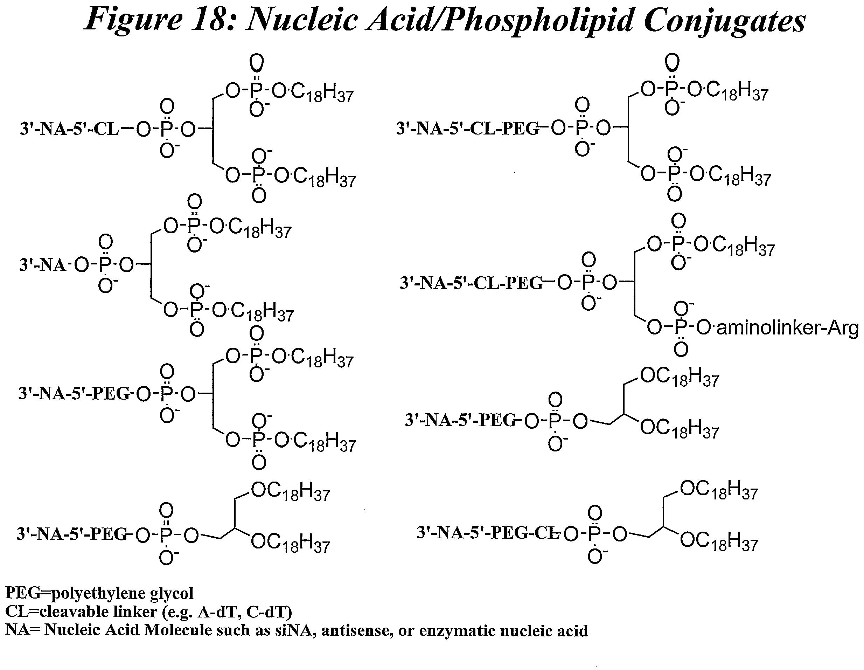

A phospholipid enzymatic nucleic acid conjugate (see **Figure 19****)** was prepared by coupling a C18H37 phosphoramidite to the 5'-end of an enzymatic nucleic acid molecule (Angiozyme™, SEQ ID NO: 24) during solid phase oligonucleotide synthesis on an ABI 394 synthesizer using standard synthesis chemistry. A 5'-terminal linker comprising 3'-AdT-di-Glycerol-5', where A is Adenosine, dT is 2'-deoxy Thymidine, and di-Glycerol is a di-DMT-Glycerol linker (Chemgenes CAT number CLP-5215), is used to attach two C18H37 phosphoramidites to the enzymatic nucleic acid molecule using standard synthesis chemistry. Additional equivalents of the C18H37 phosphoramidite were used for the bis-coupling. Similarly, other nucleic acid conjugates as shown in **Figure 18** can be prepared according to similar methodology.

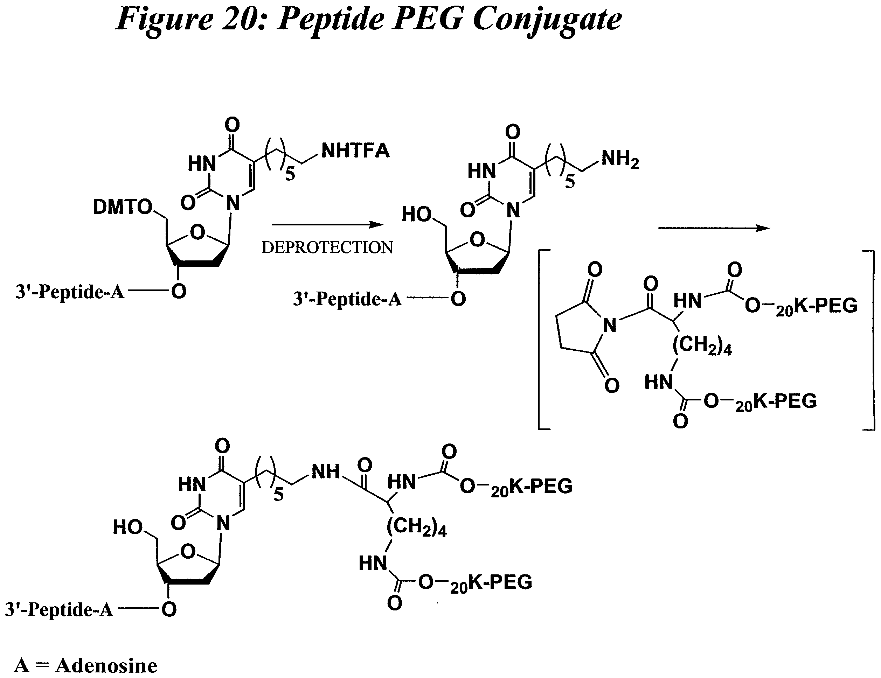

### Example 6: Synthesis of PEG enzymatic nucleic acid conjugates (Figure 20)

A 40K-PEG enzymatic nucleic acid conjugate (see **Figure 20****)** was prepared by post synthetic N-hydroxysuccinimide ester coupling of a PEG derivative (Shearwater Polymers Inc, CAT number PEG2-NHS) to the 5'-end of an enzymatic nucleic acid molecule (Angiozyme™, SEQ ID NO: 24). A 5'-terminal linker comprising 3'-AdT-C6-amine-5', where A is Adenosine, dT-C6-amine is 2'-deoxy Thymidine with a C5 linked six carbon amine linker (Glen Research CAT number 10-1039-05), is used to attach the PEG derivative to the enzymatic nucleic acid molecule using NHS coupling chemistry.

Angiozyme™ with the C6dT-NH2 at the 5' end was synthesized and deprotected using standard oligonucleotide synthesis procedures as described herein. The crude sample was subsequently loaded onto a reverse phase column and rinsed with sodium chloride solution (0.5 M). The sample was then desalted with water on the column until the concentration of sodium chloride was close to zero. Acetonitrile was used to elute the sample from the column. The crude product was then concentrated and lyophilized to dryness.

The crude material (Angiozyme™) with 5'-amino linker (50 mg) was dissolved in sodium borate buffer (1.0 mL, pH 9.0). The PEG NHS ester (200 mg) was dissolved in anhydrous DMF (1.0 mL). The Angiozyme™ buffer solution was then added to the PEG NHS ester solution. The mixture was immediately vortexed for 5 minutes. Sodium acetate buffer solution (5 mL, pH 5.2) was used to quench the reaction. Conjugated material was then purified by ion-exchange and reverse phase chromatography.

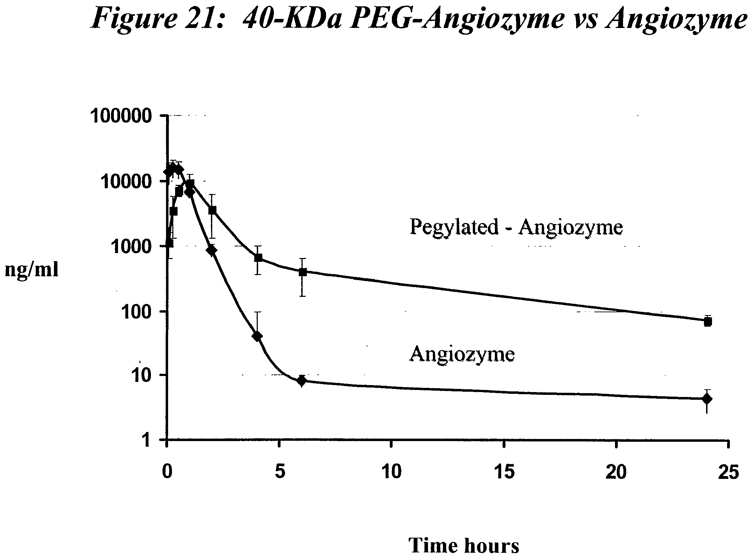

### Example 7: Phamacokinetics of PEG ribozyme acid conjugate (Figure 21)

Forty-eight female C57B1/6 mice were given a single subcutaneous (SC) bolus of 30 mg/kg Angiozyme™ and 30 mg/kg Angiozyme™/40K PEG conjugate. Plasma was collected out to 24 hours post ribozyme injection. Plasma samples were analyzed for full length ribozyme by a hybridization assay.

Oligonucleotides complimentary to the 5' and 3' ends of Angiozyme™ were synthesized with biotin at one oligo, and FITC on the other oligo. A biotin oligo and FITC labeled oligo pair are incubated at 1 ug/ml with known concentrations of Angiozyme™ at 75degrees C for 5 min. After 10 minutes at RT, the mixture is allowed to bind to streptavidin coated wells of a 96-wll plate for two hours. The plate is washed with Tris-saline and detergent, and peroxidase labeled anti-FITC antibody is added. After one hour, the wells are washed, and the enzymatic reaction is developed, then read on an ELISA plate reader. Results are shown in **Figure 21****.**

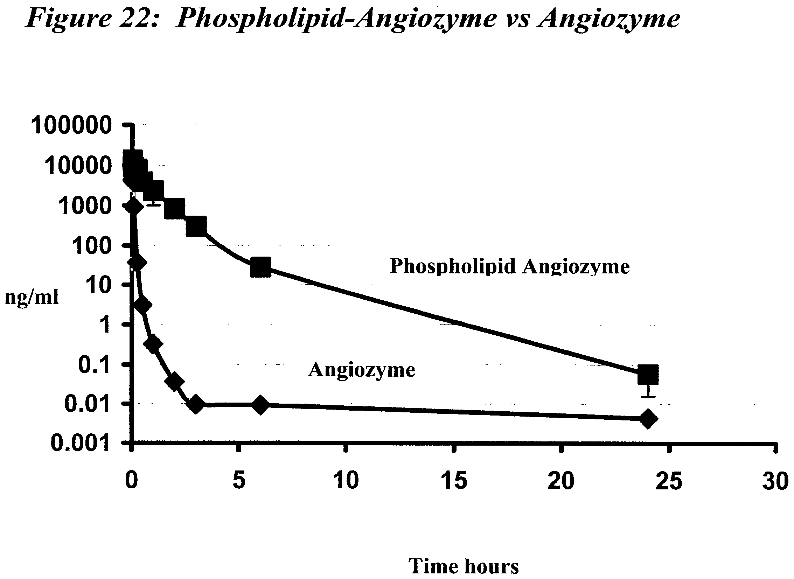

### Example 8: Phamacokinetics of Phospholipid ribozyme conjugate (Figure 22)

Seventy-two female C57B1/6 mice were given a single intravenous (4) bolus of 30 mg/kg Angiozyme™ and 30 mg/kg Angiozyme™ conjugated with phospholipid **(****Figure 19****).** Plasma was collected out to 3 hours post ribozyme injection. Plasma samples were analyzed for full length ribozyme by a hybridization assay.

Oligonucleotides complimentary to the 5' and 3' ends of Angiozyme™ were synthesized with biotin at one oligo, and FITC on the other oligo. A biotin oligo and FITC labeled oligo pair are incubated at 1 ug/ml with known concentrations of Angiozyme™ at 75degrees C for 5 min. After 10 minutes at RT, the mixture is allowed to bind to streptavidin coated wells of a 96-wll plate for two hours. The plate is washed with Tris-saline and detergent, and peroxidase labeled anti-FITC antibody is added. After one hr, the wells are washed, and the enzymatic reaction is developed, then read on an ELISA plate reader. Results are shown in **Figure 22****.**

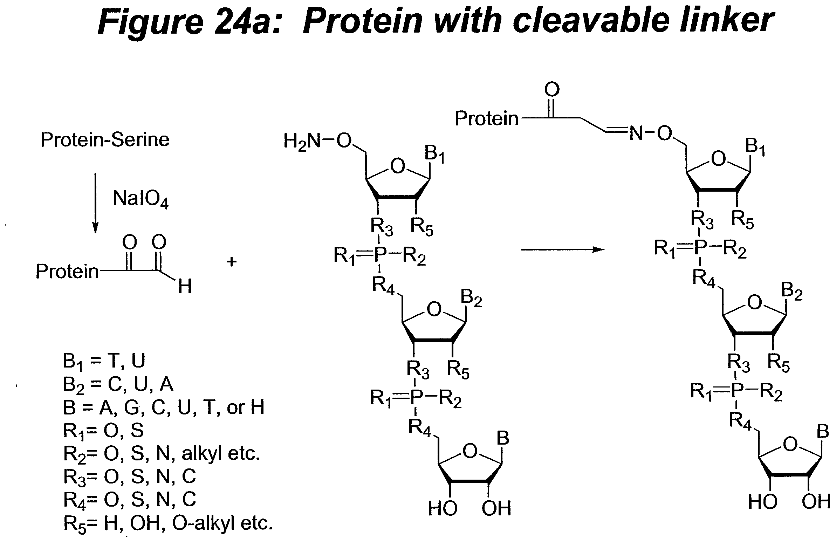

### Example 9: Synthesis of Protein or Peptide conjugates with biodegradable linkers (Figures 24-26, and 29)

Proteins and peptides can be conjugated with various molecules, including PEG, via biodegradable nucleic acid linker molecules of the invention, using oxime and morpholino linkages. For example, a therapeutic antibody can be conjugated with PEG to improve the **Figure 24** shows an example of a synthetic approach for synthesizing peptide or protein conjugates to PEG utilizing a biodegradable linker, the example shown is for a protein conjugate. Other conjugates can be synthesized in a similar manner where the protein or peptide is conjugated to molecules other than PEG, such as small molecules, toxins, radioisotopes, peptides or other proteins. (a) The protein of interest, such as an antibody or interferon, is synthesized with a terminal Serine or Threonine moiety that is oxidized, for example with sodium periodate. The oxidized protein is then coupled to a nucleic acid linker molecule that is designed to be biodegradable, for example a cytidine-deoxythymidine, cytidine-deoxyuridine, adenosine-deoxythymidine, or adenosine-deoxyuridine dimer that contains an oxyamino (O-NH₂) function. Other biodegradable nucleic acid linkers can be similarly used, for example other dimers, trimers, tetramers etc. that are designed to be biodegradable. The example shown makes use of a 5'-oxyamino moiety, however, other examples can utilize an oxyamino at other positions within the nucleic acid molecule, for example at the 2'-position, 3'-position, or at a nucleic acid base position. (b) The protein/nucleic acid conjugate is then oxidized to generate a dialdehyde function that is coupled to PEG molecule comprising an amino group (H₂N-PEG), for example a PEG molecule with an amino linker. Other amino containing molecules can be conjugated as shown in the figure, for example small molecules, toxins, or radioisotope labeled molecules.

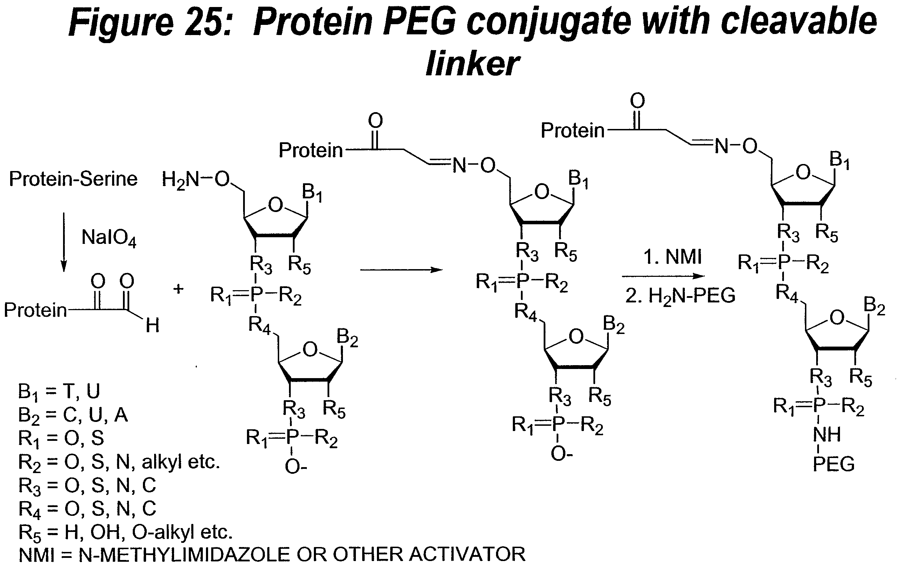

Proteins and peptides can be conjugated with various molecules, including PEG, via biodegradable nucleic acid linker molecules of the invention, using oxime and phosphoramidate linkages. **Figure 25** shows an example of a synthetic approach for synthesizing peptide or protein conjugates to PEG utilizing a biodegradable linker, the example shown is for a protein conjugate. Other conjugates can be synthesized in a similar manner where the protein or peptide is conjugated to molecules other than PEG, such as small molecules, toxins, radioisotopes, peptides or other proteins. The protein of interest, such as an antibody or interferon, is synthesized with a terminal Serine or Threonine moiety that is oxidized, for example with sodium periodate. The oxidized protein is then coupled to a nucleic acid linker molecule that is designed to be biodegradable, for example a cytidine-deoxythymidine, cytidine-deoxyuridine, adenosine-deoxythymidine, or adenosine-deoxyuridine dimer that contains an oxyamino (O-NH₂) function and a terminal phosphate group. Terminal phosphate groups can be introduced during synthesis of the nucleic acid molecule using chemical phosphorylation reagents, such as Glen Research Cat Nos. 10-1909-02, 10-1913-02, 10-1914-02, and 10-1918-02. Other biodegradable nucleic acid linkers can be similarly used, for example other dimers, trimers, tetramers etc. that are designed to be biodegradable. The example shown makes use of a 5'-oxyamino moiety, however, other examples can utilize an oxyamino at other positions within the nucleic acid molecule, for example at the 2'-position, 3'-position, or at a nucleic acid base position. The protein/nucleic acid conjugate terminal phosphate group is then activated with an activator reagent, such as NMI and/or tetrazole, and coupled a PEG molecule comprising an amino group (H₂N-PEG), for example a PEG molecule with an amino linker. Other amino containing molecules can be conjugated as shown in the figure, for example small molecules, toxins, or radioisotope labeled molecules.

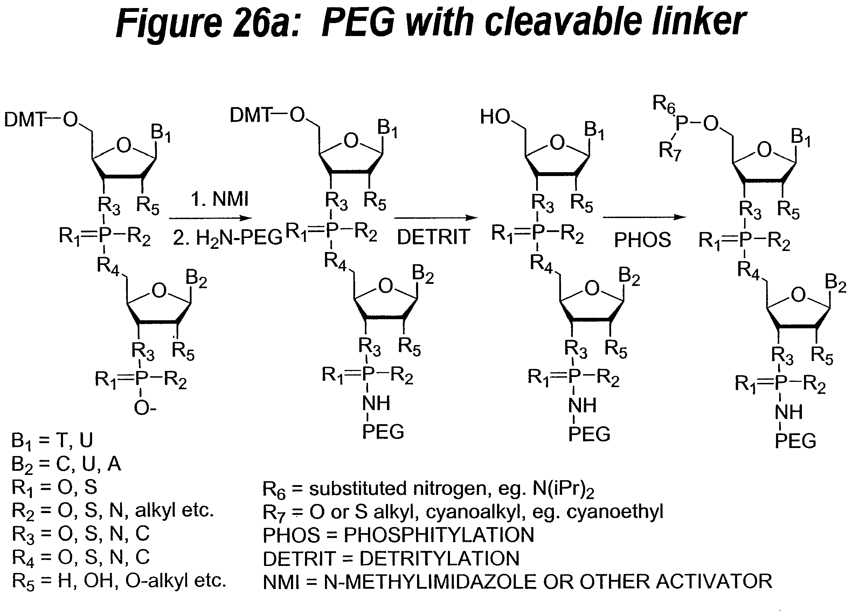

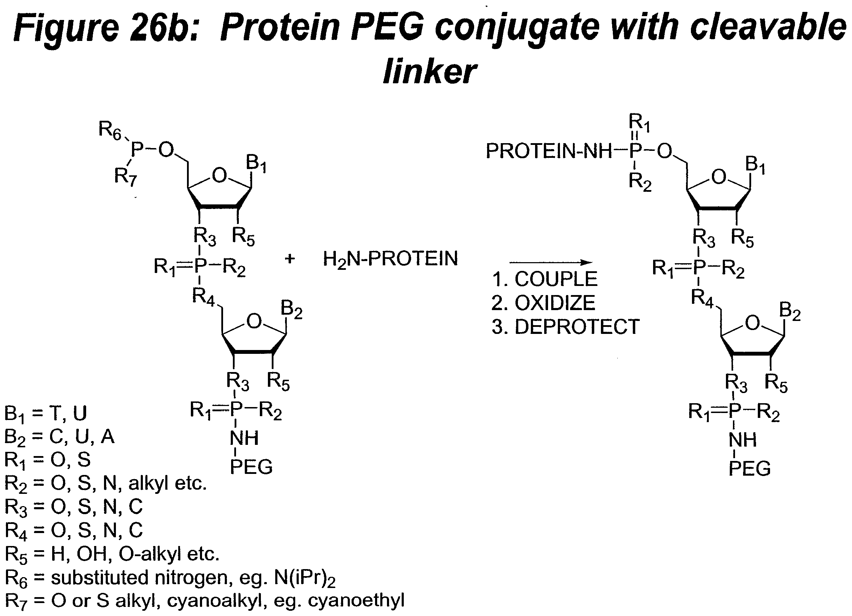

Proteins and peptides can be conjugated with various molecules, including PEG, via biodegradable nucleic acid linker molecules of the invention, using phosphoramidate linkages. **Figure 26** shows an example of a synthetic approach for synthesizing peptide or protein conjugates to PEG utilizing a biodegradable linker, the example shown is for a protein conjugate. Other conjugates can be synthesized in a similar manner where the protein or peptide is conjugated to molecules other than PEG, such as small molecules, toxins, radioisotopes, peptides or other proteins. (a) A nucleic acid linker molecule that is designed to be biodegradable, for example a cytidine-deoxythymidine, cytidine-deoxyuridine, adenosine-deoxythymidine, or adenosine-deoxyuridine dimer, is synthesized with a terminal phosphate group. Other biodegradable nucleic acid linkers can be similarly used, for example other dimers, trimers, tetramers etc. that are designed to be biodegradable. The protein/nucleic acid conjugate terminal phosphate group is then activated with an activator reagent, such as NMI and/or tetrazole, and coupled a PEG molecule comprising an amino group (H₂N-PEG), for example a PEG molecule with an amino linker. Other amino containing molecules can be conjugated as shown in the figure, for example small molecules, toxins, or radioisotope labeled molecules. The terminal protecting group, for example a dimethoxytrityl group, is removed from the conjugate and a terminal phosphite group is introduced with a phosphitylating reagent, such as N,N-diisopropyl-2-cyanoethyl chlorophosphoramidite. (b) The PEG/nucleic acid conjugate is then coupled to a peptide or protein comprising an amino group, such as the amino terminus or amino side chain of a suitably protected peptide or protein or via an amino linker. The conjugate is then oxidized and any protecting groups are removed to yield the protein/PEG conjugate comprising a biodegradable linker.

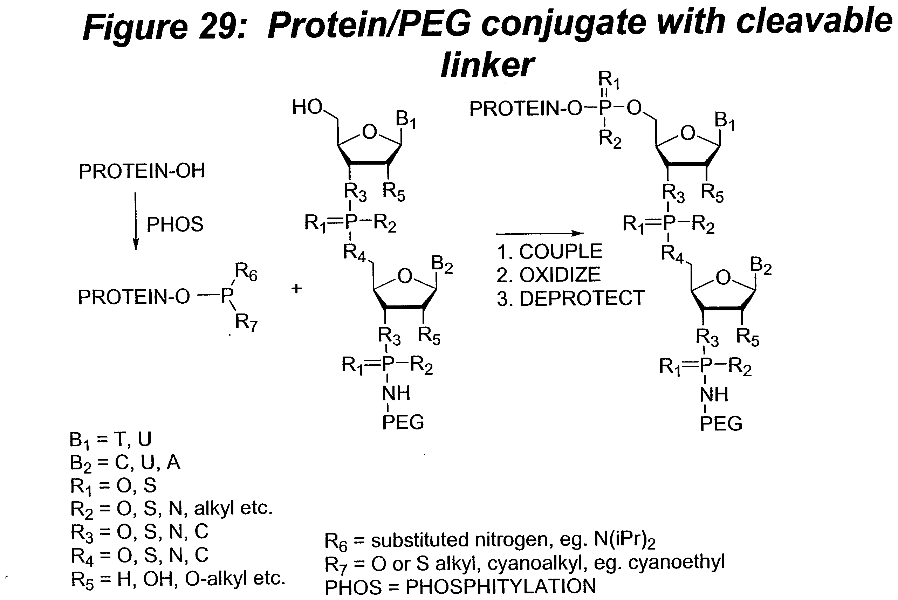

Proteins and peptides can be conjugated with various molecules, including PEG, via biodegradable nucleic acid linker molecules of the invention, using phosphoramidate linkages from coupling protein-based phosphoramidites. **Figure 29** shows an example of a synthetic approach for synthesizing peptide or protein conjugates to PEG utilizing a biodegradable linker, the example shown is for a protein conjugate. Other conjugates can be synthesized in a similar manner where the protein or peptide is conjugated to molecules other than PEG, such as small molecules, toxins, radioisotopes, peptides or other proteins. The protein of interest, such as an antibody or interferon, is synthesized with a terminal Serine, Threonin, or Tyrosine moiety that is phosphitylated, for example with N,N-diisopropyl-2-cyanoethyl chlorophosphoramidite. The phosphitylated protein is then coupled to a nucleic acid linker molecule that is designed to be biodegradable, for example a cytidine-deoxythymidine, cytidine-deoxyuridine, adenosine-deoxythymidine, or adenosine-deoxyuridine dimer that contains conjugated PEG molecule as described in Figure 18. Other biodegradable nucleic acid linkers can be similarly used, for example other dimers, trimers, tetramers etc. that are designed to be biodegradable.

### Example 10: Galactosamine ribozyme conjugate targeting HBV

A nuclease-resistance ribozyme directed against the Heptatitis B viral RNA (HBV) (HepBzyme™) is in early stages of preclinical development. HepBzyme, which targets site 273 of the Hepatitis B viral RNA, has produced statistically significant decreases in serum HBV levels in a HBV transgenic mouse model in a dose-dependent manner (30 and 100 mg/kg/day). In an effort to improve hepatic uptake by targeting the asialoglycoprotein receptor, a series of 5 branched galactosamine residues were attached via phosphate linkages to the 5'-terminus of HepBzyme (Gal-HepBzyme). The affect of the galactosamine conjugation on HepBzyme was assessed by quantitation of ³²P-labeled HepBzyme and Gal-HepBzyme in plasma, liver and kidney of mice following a single SC bolus administration of 30 mg/kg. The plasma disposition of the intact ribozyme was similar between Gal-HepBzyme and HepBzyme. An approximate three-fold increase in the maximum observed concentration of intact ribozyme in liver (Cₘₐₓ) was observed in liver for Gal-HepBzyme (6.1 ± 1.8 ng/mg) vs. HepBzyme (2.2 ± 0.8 ng/mg) (p < 0.05). The area under the curve (AUCall) for Gal-HepBzyme was also increased by approximately two-fold. This was accompanied by a substantial decrease (approximately 40%) in the AUCₐₗₗ for intact ribozyme in kidney. In addition to the significant increase in Cₘₐₓ observed for intact Gal-HepBzyme in the liver, there was an increase in the total number of ribozyme equivalents, which may be suggestive of increased affinity of both the intact ribozyme and metabolites for asialoglycoprotein receptor and galactose-specific receptors in the liver. These data demonstrate that conjugation of a ribozyme with galactosamine produces a compound with a more favorable disposition profile, and illustrates the utility of conjugated ribozymes with improved *in vivo* pharmacokinetics and biodistribution. -

### Example 11: Synthesis of siNA conjugates

siNA molecules can be designed to interact with various sites in a target RNA message, for example, target sequences within the RNA sequence. The sequence of one strand of the siNA molecule(s) is complementary to the target site sequences. The siNA molecules can be chemically synthesized using methods described herein. Inactive siNA molecules that are used as control sequences can be synthesized by scrambling the sequence of the siNA molecules such that it is not complementary to the target sequence. Generally, siNA constructs can by synthesized using solid phase oligonucleotide synthesis methods as described herein (see for example Usman et al., US Patent Nos. 5,804,683; 5,831,071; 5,998,203; 6,117,657; 6,353,098; 6,362,323; 6,437,117; 6,469,158; Scaringe et al., US Patent Nos. 6,111,086; 6,008,400; 6,111,086).

In a non-limiting example, RNA oligonucleotides are synthesized in a stepwise fashion using the phosphoramidite chemistry as is known in the art. Standard phosphoramidite chemistry involves the use of nucleosides comprising any of 5'-O-dimethoxytrityl, 2'-O-tert-butyldimethylsilyl, 3'-O-2-Cyanoethyl N,N-diisopropylphosphoroamidite groups, and exocyclic amine protecting groups (e.g. N6-benzoyl adenosine, N4 acetyl cytidine, and N2-isobutyryl guanosine). Alternately, 2'-O-Silyl Ethers can be used in conjunction with acid-labile 2'-O-orthoester protecting groups in the synthesis of RNA as described by Scaringe *supra.* Differing 2' chemistries can require different protecting groups, for example 2'-deoxy-2'-amino nucleosides can utilize N-phthaloyl protection as described by Usman et al., US Patent 5,631,360).

During solid phase synthesis, each nucleotide is added sequentially (3'- to 5'-direction) to the solid support-bound oligonucleotide. The first nucleoside at the 3'-end of the chain is covalently attached to a solid support (e.g., controlled pore glass or polystyrene) using various linkers. The nucleotide precursor, a ribonucleoside phosphoramidite, and activator are combined resulting in the coupling of the second nucleoside phosphoramidite onto the 5'-end of the first nucleoside. The support is then washed and any unreacted 5'-hydroxyl groups are capped with a capping reagent such as acetic anhydride to yield inactive 5'-acetyl moieties. The trivalent phosphorus linkage is then oxidized to a more stable phosphate linkage. At the end of the nucleotide addition cycle, the 5'-O-protecting group is cleaved under suitable conditions (e.g., acidic conditions for trityl-based groups and Fluoride for silyl-based groups). The cycle is repeated for each subsequent nucleotide.

Modification of synthesis conditions can be used to optimize coupling efficiency, for example by using differing coupling times, differing reagent/phosphoramidite concentrations, differing contact times, differing solid supports and solid support linker chemistries depending on the particular chemical composition of the siNA to be synthesized. Deprotection and purification of the siNA can be performed as is generally described in Deprotection and purification of the siNA can be performed as is generally described in Usman et al., US 5,831,071, US 6,353,098, US 6,437,117, and Bellon et al., US 6,054,576, US 6,162,909, US 6,303,773, or Scaringe *supra.* Additionally, deprotection conditions can be modified to provide the best possible yield and purity of siNA constructs. For example, applicant has observed that oligonucleotides comprising 2'-deoxy-2'-fluoro nucleotides can degrade under inappropriate deprotection conditions. Such oligonucleotides are deprotected using aqueous methylamine at about 35°C for 30 minutes. If the 2'-deoxy-2'-fluoro containing oligonucleotide also comprises ribonucleotides, after deprotection with aqueous methylamine at about 35°C for 30 minutes, TEA-HF is added and the reaction maintained at about 65°C for an additional 15 minutes.

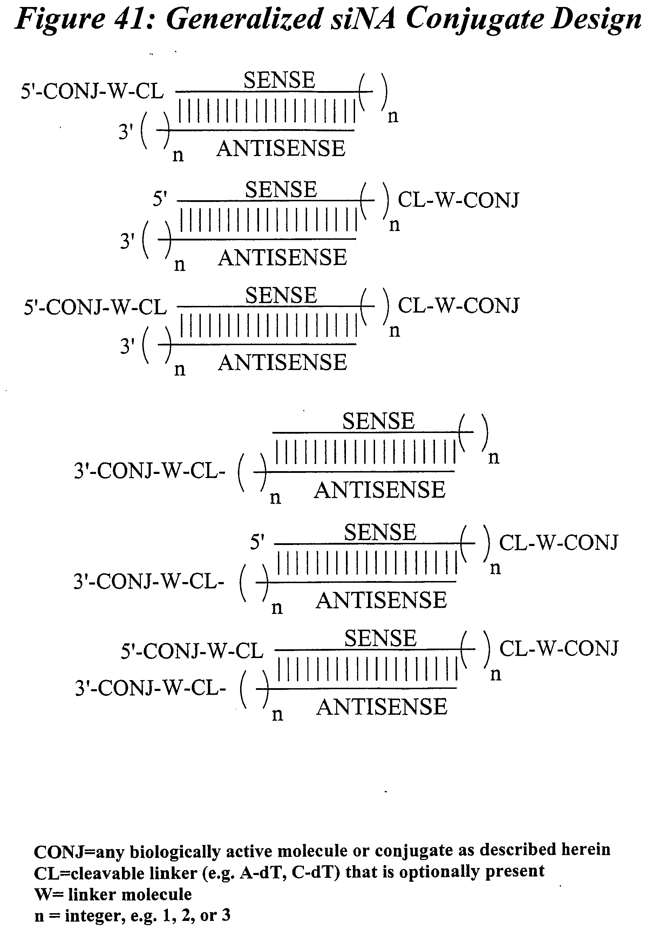

The introduction of conjugate moieties is accomplished either during solid phase synthesis using phosphoramidite chemistry described above, or post-synthetically using, for example, N-hydroxysuccinimide (NHS) ester coupling to an amino linker present in the siNA. Typically, a conjugate introduced during solid phase synthesis will be added to the 5'-end of a nucleic acid sequence as the final coupling reaction in the synthesis cycle using the phosphoramidite approach. Coupling conditions can be optimized for high yield coupling, for example by modification of coupling times and reagent concentrations to effectuate efficient coupling. As such, the 5'-end of the sense strand of a siNA molecule is readily conjugated with a conjugate moiety having a reactive phosphorus group available for coupling (e.g., a compound having Formulae 1, 5, 8, 55, 56, 57, 60, 86, 92, 104, 110, 113, 115, 116, 117, 118, 120, or 122) using the phosphoramidite approach, providing a 5'-terminal conjugate (see for example **Figure 41**).

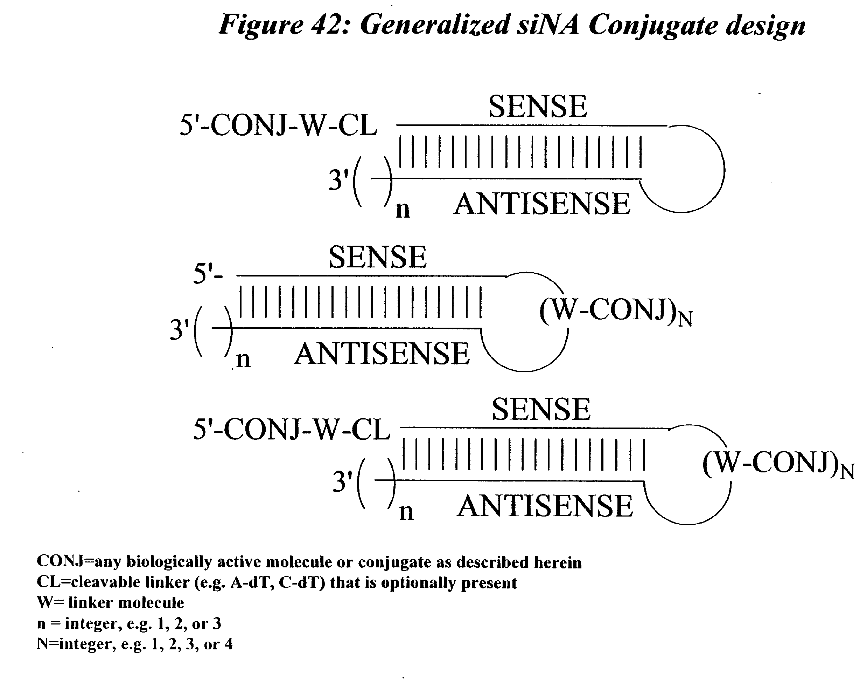

Conjugate precursors having a reactive phosphorus group and a protected hydroxyl group can be used to incorporate a conjugate moiety anywhere in the siNA sequence, such as in the loop portion of a single stranded hairpin siNA construct (see for example **Figure 42**). For example, using the phosphoramidite approach, a conjugate moiety comprising a phosphoramidite and protected hydroxyl (e.g., a compound having Formulae 86, 92, 104, 113, 115, 116, 117, 118, 120, or 122 herein) is first coupled at the desired position within the siNA sequence using solid phase synthesis phosphoramidite coupling. Second, removal of the protecting group (e.g., dimethoxytrityl) allows coupling of additional nucleotides to the siNA sequence. This approach allows the conjugate moiety to be positioned anywhere within the siNA molecule.

Conjugate derivatives can also be introduced to a siNA molecule post synthetically. Post synthetic conjugation allows a conjugate moiety to be introduced at any position within the siNA molecule where an appropriate functional group is present (e.g., a C5 alkylamine linker present on a nucleotide base or a 2'-alkylamine linker present on a nucleotide sugar can provide a point of attachment for an NHS-conjugate moiety). Generally, a reactive chemical group present in the siNA molecule is unmasked following synthesis, thus allowing post-synthetic coupling of the conjugate to occur. In a non-limiting example, an protected amino linker containing nucleotide (e.g., TFA protected C5 propylamino thymidine) is introduced at a desired position of the siNA during solid phase synthesis. Following cleavage and deprotection of the siNA, the free amine is made available for NHS ester coupling of the conjugate at the desired position within the siNA sequence, such as at the 3'-end of the sense and/or antisense strands, the 3' and/or 5'-end of the sense strand, or within the siNA sequence, such as in the loop portion of a single stranded hairpin siNA sequence.

A conjugate moiety can be introduced at different locations within a siNA molecule using both solid phase synthesis and post-synthetic coupling approaches. For example, solid phase synthesis can be used to introduce a conjugate moiety at the 5'-end of the siNA (e.g. sense strand) and post-synthetic coupling can be used to introduce a conjugate moiety at the 3'-end of the siNA (e.g. sense strand and/or antisense strand). As such, a siNA sense strand having 3' and 5' end conjugates can be synthesized (see for example **Figure 41**). Conjugate moieties can also be introduced in other combinations, such as at the 5'-end, 3'-end and/or loop portions of a siNA molecule (see for example **Figure 42**).

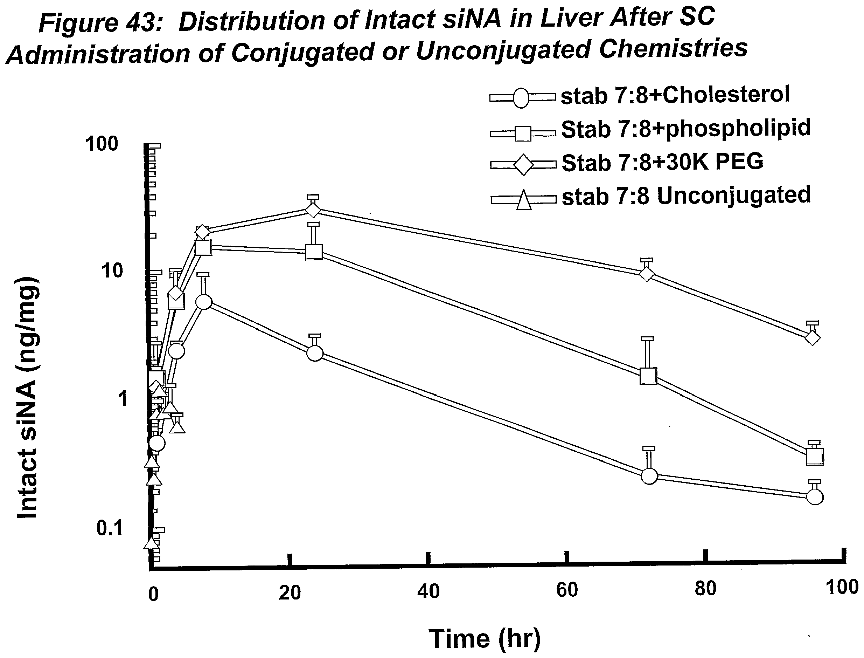

### Example 12: Phamacokinetics of siNA conjugates (Figure 43)

Three nuclease resistant siNA molecule targeting site 1580 of hepatitis B virus (HBV) RNA were designed using Stab 7/8 chemistry (see **Table IV**) and a 5'-terminal conjugate moiety.

One siNA conjugate comprises a branched cholesterol conjugate linked to the sense strand of the siNA. The "cholesterol" siNA conjugate molecule has the structure shown below: 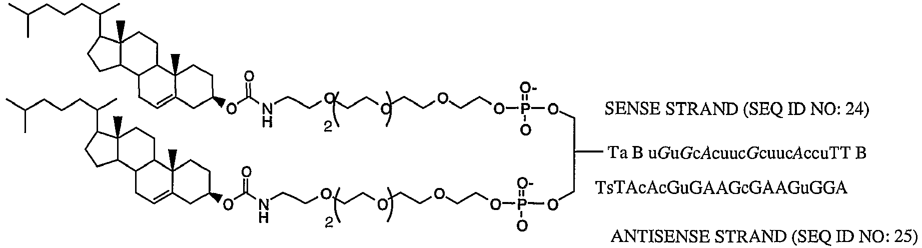 where T stands for thymidine, B stands for inverted deoxyabasic, G stands for 2'-deoxy guanosine, A stands for 2'-deoxy adenosine, G stands for 2'-O-methyl guanosine, A stands for 2'-O-methyl adenosine, u stands for 2'-fluoro uridine, c stands for 2'-fluoro cytidine, a stands for adenosine, and s stands for phosphorothioate linkage.

Another siNA conjugate comprises a branched phospholipid conjugate linked to the sense strand of the siNA. The "phospholipid" siNA conjugate molecule has the structure shown below: 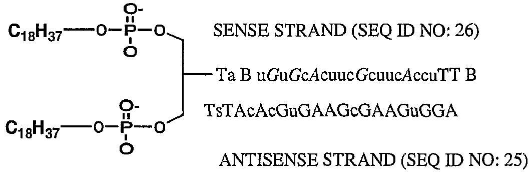 where T stands for thymidine, B stands for inverted deoxyabasic, G stands for 2'-deoxy guanosine, A stands for 2'-deoxy adenosine, G stands for 2'-O-methyl guanosine, A stands for 2'-O-methyl adenosine, u stands for 2'-fluoro uridine, c stands for 2'-fluoro cytidine, a stands for adenosine, and s stands for phosphorothioate linkage.

Another siNA conjugate comprises a polyethylene glycol (PEG) conjugate linked to the sense strand of the siNA. The "PEG" siNA conjugate molecule has the structure shown below: 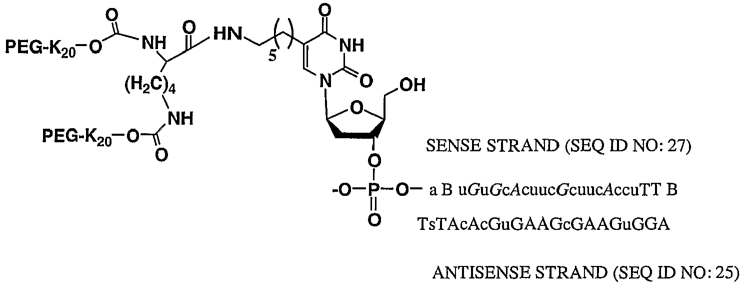 where T stands for thymidine, B stands for inverted deoxyabasic, G stands for 2'-deoxy guanosine, A stands for 2'-deoxy adenosine, G stands for 2'-O-methyl guanosine, A stands for 2'-O-methyl adenosine, u stands for 2'-fluoro uridine, c stands for 2'-fluoro cytidine, a stands for adenosine, and s stands for phosphorothioate linkage.

The Cholesterol, Phospholipid, and PEG conjugates were evaluated for pharmakokinetic properties in mice compared to a non-conjugated siNA construct having matched chemistry and sequence. This study was conducted in female CD-1 mice approximately 26 g (6-7 weeks of age). Animals were housed in groups of 3. Food and water were provided ad libitum. Temperature and humidity were according to Pharmacology Testing Facility performance standards (SOP's) which are in accordance with the 1996 Guide for the Care and Use of Laboratory Animals (NRC). Animals were acclimated to the facility for at least 3 days prior to experimentation.

Absorbance at 260 nm was used to determine the actual concentration of the stock solution of pre-annealed HBV siNA. An appropriate amount of HBV siNA was diluted in sterile veterinary grade normal saline (0.9%) based on the average body weight of the mice. A small amount of the antisense (Stab 7) strand was internally labeled with gamma 32P-ATP. The 32P-labeled stock was combined with excess sense strand (Stab 8) and annealed. Annealing was confirmed prior to combination with unlabled drug. Each mouse received a subcutaneous bolus of 30 mg/kg (based on duplex) and approximately 10 million cpm (specific activity of approximately 15 cpm/ng).

Three animals per timepoint (1, 4, 8, 24, 72, 96 h) were euthanized by CO2 inhalation followed immediately by exsanguination. Blood was sampled from the heart and collected in heparinized tubes. After exsanguination, animals were perfused with 10-15 mL of sterile veterinary grade saline via the heart. Samples of liver were then collected and frozen.

Tissue samples were homogenized in a digestion buffer prior to compound quantitation. Quantitation of intact compound was determined by scintillation counting followed by PAGE and phosphorimage analysis. Results are shown in **Figure 43****.** As shown in the figure, the conjugated siNA constructs shown vastly improved liver PK compared to the unconjugated siNA construct.

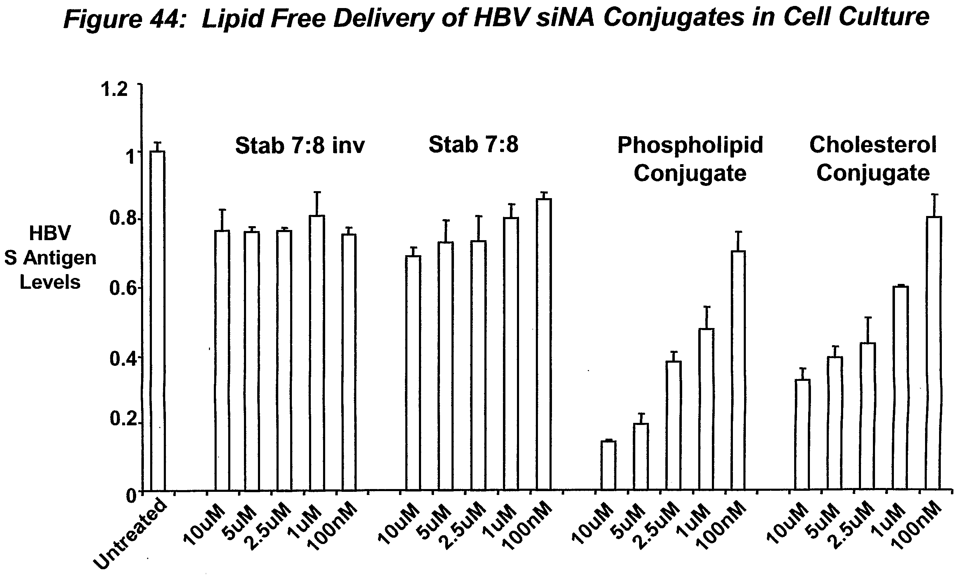

### Example 13: Cell culture of siNA conjugates (Figure 44)

The Cholesterol conjugates and Phospholipid conjugated siNA constructs described in Example 12 above were evaluated for cell culture efficacy in a HBV cell culture system.

### Transfection of HepG2 Cells with psHBV-1 and siNA

The human hepatocellular carcinoma cell line Hep G2 was grown in Dulbecco's modified Eagle media supplemented with 10% fetal calf serum, 2 mM glutamine, 0.1 mM nonessential amino acids, 1 mM sodium pyruvate, 25 mM Hepes, 100 units penicillin, and 100 µg/ml streptomycin. To generate a replication competent cDNA, prior to transfection the HBV genomic sequences are excised from the bacterial plasmid sequence contained in the psHBV-1 vector. Other methods known in the art can be used to generate a replication competent cDNA. This was done with an EcoRI and Hind III restriction digest. Following completion of the digest, a ligation was performed under dilute conditions (20 µg/ml) to favor intermolecular ligation. The total ligation mixture was then concentrated using Qiagen spin columns.

### siNA Activity Screen and Dose Response Assay

Transfection of the human hepatocellular carcinoma cell line, Hep G2, with replication-competent HBV DNA results in the expression of HBV proteins and the production of virions. To test the efficacy of siNA conjugates targeted against HBV RNA, the Cholesterol siNA conjugate and Phospholipid siNA conjugate described in Example 12 were compared to a non-conjugated control siNA (see **Figure 44**). An inverted sequence duplex was used as a negative control for the unconjugated siNA. Dose response studies were performed in which HBV genomic DNA was transfected with HBV genomic DNA with lipid at 12.5 ug/ml into Hep G2 cells. 24 hours after transfection with HBV DNA, cell culture media was removed and siNA duplexes were added to cells without lipid at 10 uM, 5, uM, 2.5 uM, 1 uM, and 100 nm and the subsequent levels of secreted HBV surface antigen (HBsAg) were analyzed by ELISA 72 hours post treatment (see **Figure 44**). To determine siNA activity, HbsAg levels were measured following transfection with siNA. Immulon 4 (Dynax) microtiter wells were coated overnight at 4° C with anti-HBsAg Mab (Biostride B88-95-31ad,ay) at 1 µg/ml in Carbonate Buffer (Na2CO3 15 mM, NaHCO3 35 mM, pH 9.5). The wells were then washed 4x with PBST (PBS, 0.05% Tween® 20) and blocked for 1 hr at 37° C with PBST, 1% BSA. Following washing as above, the wells were dried at 37° C for 30 min. Biotinylated goat ant-HBsAg (Accurate YVS1807) was diluted 1:1000 in PBST and incubated in the wells for 1 hr. at 37° C. The wells were washed 4x with PBST. Streptavidin/Alkaline Phosphatase Conjugate (Pierce 21324) was diluted to 250 ng/ml in PBST, and incubated in the wells for 1 hr. at 37° C. After washing as above, p-nitrophenyl phosphate substrate (Pierce 37620) was added to the wells, which were then incubated for 1 hour at 37° C. The optical density at 405 nm was then determined. As shown in **Figure 44****,** the phospholipid and cholesterol conjugates demonstrate marked dose dependent inhibition of HBsAg expression compared to the unconjugated siNA construct when delivered to cells without any transfection agent (lipid).

### Example 14: Purification of Nucleic Acid Conjugates

Nucleic acid conjugates of the invention can be purified using, for example, anion exchange, reverse phase, and/or hydrophobic interaction chromoatography. Non-lipophilic nucleic acid conjugates of the invention (e.g., PEG, PEI, polyamine conjugates) are readily purified using anion exchange chromatography as is known in the art. Lipophilic nucleic acid conjugates of the invention (e.g., cholesterol, phospholipid, or alkyl polymer conjugates) can be purified using reverse phase chromatography as is known in the art and/or by hydrophobic interaction chromatography. Hydrophobic interaction chromotography (HIC) allows high efficiency purification of nucleic acid conjugates of the invention without the use of organic solvents.

Hydrophobic interaction chromatography is based on interactions between hydrophobic groups on the molecules to be purified or isolated and the corresponding HIC resin. HIC utilizes such hydrophobic interactions in highly polar non-denaturing buffers. Several different HIC resins can be utilized in purifying hydrophobic polynucleotide conjugates (e.g., siNA conjugates). Examples of HIC resins include but are not limited to ether, phenyl, butyl, or hexyl stationary phases such as Toyopearl.650 S Phenyl, TSK GEL Phenyl-5PW, TSK GEL Ether-5PW, Toyopearl Ether-650, Toyopearl Phenyl-650, Toyopearl Butyl-650, and Toyopearl Hexyl-650 from TosoHaas and Fractogel EMD Phenyl S from Merck. Various conditions that can be altered to achieve highly purified material using HIC include alterations in the concentration of salts in buffers, use of different resins, varying pH and temperature.

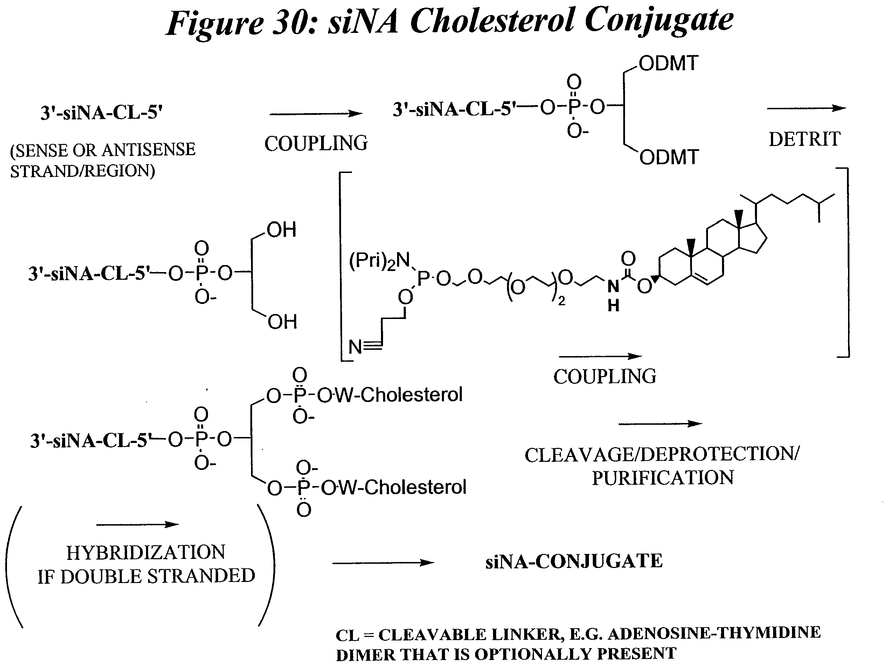

In a non-limiting example, HIC was used to purify a Stab 7/8 (**Table IV**) siNA cholesterol conjugate having SEQ ID NO: 24 (see for example **Figure 30**) and a Stab 7/8 (**Table IV**) siNA phospholipid conjugate having SEQ ID NO: 26 (see for example **Figure 19**). The purification buffer reagents used in the HIC purification consisted of Ammonium Sulfate, Sodium Phosphate Monobasic and Dibasic (see **Table V**) which were purchased from VWR. The material was purified on a Waters LC-2000 preparative system including an LC Controller and pump and a waters 24487 dual wavelength UV Detector. The system is controlled by Millennium software version 4. The buffers and loading material is passed through a heat exchanger, such as a Timberline TL50D (Timberline Instruments (Boulder, CO). The Columns used in the development included a Pharmacia HR 5/5, Pharmacia HR 16/10 and a Pharmacia Fineline 35. The columns were packed with Toyopearl Phenyl-650s (Tosoh Bioscience, LLC Montgomeryville, PA) to a bed height of 5 cm, 10 cm and 10 cm respectively as the process was scaled up. Further work included the Toyopearl 650 S Phenyl to the Tosoh Bioscience TSK GEL Phenyl-5PW and the Fractogel EMD Phenyl S.

The deprotected cholesterol siNA and phospholipid siNA conjugates were diluted in Milli-Q-water and ammonium sulfate was added to a 2M concentration following filtration and rinsing of the filter with Milli-Q-water. The addition of solid ammonium sulfate as a dry powder resulted in precipitation of the siNA conjugates. This process has been further optimized so that the oligonucleotide is diluted in Milli-Q-water and then the volume is doubled with 2 M ammonium sulfate yielding a solution of 1 M ammonium sulfate with 10 mM Sodium Phosphate.

The purified conjugated siRNA is eluted from the column during step 2 of the gradient (see **Table VI**). This step also desalts the molecule. The eluate was collected as fractions, which were analyzed by SAX or RP HPLC to determine purity. Fractions containing the conjugated product were pooled and this pool was analyzed by SAX or RP HPLC for purity. The pooled material was stored 2-8° C until annealed to the complementary strand and desalted.

**TABLE I**

| **Characteristics of naturally occurring ribozymes** | |
|---|---|
| **Group I Introns** | |
| • | Seize: ∼150 to >1000 nucleotides. |
| • | Requires a U in the target sequence immediately 5' of the cleavage site. |
| • | Binds 4-6 nucleotides at the 5'-side of the cleavage site. |
| • | Reaction mechanism: attack by the 3'-OH of guanosine to generate cleavage products with 3'-OH and 5'-guanosine. |
| • | Additional protein cofactors required in some cases to help folding and maintenance of the active structure. |
| • | Over 300 known members of this class. Found as an intervening sequence in *Tetrahymena thermophila* rRNA, fungal mitochondria, chloroplasts, phage T4, blue-green algae, and others. |
| • | Major structural features largely established through phylogenetic comparisons, mutagenesis, and biochemical studies [ⁱ,ⁱⁱ]. |
| • | Complete kinetic framework established for one ribozyme rⁱⁱⁱ,^{iv},^{v},^{vi}]. |
| • | Studies of ribozyme folding and substrate docking underway [ⁱⁱⁱ,^{viii},^{ix}]. |
| • | Chemical modification investigation of important residues well established [^{x},^{xi}]. |
| • | The small (4-6 nt) binding site may make this ribozyme too non-specific for targeted RNA cleavage, however, the Tetrahymena group I intron has been used to repair a "defective" β-galactosidase message by the ligation of new β-galactosidase sequences onto the defective message [^{xii}]. |

| **RNAse P RNA (M1 RNA)** | |
|---|---|
| • | Size: ∼290 to 400 nucleotides. |
| • | RNA portion of a ubiquitous ribonucleoprotein enzyme. |
| • | Cleaves tRNA precursors to form mature tRNA [^{xiii}]. |
| • | Reaction mechanism: possible attack by M²⁺-OH to generate cleavage products with 3'-OH and 5'-phosphate. |
| • | RNAse P is found throughout the prokaryotes and eukaryotes. The RNA subunit has been sequenced from bacteria, yeast, rodents, and primates. |
| • | Recruitment of endogenous RNAse P for therapeutic applications is possible through hybridization of an External Guide Sequence (EGS) to the target RNA [^{xiv},^{xv}] |
| • | Important phosphate and 2' OH contacts recently identified [^{xvi},^{xvii}] |
| | |

| **Group II Introns** | |
|---|---|
| • | Size: >1000 nucleotides. |
| • | Trans cleavage of target RNAs recently demonstrated [^{xviii},^{xix}]. |
| • | Sequence requirements not fully determined. |
| • | Reaction mechanism: 2'-OH of an internal adenosine generates cleavage products with 3'-OH and a "lariat" RNA containing a 3'-5' and a 2'-5' branch point. |
| • | Only natural ribozyme with demonstrated participation in DNA cleavage [^{xx},^{xxi}] in addition to RNA cleavage and ligation. |
| • | Major structural features largely established through phylogenetic comparisons [^{xxii}]. |
| • | Important 2' OH contacts beginning to be identified [^{xxiii}] |
| • | Kinetic framework under development [^{xxiv}] |
| | |

| **Neurospora VS RNA** | |
|---|---|
| • | Size: ∼144 nucleotides. |
| • | Trans cleavage of hairpin target RNAs recently demonstrated [^{xxv}], |
| • | Sequence requirements not fully determined. |
| • | Reaction mechanism: attack by 2'-OH 5' to the scissile bond to generate cleavage products with 2',3'-cyclic phosphate and 5'-OH ends. |
| • | Binding sites and structural requirements not fully determined. |
| • | Only 1 known member of this class. Found in Neurospora VS RNA. |
| | |

| **Hammerhead Ribozyme** (see text for references) | |
|---|---|
| • | Size: ∼13 to 40 nucleotides. |
| • | Requires the target sequence UH immediately 5' of the cleavage site. |
| • | Binds a variable number nucleotides on both sides of the cleavage site. |
| • | Reaction mechanism: attack by 2'-OH 5' to the scissile bond to generate cleavage products with 2',3'-cyclic phosphate and 5'-OH ends. |
| • | 14 known members of this class. Found in a number of plant pathogens (virusoids) that use RNA as the infectious agent. |
| • | Essential structural features largely defined, including 2 crystal structures [^{xxvi},^{xxvii}] |
| • | Minimal ligation activity demonstrated (for engineering through *in vitro* selection) [^{xxviii}] |
| • | Complete kinetic framework established for two or more ribozymes [^{xxix}]. |
| • | Chemical modification investigation of important residues well established [^{xxx}]. |

| **Hairpin Ribozyme** | |
|---|---|
| • | Size: ∼50 nucleotides. |
| • | Requires the target sequence GUC immediately 3' of the cleavage site. |
| • | Binds 4-6 nucleotides at the 5'-side of the cleavage site and a variable number to the 3'-side of the cleavage site. |
| • | Reaction mechanism: attack by 2'-OH 5' to the scissile bond to generate cleavage products with 2',3'-cyclic phosphate and 5'-OH ends. |
| • | 3 known members of this class. Found in three plant pathogen (satellite RNAs of the tobacco ringspot virus, arabis mosaic virus and chicory yellow mottle virus) which uses RNA as the infectious agent. |
| • | Essential structural features largely defined [^{xxxi},^{xxxii},^{xxxiii},^{xxxiv}] |
| • | Ligation activity (in addition to cleavage activity) makes ribozyme amenable to engineering through *in vitro* selection [^{xxxv}] |
| • | Complete kinetic framework established for one ribozyme [^{xxxvi}]. |
| • | Chemical modification investigation of important residues begun [^{xxxvii},^{xxxviii}]. |

| **Hepatitis Delta Virus (HDV) Ribozyme** | |
|---|---|
| • | Size: ∼60 nucleotides. |
| • | Trans cleavage of target RNAs demonstrated [^{xxxix}]. |
| • | Binding sites and structural requirements not fully determined, although no sequences 5' of cleavage site are required. Folded ribozyme contains a pseudoknot structure [^{xl}]. |
| • | Reaction mechanism: attack by 2'-OH 5' to the scissile bond to generate cleavage products with 2',3'-cyclic phosphate and 5'-OH ends. |
| • | Only 2 known members of this class. Found in human HDV. |
| • | Circular form of HDV is active and shows increased nuclease stability [^{xli}] |

| | |
|---|---|
| ⁱ. Michel, Francois; Westhof, Eric. Slippery substrates. Nat. Struct. Biol. (1994), 1(1), 5-7. ⁱⁱ. Lisacek, Frederique; Diaz, Yolande; Michel, Francois. Automatic identification of group I intron cores in genomic DNA sequences. J. Mol. Biol. (1994), 235(4), 1206-17. ⁱⁱⁱ. Herschlag, Daniel; Cech, Thomas R.. Catalysis of RNA cleavage by the Tetrahymena thermophila ribozyme. 1. Kinetic description of the reaction of an RNA substrate complementary to the active site. Biochemistry (1990), 29(44), 10159-71. ^{iv}. Herschlag, Daniel; Cech, Thomas R.. Catalysis of RNA cleavage by the Tetrahymena thermophila ribozyme. 2. Kinetic description of the reaction of an RNA substrate that forms a mismatch at the active site. Biochemistry (1990), 29(44), 10172-80. ^{v}. Knitt, Deborah S.; Herschlag, Daniel. pH Dependencies of the Tetrahymena Ribozyme Reveal an Unconventional Origin of an Apparent pKa. Biochemistry (1996), 35(5), 1560-70. ^{vi}. Bevilacqua, Philip C.; Sugimoto, Naoki; Turner, Douglas H.. A mechanistic framework for the second step of splicing catalyzed by the Tetrahymena ribozyme. Biochemistry (1996), 35(2), 648-58. ^{vii}. Li, Yi; Bevilacqua, Philip C.; Mathews, David; Turner, Douglas H.. Thermodynamic and activation parameters for binding of a pyrene-labeled substrate by the Tetrahymena ribozyme: docking is not diffusion-controlled and is driven by a favorable entropy change. Biochemistry (1995), 34(44), 14394-9. ^{viii}. Banerjee, Aloke Raj; Turner, Douglas H.. The time dependence of chemical modification reveals slow steps in the folding of a group I ribozyme. Biochemistry (1995), 34(19), 6504-12. ^{ix}. Zarrinkar, Patrick P.; Williamson, James R.. The P9.1-P9.2 peripheral extension helps guide folding of the Tetrahymena ribozyme. Nucleic Acids Res. (1996), 24(5), 854-8. ^{x}. Strobel, Scott A.; Cech, Thomas R.. Minor groove recognition of the conserved G.cntdot.U pair at the Tetrahymena ribozyme reaction site. Science (Washington, D. C.) (1995), 267(5198), 675-9. ^{xi}. Strobel, Scott A.; Cech, Thomas R.. Exocyclic Amine of the Conserved G.cntdot.U Pair at the Cleavage Site of the Tetrahymena Ribozyme Contributes to 5'-Splice Site Selection and Transition State Stabilization. Biochemistry (1996), 35(4), 1201-11. ^{xii}. Sullenger, Bruce A.; Cech, Thomas R.. Ribozyme-mediated repair of defective mRNA by targeted trans-splicing. Nature (London) (1994), 371(6498), 619-22. ^{xii}. Robertson, H.D.; Altman, S.; Smith, J.D. J. Biol. Chem., 247, 5243-5251 (1972). ^{xiv}. Forster, Anthony C.; Altman, Sidney. External guide sequences for an RNA enzyme. Science (Washington, D. C., 1883-) (1990), 249(4970), 783-6. ^{xv}. Yuan, Y.; Hwang, E. S.; Altman, S. Targeted cleavage of mRNA by human RNase P. Proc. Natl. Acad. Sci. USA (1992) 89,8006-10. ^{xvi}. Harris, Michael E.; Pace, Norman R.. Identification of phosphates involved in catalysis by the ribozyme RNase P RNA. RNA (1995), 1(2), 210-18. ^{xvii}. Pan, Tao; Loria, Andrew; Zhong, Kun. Probing of tertiary interactions in RNA: 2'-hydroxyl-base contacts between the RNase P RNA and pre-tRNA. Proc. Natl. Acad. Sci. U. S. A. (1995), 92(26), 12510-14. ^{xviii}. Pyle, Anna Marie; Green, Justin B.. Building a Kinetic Framework for Group II Intron Ribozyme Activity: Quantitation of Interdomain Binding and Reaction Rate. Biochemistry (1994), 33(9), 2716-25. ^{xix}. Michels, William J. Jr.; Pyle, Anna Marie. Conversion of a Group II Intron into a New Multiple-Turnover Ribozyme that Selectively Cleaves Oligonucleotides: Elucidation of Reaction Mechanism and Structure/Function Relationships. Biochemistry (1995), 34(9), 2965-77. ^{xx}. Zimmerly, Steven; Guo, Huatao; Eskes, Robert; Yang, Jian; Perlman, Philip S.; Lambowitz, Alan M.. A group II intron RNA is a catalytic component of a DNA endonuclease involved in intron mobility. Cell (Cambridge, Mass.) (1995), 83(4), 529-38. ^{xxi}. Griffin, Edmund A., Jr.; Qin, Zhifeng; Michels, Williams J., Jr.; Pyle, Anna Marie. Group II intron ribozymes that cleave DNA and RNA linkages with similar efficiency, and lack contacts with substrate 2'-hydroxyl groups. Chem. Biol. (1995), 2(11), 761-70. ^{xxii}. Michel, Francois; Ferat, Jean Luc. Structure and activities of group II introns. Annu. Rev. Biochem. (1995), 64, 435-61. ^{xxiii}. Abramovitz, Dana L.; Friedman, Richard A.; Pyle, Anna Marie. Catalytic role of 2'-hydroxyl groups within a group II intron active site. Science (Washington, D. C.) (1996), 271(5254),1410-13. ^{xxiv}. Daniels, Danette L.; Michels, William J., Jr.; Pyle, Anna Marie. Two competing pathways for self-splicing by group II introns: a quantitative analysis of in vitro reaction rates and products. J. Mol. Biol. (1996), 256(1), 31-49. ^{xxv}. Guo, Hans C. T.; Collins, Richard A.. Efficient trans-cleavage of a stem-loop RNA substrate by a ribozyme derived from Neurospora VS RNA. EMBO J. (1995), 14(2), 368-76. ^{xxvi}. Scott, W.G., Finch, J.T., Aaron,K. The crystal structure of an all RNA hammerhead ribozyme:Aproposed mechanism for RNA catalytic cleavage. Cell, (1995), 81, 991-1002. ^{xxvii}. McKay, Structure and function of the hammerhead ribozyme: an unfinished story. RNA, (1996), 2, 395-403. ^{xxviii}. Long, D., Uhlenbeck, O., Hertel, K. Ligation with hammerhead ribozymes. US Patent No. 5,633,133. ^{xxix}. Hertel, K.J., Herschlag, D., Uhlenbeck, O. A kinetic and thermodynamic framework for the hammerhead ribozyme reaction. Biochemistry, (1994) 33, 3374-3385.Beigelman, L., et al., Chemical modifications of hammerhead ribozymes. J. Biol. Chem., (1995) 270, 25702-25708. ^{xxx}. Beigelman, L., et al., Chemical modifications of hammerhead ribozymes. J. Biol. Chem., (1995) 270,25702-25708. ^{xxxi}. Hampel, Arnold; Tritz, Richard; Hicks, Margaret; Cruz, Phillip. 'Hairpin' catalytic RNA model: evidence for helixes and sequence requirement for substrate RNA. Nucleic Acids Res. (1990), 18(2), 299-304. ^{xxxii}. Chowrira, Bharat M.; Berzal-Herranz, Alfredo; Burke, John M.. Novel guanosine requirement for catalysis by the hairpin ribozyme. Nature (London) (1991), 354(6351), 320-2. ^{xxxiii}. Berzal-Herranz, Alfredo; Joseph, Simpson; Chowrira, Bharat M.; Butcher, Samuel E.; Burke, John M.. Essential nucleotide sequences and secondary structure elements of the hairpin ribozyme. EMBO J. (1993), 12(6), 2567-73. ^{xxxiv}. Joseph, Simpson; Berzal-Herranz, Alfredo; Chowrira, Bharat M.; Butcher, Samuel E.. Substrate selection rules for the hairpin ribozyme determined by in vitro selection, mutation, and analysis of mismatched substrates. Genes Dev. (1993), 7(1), 130-8. ^{xxxv}. Berzal-Herranz, Alfredo; Joseph, Simpson; Burke, John M.. In vitro selection of active hairpin ribozymes by sequential RNA-catalyzed cleavage and ligation reactions. Genes Dev. (1992), 6(1), 129-34. ^{xxxvi}. Hegg, Lisa A.; Fedor, Martha J.. Kinetics and Thermodynamics of Intermolecular Catalysis by Hairpin Ribozymes. Biochemistry (1995), 34(48), 15813-28. ^{xxxvii}. Grasby, Jane A.; Mersmann, Karin; Singh, Mohinder; Gait, Michael J.. Purine Functional Groups in Essential Residues of the Hairpin Ribozyme Required for Catalytic Cleavage of RNA. Biochemistry (1995), 34(12), 4068-76. ^{xxxviii}. Schmidt, Sabine; Beigelman, Leonid; Karpeisky, Alexander; Usman, Nassim; Sorensen, Ulrik S.; Gait, Michael J.. Base and sugar requirements for RNA cleavage of essential nucleoside residues in internal loop B of the hairpin ribozyme: implications for secondary structure. Nucleic Acids Res. (1996), 24(4), 573-81. ^{xxxix}. Perrotta, Anne T.; Been, Michael D.. Cleavage of oligoribonucleotides by a ribozyme derived from the hepatitis .delta. virus RNA sequence. Biochemistry (1992), 31(1), 16-21. ^{xl}. Perrotta, Anne T.; Been, Michael D.. A pseudoknot-like structure required for efficient self-cleavage of hepatitis delta virus RNA. Nature (London) (1991), 350(6317), 434-6. ^{xli}. Puttaraju, M.; Perrotta, Anne T.; Been, Michael D.. A circular trans-acting hepatitis delta virus ribozyme. Nucleic Acids Res. (1993), 21(18), 4253-8. | |

**Table II:**

| **A. 2.5 *µ*mol Synthesis Cycle ABI 394 Instrument** | | | | | |
|---|---|---|---|---|---|
| **Reagent** | **Equivalents** | **Amount** | **Wait Time* DNA** | **Wait Time* 2'-O-methyl** | **Wait Time*RNA** |
| | | | | | |
| Phosphoramidites | 6.5 | 163 *µ*L | 45 sec | 2.5 min | 7.5 min |
| *S*-Ethyl Tetrazole | 23.8 | 238 *µ*L | 45 sec | 2.5 min | 7.5 min |
| Acetic Anhydride | 100 | 233 *µ*L | 5 sec | 5 sec | 5 sec |
| *N*-Methyl Imidazole | 186 | 233 *µ*L | 5 sec | 5 sec | 5 sec |
| TCA | 176 | 2.3 mL | 21 sec | 21 sec | 21 sec |
| Iodine | 11.2 | 1.7 mL | 45 sec | 45 sec | 45 sec |
| Beaucage | 12.9 | 645 *µ*L | 100 sec | 300 sec | 300 sec |
| Acetonitrile | NA | 6.67 mL | NA | NA | NA |

| **B. 0.2 pmol Synthesis Cycle ABI 394 Instrument** | | | | | |
|---|---|---|---|---|---|
| **Reagent** | **Equivalents** | **Amount** | **Wait Time* DNA** | **Wait Time* 2'-O-methyl** | **Wait Time*RNA** |
| | | | | | |
| Phosphoramidites | 15 | 31 *µ*L | 45 sec | 233 sec | 465 sec |
| S-Ethyl Tetrazole | 38.7 | 31 *µ*L | 45 sec | 233 min | 465 sec |
| Acetic Anhydride | 655 | 124 *µ*L | 5 sec | 5 sec | 5 sec |
| *N*-Methyl Imidazole | 1245 | 124 *µ*L | 5 sec | 5 sec | 5 sec |
| TCA | 700 | 732 *µ*L | 10 sec | 10 sec | 10 sec |
| Iodine | 20.6 | 244 *µ*L | 15 sec | 15 sec | 15 sec |
| Beaucage | 7.7 | 232 *µ*L | 100 sec | 300 sec | 300 sec |
| Acetonitrile | NA | 2.64 mL | NA | NA | NA |

| **C. 0.2 *µ*mol Synthesis Cycle 96 well Instrument** | | | | | |
|---|---|---|---|---|---|
| **Reagent** | **Equivalents:DNA/2'-O-methyl/Ribo** | **Amount: DNA/2'-O-methyl/Ribo** | **Wait Time* DNA** | **Wait Time* 2'-O-methyl** | **Wait Time* Ribo** |
| | | | | | |
| Phosphoramidites | 22/33/66 | 40/60/120 *µ*L | 60 sec | 180 sec | 360sec |
| S-Ethyl Tetrazole | 70/105/210 | 40/60/120 *µ*L | 60 sec | 180 min | 360 sec |
| Acetic Anhydride | 265/265/265 | 50/50/50 *µ*L | 10 sec | 10 sec | 10 sec |
| *N*-Methyl Imidazole | 502/502/502 | 50/50/50 *µ*L | 10 sec | 10 sec | 10 sec |
| TCA | 238/475/475 | 250/500/500 *µ*L | 15 sec | 15 sec | 15 sec |
| Iodine | 6.8/6.8/6.8 | 80/80/80 *µ*L | 30 sec | 30 sec | 30 sec |
| Beaucage | 34/51/51 | 80/120/120 | 100 sec | 200 sec | 200 sec |
| Acetonitrile | NA | 1150/1150/1150 *µ*L | NA | NA | NA |

| | | | | | |
|---|---|---|---|---|---|
| • **Wait time does not include contact time during delivery.** | | | | | |

**Table III: Peptides for Conjugation**

| **Peptide** | **Sequence** | **SEQ ID NO** |
|---|---|---|
| ANTENNAP EDIA | RQI KIW FQN RRM KWK K amide | 14 |
| Kaposi fibroblast growth factor | | 15 |
| *caiman crocodylus* Ig(5) light chain | MGL GLH LLV LAA ALQ GA | 16 |
| HIVenvelope glycoprotein gp41 | | 17 |
| HIV-1 Tat | RKK RRQ RRR | 18 |
| Influenza hemagglutini n envelop glycoprotein | GLFEAIAGFIENGWEGMIDGGGYC | 19 |
| RGD peptide | X-RGD-X where X is any amino acid or peptide | 20 |
| transportan A | | 21 |
| Somatostatin (tyr-3-octreotate) | (S)*F*C YWK TCT | 22 |
| Pre-S-peptide | (S)DH QLN PAF | 23 |

| | | |
|---|---|---|
| (S) optional Serine for coupling *Italic* = optional D isomer for stability | | |

**Table IV**

| Examples of Stabilization Chemistries for chemically modified siNA constructs | | | | | |
|---|---|---|---|---|---|
| **Chemistry** | **pyrimidine** | **Purine** | **cap** | **p=S** | **Strand** |
| **"Stab 00"** | Ribo | Ribo | TT at 3'-ends | | S/AS |
| **"Stab 1"** | Ribo | Ribo | - | 5 at 5'-end | S/AS |
| | | | | 1 at 3'-end | |
| **"Stab 2"** | Ribo | Ribo | - | All linkages | Usually AS |
| **"Stab 3"** | 2'-fluoro | Ribo | - | 4 at 5'-end | Usually S |
| | | | | 4 at 3'-end | |
| **"Stab 4"** | 2'-fluoro | Ribo | 5' and 3'-ends | - | Usually S |
| **"Stab 5"** | 2'-fluoro | Ribo | - | 1 at 3'-end | Usually AS |
| **"Stab 6"** | 2'-O-Methyl | Ribo | 5' and 3'-ends | - | Usually S |
| **"Stab 7"** | 2'-fluoro | 2'-deoxy | 5' and 3'-ends | - | Usually S |
| **"Stab 8"** | 2'-fluoro | 2'-O-Methyl | - | 1 at 3'-end | Usually AS |
| **"Stab 9"** | Ribo | Ribo | 5' and 3'-ends | - | Usually S |
| **"Stab 10"** | Ribo | Ribo | - | 1 at 3'-end | Usually AS |
| **"Stab 11"** | 2'-fluoro | 2'-deoxy | - | 1 at 3'-end | Usually AS |
| **"Stab 12"** | 2'-fluoro | LNA | 5' and 3'-ends | | Usually S |
| **"Stab 13"** | 2'-fluoro | LNA | | 1 at 3'-end | Usually AS |
| **"Stab 14"** | 2'-fluoro | 2'-deoxy | | 2 at 5'-end | Usually AS |
| | | | | 1 at 3'-end | |
| **"Stab 15"** | 2'-deoxy | 2'-deoxy | | 2 at 5'-end | Usually AS |
| | | | | 1 at 3'-end | |
| **"Stab 16** | Ribo | 2'-O-Methyl | 5' and 3'-ends | | Usually S |
| **"Stab 17"** | 2'-O-Methyl | 2'-O-Methyl | 5' and 3'-ends | | Usually S |
| **"Stab 18"** | 2'-fluoro | 2'-O-Methyl | 5' and 3'-ends | 1 at 3' - end | Usually S |
| **"Stab 19"** | 2'-fluoro | 2'-O-Methyl | 3'-end | | Usually AS |
| **"Stab 20"** | 2'-fluoro | 2'-deoxy | 3'-end | | Usually AS |
| **"Stab 21"** | 2'-fluoro | Ribo | 3'-end | | Usually AS |
| **"Stab 22"** | Ribo | Ribo | 3'-end | | Usually AS |
| **"Stab 23"** | 2'-fluoro* | 2'-deoxy* | 5' and 3'-ends | | Usually S |
| **"Stab 24"** | 2'-fluoro* | 2'-O-Methyl* | - | 1 at 3'-end | Usually AS |

| | | | | | |
|---|---|---|---|---|---|
| CAP = any terminal cap. All Stab 1-24 chemistries can comprise 3'-terminal thymidine (TT) residues All Stab 1-24 chemistries typically comprise about 21 nucleotides, but can vary as described herein. S = sense strand AS = antisense strand *Stab 23 has single ribonucleotide adjacent to 3'-CAP *Stab 24 has single ribonucleotide at 5'-terminus | | | | | |

**Table V: Typical Hydrophobic Interaction Chromatography (HIC) Buffers**

| | | | pH | Conductivity |
|---|---|---|---|---|
| Equilibrium (A) | 1.0 M Ammonium Sulfate, | 10 mM Sodium Phosphate, | 7.0 ± 0.3 | |
| Elution (B) | N/A | 100 mM Sodium Phosphate, | 7.0 ± 0.3 | |
| Elution 2 (C) | N/A | N/A | N/A | < 20 µS/cm |

**Table VI: Typical HIC Gradient**

| Gradient Step | Buffer | Time |
|---|---|---|
| Step 1 | 100% A to 100 % B | 20 cv |
| Step 2 | 100% B to 20% C | 30 cv |
| Step 3 | 20% C to 100% C | 30 cv |

## Claims

1. A compound having Formula 107: 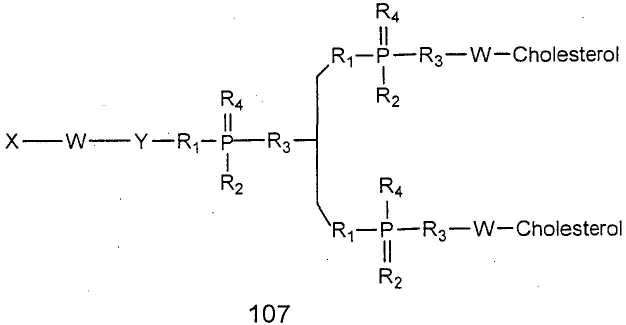 wherein X comprises a siNA molecule or a portion thereof;
each W independently comprises a linker molecule or chemical linkage selected from the group consisting of amide, phosphate, phosphate ester, phosphoramidate, or thiophosphate ester linkage;
Y comprises a linker molecule that can be present or absent; and
each R₁, R₂, R₃, and R₄ independently comprises O, OH, H, alkyl, alkylhalo, O-alkyl, O-alkylcyano, S, S-alkyl, S-alkylcyano, N or substituted N;
wherein said W-cholesterol comprises a compound having Formula 109: 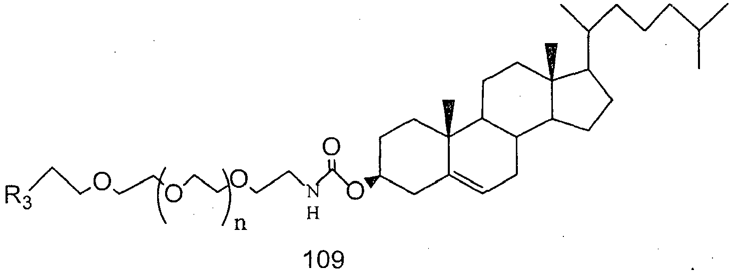 wherein n is independently an integer from about 1 to about 20.

2. The compound of claim 1, wherein said siNA molecule comprises a sense strand and an antisense strand, and wherein said sense strand is conjugated with a compound comprising Formula 107.

## Patentansprüche

1. Verbindung mit der Formel 107: 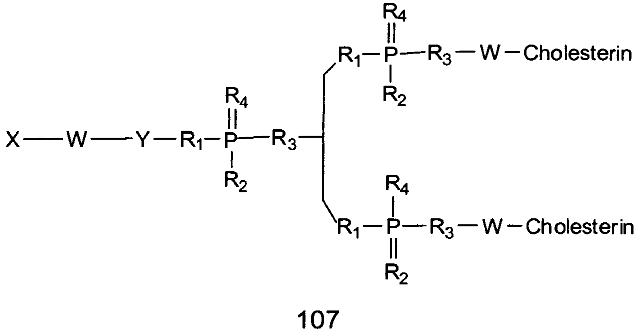 wobei X ein SiNA-Molekül oder einen Teil davon umfasst;
jedes W unabhängig ein Linker-Molekül oder chemische Bindung, ausgewählt aus der Gruppe, bestehend aus Amid, Phosphat, Phosphatester, Phosphoramidat oder Thiophosphatesterbindung, umfasst;
Y ein Linker Molekül, das vorhanden oder abwesend sein kann, umfasst; und jedes R₁, R₂, R₃ und R₄ unabhängig O, OH, H, Alkyl, Alkylhalid, O-Alkyl, O-Alkylcyano, S, S-Alkyl, S-Alkylcyano, N oder substituiertes N umfasst;
wobei das W-Cholesterin eine Verbindung mit der Formel 109 umfasst: 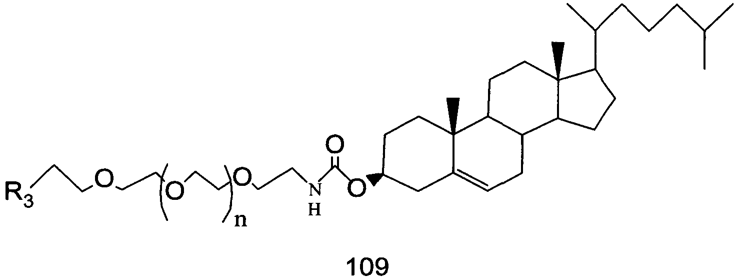 wobei n unabhängig eine ganze Zahl von ungefähr 1 bis ungefähr 20 ist.

2. Verbindung nach Anspruch 1, wobei das siNA-Molekül einen codierenden Strang und einen codogenen Strang umfasst und wobei der codierende Strang mit einer Verbindung, umfassend Formel 107, konjugiert ist.

## Revendications

1. Composé de formule 107 : 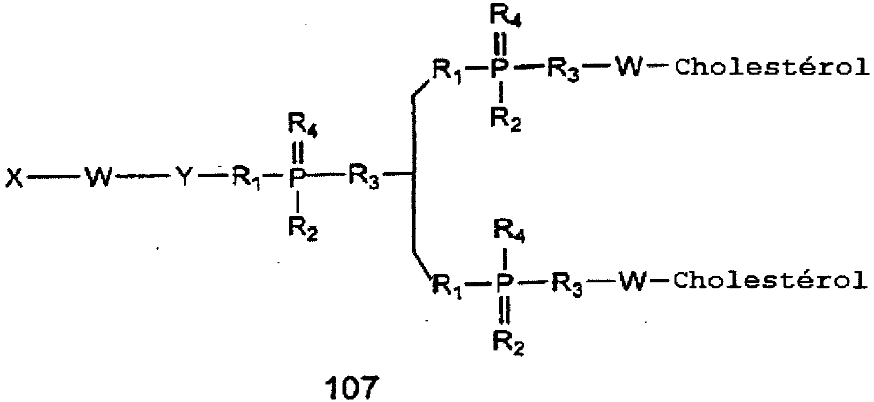 dans laquelle X comprend une molécule d'ANsi ou une partie de celle-ci ;
chaque W comprend indépendamment une molécule de lieur ou une liaison chimique choisie dans le groupe constitué par la liaison amide, phosphate, ester de phosphate, phosphoramidate, ou ester de thiophosphate ;
Y comprend une molécule de lieur qui peut être présente ou absente ; et
chaque R₁, R₂, R₃, et R₄ comprend indépendamment O, OH, H, un alkyle, un alkylhalo, un O-alkyle, un O-alkylcyano, S, un S-alkyle, un S-alkylcyano, N ou N substitué ;
dans laquelle ledit W-cholestérol comprend un composé de formule 109 : 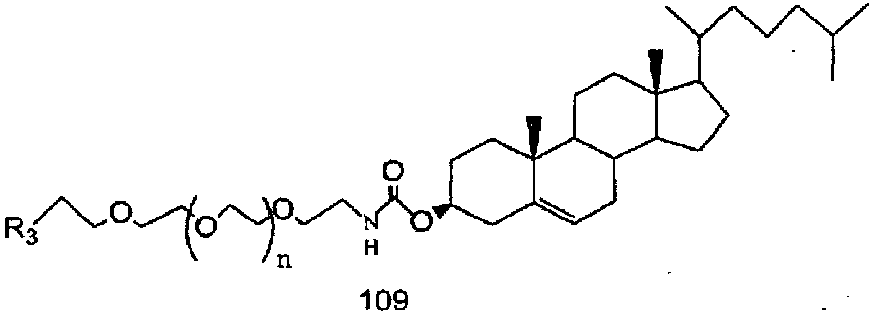 dans laquelle n est indépendamment un nombre entier allant d'environ 1 à environ 20.

2. Composé selon la revendication 1, dans lequel ladite molécule d'ANsi comprend un brin sens et un brin antisens, et dans lequel ledit brin sens est conjugué à un composé comprenant la formule 107.
